# EUROPEAN PATENT SPECIFICATION

(11) **EP 3 225 690 B1**
(45) Date of publication and mention of the grant of the patent: **23.02.2022**
(21) Application number: 15862847.9
(22) Date of filing: 01.09.2015
(51) Int. Cl.: C12N 15/70, C12N 15/74, C07K 19/00, A61K 39/104, A61K 39/08, A61K 39/112, A61P 31/04

(54) **METHOD FOR PREPARING BACTERIAL POLYSACCHARIDE-MODIFIED RECOMBINANT FUSION PROTEIN AND USE THEREOF**
VERFAHREN ZUR HERSTELLUNG BAKTERIELLER POLYSACCHARIDMODIFIZIERTER REKOMBINANTER FUSIONSPROTEINE UND VERWENDUNG DAVON
PROCÉDÉ DE PRÉPARATION DE PROTÉINE DE FUSION RECOMBINANTE MODIFIÉE PAR UN POLYSACCHARIDE BACTÉRIEN ET SON UTILISATION

(30) Priority: 27.11.2014 CN 201410709118
(43) Date of publication of application: 04.10.2017
(73) Proprietor: Institute of Biotechnology, Academy of Military Medical Sciences, China, Beijing 100071 (CN)
(72) Inventor: WANG, Hengliang, Beijing 100071 (CN); SUN, Peng, Beijing 100071 (CN); WU, Jun, Beijing 100071 (CN); ZHU, Li, Beijing 100071 (CN); PAN, Chao, Beijing 100071 (CN); LIU, Bo, Beijing 100071 (CN); PENG, Zhehui, Beijing 100071 (CN); CHANG, Shaohong, Beijing 100071 (CN); GONG, Xin, Beijing 100071 (CN); FENG, Erling, Beijing 100071 (CN); WANG, Bin, Beijing 100071 (CN); ZENG, Ming, Beijing 100071 (CN)
(74) Representative: Weickmann & Weickmann PartmbB
(86) International application number: PCT/CN2015/088737
(87) International publication number: WO 2016/082597

(56) References cited:
- CN-A- 1 255 861
- CN-A- 1 425 465
- US-A1- 2011 243 980
- FARIDMOAYER A ET AL: "Extreme substrate promiscuity of the Neisseria oligosaccharyl transferase involved in protein O-glycosylation", JOURNAL OF BIOLOGICAL CHEMISTRY, AMERICAN SOCIETY FOR BIOCHEMISTRY AND MOLECULAR BIOLOGY, US, vol. 283, no. 50, 12 December 2008 (2008-12-12), pages 34596-34604, XP002616754, ISSN: 0021-9258, DOI: 10.1074/JBC.M807113200
- M. A. MUSUMECI ET AL: "In Vitro Activity of Neisseria meningitidis PglL O-Oligosaccharyltransferase with Diverse Synthetic Lipid Donors and a UDP-activated Sugar", JOURNAL OF BIOLOGICAL CHEMISTRY, vol. 288, no. 15, 4 March 2013 (2013-03-04), pages 10578-10587, XP055307419, US ISSN: 0021-9258, DOI: 10.1074/jbc.M112.432815
- KOWARIK M ET AL: "Definition of the bacterial N-glycosylation site consensus sequence", EMBO (EUROPEAN MOLECULAR BIOLOGY ORGANIZATION) JOURNAL, WILEY, DE, vol. 25, no. 9, 13 April 2006 (2006-04-13) , pages 1957-1966, XP002408605, ISSN: 0261-4189, DOI: 10.1038/SJ.EMBOJ.7601087
- IHSSEN J. ET AL.: 'Production of Glycoprotein Vaccines in Escherichia Coli' MICROBIAL CELL FACTORIES vol. 9, no. 1, 11 August 2010, pages 1 - 13, XP021077209 DOI: 10.1186/1475-2859-9-61
- XU M.R. ET AL.: 'Production of 0157 Polysaccharides-pilin Conjugates in Escherichia Coli' MIL MED SCI vol. 37, no. 8, 25 August 2013, XP009503246
- None
- Schulz: "Identification of Bacterial Protein OOligosaccharyltransferases and Their Glycoprotein Substrates", PLOS ONE, vol. 8, 1 January 2013 (2013-01-01), page e62768, XP055813347,

## Description

### Technical field

The invention relates to a method for preparing a polysaccharide-modified protein and use of the protein; particularly, relates to a method for preparing a bacterial polysaccharide-modified recombinant fusion protein and use of the protein, and belongs to the field of biomedicine.

### Background Art

O-polysaccharide (OPS), capsular polysaccharide (CPS) and the like of pathogenic bacteria are generally the important protective antigens, such as OPS of *E*. *coli* type 0157, OPS of *Vibrio cholerae* types O1 and 0139, CPS of *Streptococcus pneumoniae* types 4, 6B, 9V, 14, 18C, 19F, 23F, etc., and CPS of *Staphylococcus aureus* types CP5 and CP8. Many antibodies against polysaccharides are the neutralizing antibodies of pathogenic bacteria, and therefore development of polysaccharide vaccines is one of the most important aspects in development of bacterial vaccines.

A lot of polysaccharide vaccines such as pneumonia polysaccharide vaccine, meningitis polysaccharide vaccine, haemophilus influenza polysaccharide vaccine, and Typhoid Vi Polysaccharide Vaccine, have been available in market for many years. However, polysaccharide vaccines have a weak immunologic memory and immunogenicity, particularly in children under 2 years old and old people. The research focus has been changed from development of this class of vaccines to development of polysaccharide-protein conjugate vaccines. Among them, 7-Valent pneumococcal polysaccharide-protein conjugate vaccine, 4-valent meningococca polysaccharide-protein conjugate vaccine, and Haemophilus influenzae type b conjugate vaccine have been available in market, and some polysaccharide-protein conjugate vaccines are in different development phases. However, a chemical process for preparing a polysaccharide-protein conjugate vaccine comprises the following steps: (1) culturing pathogenic bacteria (or vaccine strains thereof) in a large scale, from which polysaccharides are extracted; (2) preparing and purifying a toxin protein such as diphtheria toxin and tetanus toxin, which is used as a carrier protein after inactivation; (3) chemically linking the activated polysaccharide to the carrier protein; and (4) further purifying the cross-linked polysaccharide-protein conjugate to prepare a vaccine. The process has the following problems: (1) there is a certain safety risk when culturing less virulent vaccine stains of pathogenic bacteria in a large scale for extracting polysaccharides, sometimes negative pressure workshops are needed, and for some virulent pathogens, they cannot be cultured in a large scale to extract polysaccharides unless the attenuated strains of the pathogenic bacteria are obtained; (2) the polysaccharides extracted by chemical methods are generally a mixture, and have disadvantages such as low purity, uncontrollable quality, and causing side effects easily; (3) the cross-linking of a polysaccharide to a carrier protein is random, the product has a poor homogenicity, and there are difficulties in purification and quality control; and (4) since there are too many steps in the process, the yield is low and the cost is high.

In recent years, with the development in sequencing technology and bioinformatics, native protein glycosylation systems are found in many bacteria, such as N-glycosylation system in *Campylobacter jejuni,* and O-glycosylation system in *Neisseria meningitidis* and *Pseudomonas aeruginosa.* In these glycosylation systems, the polysaccharide synthesis pathway is very similar to the lipopolysaccharide and capsular polysaccharide synthesis pathways in bacteria, wherein the polysaccharide structure depends on gene clusters for glycosyl synthesis, and the oligosaccharyltransferase has a low specificity for the polysaccharide to be transferred thereby. In the N-glycosylation system in *Campylobacter jejuni,* polysaccharide substrates for N-oligosaccharyltransferase PglB are those which are required to have an acetamido group at the C2 position of the first glycosyl at the reducing terminus, and wherein the second glycosyl cannot be linked to the first glycosyl via a β(1-4) linkage. However, in the O-glycosylation system of *Neisseria meningitidis,* O-oligosaccharyltransferase PglL has no such requirements for polysaccharide substrates. PglL can transfer a polysaccharide which does not have an acetamido group at the C2 position of the first glycosyl at the reducing terminus, to the glycosylation site of a protein, and thus has a wider application prospect. E.g. US 2011/243980 A1 describes methods and systems for O-glycosylating proteins. DNA comprising a gene that produces a PgIL-like oligosaccharyltransferase and DNA comprising a gene that produces a protein to be glycosylated are used in such methods. However, there are few researches on the specificity of PglL for protein substrates, and its known substrate is only *Neisseria meningitides* pilin PilE, which restricts its application in preparation of polysaccharide-protein conjugate vaccines.

### Contents of Invention

The purpose of the invention is to provide a method for preparing a bacterial polysaccharide-modified recombinant fusion protein and use of the bacterial polysaccharide-modified recombinant fusion protein.

The invention is defined by the claims and provides a method for preparing a bacterial polysaccharide-modified recombinant fusion protein, comprising co-expressing a recombinant fusion protein and *Neisseria meningitidis* O-oligosaccharyltransferase PglL in an O-antigen ligase gene-defective bacterium, and linking a polysaccharide endogenous or exogenous for the bacterium to the recombinant fusion protein by the *Neisseria meningitidis* O-oligosaccharyltransferase PglL, to obtain the bacterial polysaccharide-modified recombinant fusion protein; wherein the recombinant fusion protein comprises an N-terminal signal peptide, a peptide fragment having a glycosylation site of *Neisseria meningitidis* O-oligosaccharyltransferase PglL, which is the serine at position 63 starting from the N-terminus of *Neisseria meningitidis* pilin PilE, and a carrier protein; the carrier protein is a nontoxic mutant of *Pseudomonas aeruginosa* exotoxin A or a fragment of a bacterial toxin protein, wherein the fragment of a bacterial toxin protein is cholera toxin B subunit or fragment C of tetanus toxin; the peptide fragment having a glycosylation site of *Neisseria meningitidis* O-oligosaccharyltransferase PglL is a peptide fragment comprising at least the amino acids from positions 55 to 66 starting from N-terminus of *Neisseria meningitidis* pilin PilE.

The peptide fragment having a glycosylation site of *Neisseria meningitidis* O-oligosaccharyltransferase PglL is located at N-terminus or C-terminus of the carrier protein. The amino acid sequence of the *Neisseria meningitidis* O-oligosaccharyltransferase PglL is set forth in SEQ ID No.26, or a mutant thereof having a similar function. The bacterial polysaccharide-modified recombinant fusion protein is a recombinant fusion protein modified by a polysaccharide endogenous for the bacterium or a recombinant fusion protein modified by a polysaccharide exogenous for the bacterium. Due to the deficiency in O-antigen ligase gene, O-antigen polysaccharide cannot be ligated to lipoid A-core oligosaccharide, and therefore lipopolysaccharide cannot be formed.

In said method, the signal peptide may be a signal peptide such as PelB, DsbA, STII, OmpA, PhoA, LamB, SpA, and Enx. In the invention, DsbA signal peptide is used, and has an amino acid sequence from positions 1 to 19 starting from N-terminus of SEQ ID No.32.

According to the invention, the glycosylation site of *Neisseria meningitidis* O-oligosaccharyltransferase PglL is the serine at position 63 starting from N-terminus of *Neisseria meningitidis* pilin PilE, and the peptide fragment having the glycosylation site of Neisseria meningitidis O-oligosaccharyltransferase PglL is a peptide fragment of *Neisseria meningitidis* pilin PilE comprising the serine at position 63 starting from N-terminus of *Neisseria meningitidis* pilin PilE; and further comprising at least the amino acids from positions 55 to 66 starting from N-terminus of *Neisseria meningitidis* pilin PilE; more particularly, the peptide fragment is any one of: (1) the peptide fragment set forth in the amino acid sequence from positions 128 to 156 starting from N-terminus of SEQ ID No.32; (2) the peptide fragment set forth in the amino acid sequence from positions 128 to 154 starting from N-terminus of SEQ ID No.34; (3) the peptide fragment set forth in the amino acid sequence from positions 128 to 152 starting from N-terminus of SEQ ID No.36; (4) the peptide fragment set forth in the amino acid sequence from positions 128 to 150 starting from N-terminus of SEQ ID No.38; (5) the peptide fragment set forth in the amino acid sequence from positions 22 to 40 starting from N-terminus of SEQ ID No.48; (6) the peptide fragment set forth in the amino acid sequence from positions 22 to 36 starting from N-terminus of SEQ ID No.50.

The nontoxic mutant of *Pseudomonas aeruginosa* exotoxin A has an amino acid sequence from positions 20 to 631 starting from N-terminus of SEQ ID No.46. The cholera toxin B subunit has an amino acid sequence from positions 20 to 122 starting from N-terminus of SEQ ID No.32. The fragment C of tetanus toxin has an amino acid sequence from positions 20 to 455 starting from N-terminus of SEQ ID No.60.

In any of the above methods, the amino acid sequence of the recombinant fusion protein is any one of: (1) the amino acid sequence set forth in SEQ ID No.32; (2) the amino acid sequence set forth in SEQ ID No.34; (3) the amino acid sequence set forth in SEQ ID No.36; (4) the amino acid sequence set forth in SEQ ID No.38; (6) the amino acid sequence set forth in SEQ ID No.46; (7) the amino acid sequence set forth in SEQ ID No.48; (8) the amino acid sequence set forth in SEQ ID No.50; (10) the amino acid sequence set forth in SEQ ID No.56; (11) the amino acid sequence set forth in SEQ ID No.58; and (12) the amino acid sequence set forth in SEQ ID No.60.

In any of the above methods, the recombinant fusion protein is introduced into the bacterium by a recombinant expression vector, and the recombinant expression vector is obtained by inserting a gene encoding the recombinant fusion protein into the multiple cloning site of pMMB66EH. The gene encoding the recombinant fusion protein is as follows: (1) when the recombinant fusion protein has the amino acid sequence set forth in SEQ ID No.32, its coding gene is set forth in SEQ ID No.31; (2) when the recombinant fusion protein has the amino acid sequence set forth in SEQ ID No.34, its coding gene is set forth in SEQ ID No.33; (3) when the recombinant fusion protein has the amino acid sequence set forth in SEQ ID No.36, its coding gene is set forth in SEQ ID No.35; (4) when the recombinant fusion protein has the amino acid sequence set forth in SEQ ID No.38, its coding gene is set forth in SEQ ID No.37; (6) when the recombinant fusion protein has the amino acid sequence set forth in SEQ ID No.46, its coding gene is set forth in SEQ ID No.45; (7) when the recombinant fusion protein has the amino acid sequence set forth in SEQ ID No.48, its coding gene is set forth in SEQ ID No.47; (8) when the recombinant fusion protein has the amino acid sequence set forth in SEQ ID No.50, its coding gene is set forth in SEQ ID No.49; (10) when the recombinant fusion protein has the amino acid sequence set forth in SEQ ID No.56, its coding gene is set forth in SEQ ID No.55; (11) when the recombinant fusion protein has the amino acid sequence set forth in SEQ ID No.58, its coding gene is set forth in SEQ ID No.57; (12) when the recombinant fusion protein has the amino acid sequence set forth in SEQ ID No.60, its coding gene is set forth in SEQ ID No.59. The multiple cloning site is *Eco*RI and *Hin*dIII site.

In any of the above methods, the *Neisseria meningitidis* O-oligosaccharyltransferase PglL is introduced into the bacterium by a recombinant expression vector, and the recombinant expression vector is obtained by inserting an expression cassette of the *Neisseria meningitidis* O-oligosaccharyltransferase PglL into the multiple cloning site of pET28a(+). The expression cassette of the *Neisseria meningitidis* O-oligosaccharyltransferase PglL is set forth in SEQ ID No.30. The multiple cloning site is *Bgl*II site.

In any of the above methods, an exogenous polysaccharide is introduced into the bacterium by a recombinant expression vector comprising a gene cluster for polysaccharide synthesis. The gene cluster for polysaccharide synthesis is set forth in SEQ ID No.79. The recombinant expression vector comprising the gene cluster for polysaccharide synthesis is prepared by the following method: subjecting the DNA molecule set forth in SEQ ID No.79 to double enzyme digestion by *Asc*I and *Not*I, to obtain a gene fragment; subjecting the DNA molecule set forth in SEQ ID No.82 to double enzyme digestion by *Asc*I and *Not*I, to obtain a large fragment; and ligating the gene fragment to the large fragment to obtain the recombinant expression vector.

In any of the above methods, the O-antigen ligase gene-defective bacterium is O-antigen ligase gene-defective *Shigella flexneri,* O-antigen ligase gene-defective *Salmonella paratyphi* A or O-antigen ligase gene-defective *Escherichia coli.* Preferably, the O-antigen ligase gene-defective *Escherichia coli* is a strain *of E. coli* K12.

In any of the above methods, when the bacterium is *Shigella flexneri,* the method of co-expressing the recombinant fusion protein and *Neisseria meningitidis* O-oligosaccharyltransferase PglL in an O-antigen ligase gene-defective bacterium comprises introducing an expression vector encoding the recombinant fusion protein and an expression vector encoding the *Neisseria meningitidis* O-oligosaccharyltransferase PglL into an O-antigen ligase gene-defective *Shigella flexneri,* and inducing the expression thereof. The *Shigella flexneri* is a large virulence plasmid-deleted *Shigella flexneri.* The method for preparing a large virulence plasmid-deleted *Shigella flexneri* is as follows: introducing pKD*inc* into the *Shigella flexneri,* deleting the large virulence plasmid based on the plasmid incompatibility, and then removing pKD*inc* based on temperature-sensitive characteristic of pKD*inc.* The O-antigen ligase gene is *waaI* gene. The bacterial polysaccharide-modified recombinant fusion protein is a bacterial polysaccharide-modified recombinant fusion protein obtained by linking O-polysaccharide endogenous for *Shigella flexneri* to the recombinant fusion protein by *Neisseria meningitidis* O-oligosaccharyltransferase PglL.

In any of the above methods, when the bacterium is *Salmonella paratyphi* A, the method of co-expressing the recombinant fusion protein and *Neisseria meningitidis* O-oligosaccharyltransferase PglL in an O-antigen ligase gene-defective bacterium comprises introducing an expression vector encoding the recombinant fusion protein and an expression vector encoding the *Neisseria meningitidis* O-oligosaccharyltransferase PglL into an O-antigen ligase gene-defective *Salmonella paratyphi* A, and inducing the expression thereof. The O-antigen ligase gene is *waaL* gene. The bacterial polysaccharide-modified recombinant fusion protein is a bacterial polysaccharide-modified recombinant fusion protein obtained by linking O-polysaccharide endogenous for *Salmonella paratyphi* A to the recombinant fusion protein by the *Neisseria meningitidis* O-oligosaccharyltransferase PglL.

In any of the above methods, when the bacterium is a strain of *E*. *coli* K12, the method of co-expressing the recombinant fusion protein and *Neisseria meningitidis* O-oligosaccharyltransferase PglL in an O-antigen ligase gene-defective bacterium comprises introducing an expression vector encoding the recombinant fusion protein, an expression vector encoding the *Neisseria meningitidis* O-oligosaccharyltransferase PglL, and an expression vector comprising a gene cluster for polysaccharide synthesis fragment into an O-antigen ligase gene-defective strain of *E*. *coli* K12, and inducing the expression thereof. The O-antigen ligase gene is *waaL* gene. The strain of *E*. *coli* K12 is unable to synthesize endogenous O-antigen, as the gene encoding the key glycosyltransferase (i.e., rhamnosyltransferase) in the O-antigen synthesis pathway, *wbbL,* is naturally inactivated. The bacterial polysaccharide-modified recombinant fusion protein is a bacterial polysaccharide-modified recombinant fusion protein obtained by linking a polysaccharide exogenous for the bacterium to the recombinant fusion protein by *Neisseria meningitidis* O-oligosaccharyltransferase PglL. The exogenous polysaccharide is obtained by introducing an expression vector comprising a gene cluster for polysaccharide synthesis into the strain of *E*. *coli* K12. The gene cluster for polysaccharide synthesis is set forth in SEQ ID No.79. The expression vector comprising the gene cluster for polysaccharide synthesis is prepared by the following method: subjecting the DNA molecule set forth in SEQ ID No.79 to double enzyme digestion by *Asc*I and *Not*I, to obtain a gene fragment; subjecting the DNA molecule set forth in SEQ ID No.82 to double enzyme digestion by *Asc*I and *Not*I, to obtain a large fragment; and ligating the gene fragment to the large fragment to obtain the expression vector.

Preferably, any of the above methods further comprises the steps of cell lysis, centrifugation and collection of supernatant, desalting and/or separation and purification, after the expression. The separation and purification is conducted by ion exchange chromatography and/or molecular sieve chromatography.

The bacterial polysaccharide-modified recombinant fusion protein prepared by any of the methods according to the appended claims also falls into the protection scope of the invention.

The invention further seeks to protect a vaccine prepared from the bacterial polysaccharide-modified recombinant fusion protein prepared by any of the methods according to the appended claims. The vaccine can induce generation of antibodies against O-antigen polysaccharides in animal. Preferably, the vaccine comprises an aluminium hydroxide adjuvant.

The bacterial polysaccharide-modified recombinant fusion protein prepared by any of the methods according to the appended claims for use in inducing generation of antibodies against O-antigen polysaccharides in animal, also falls into the protection scope of the invention. Preferably, the product is a vaccine.

Preferably, the bacterium is *Shigella flexneri, Salmonella paratyphi* A or *Escherichia coli.*

Preferably, the vaccine comprises an aluminium hydroxide adjuvant.

The bacterial polysaccharide-modified recombinant fusion protein prepared by any of the methods according to the appended claims for use in preventing and/or treating a disease caused by a bacterium, also falls into the protection scope of the invention. Preferably, the product is a vaccine. Preferably, the bacterium is *Shigella flexneri, Salmonella paratyphi* A or *Escherichia coli.* Preferably, the vaccine comprises an aluminium hydroxide adjuvant.

In the invention, in an O-antigen ligase gene-defective host bacterium, *Neisseria meningitidis* O-oligosaccharyltransferase PglL gene and a recombinant fusion protein gene are co-expressed; or in a host bacterium defective in both an O-antigen ligase gene and O-antigen synthesis gene, *Neisseria meningitidis* O-oligosaccharyltransferase PglL gene, a recombinant fusion protein gene, and a gene cluster for synthesis of exogenous bacterial polysaccharide are co-expressed, to obtain a bacterial polysaccharide-modified recombinant fusion protein.

### Description of Drawings

Fig. 1 shows the results of PCR identification of *S. flexneri* 2a 301ΔpCP.
Fig. 2 shows the results of PCR identification of *S. flexneri* 2a 301ΔpCPΔ*waaI*.
Fig. 3 shows the verification results of lipopolysaccharide synthesis deficiency in S. *flexneri* 2a 301ΔpCPΔ*waaI* by silver staining.
Fig. 4 shows the tertiary structure of *Neisseria meningitidis* pilin PilE.
Fig. 5 shows the detection result of glycosylation of the recombinant CTB fusion protein by WB assay.
Fig. 6 shows the detection result of glycosylation of the recombinant EPA fusion protein by WB assay.
Fig. 7 shows the purification result of the glycoprotein rCTB4573-OPS_{*Sf*301} detected by SDS-PAGE and WB assay.
Fig. 8 shows the purification result of the glycoprotein rEPA4573-OPS_{*Sf*301} detected by SDS-PAGE and WB assay.
Fig. 9 shows the immunogenicity evaluation of the glycoproteins.
Fig. 10 shows the detection result of glycosylation of the recombinant fusion proteins having multiple glycosylation sites by WB assay.
Fig. 11 shows the result of WB assay for the glycoprotein rTTC4573-OPS_{*Sf*301}.
Fig. 12 shows the results of PCR identification of *S. paratyphi* CMCC50973Δ*waaL*.
Fig. 13 shows the verification result of lipopolysaccharide synthesis deficiency in S. *paratyphi* CMCC50973Δ*waaL* by silver staining.
Fig. 14 shows the result of WB assay for glycoprotein rEPA4573-OPS_{*Spty*50973}.
Fig. 15 shows the result of WB assay for the glycoprotein rCTB4573-OPS_{*Spty*50973}.
Fig. 16 shows the result of PCR identification *of E. coli* W3110Δ*waaL.*
Fig. 17 shows the result of WB assay for the glycoprotein rCTB4573-OPS_{*Ec*O157}.

### Specific modes for carrying out the Invention

Unless otherwise specified, the experimental methods used in the following examples are the conventional methods. Unless otherwise specified, the materials, reagents and the like used in the following examples are commercially available. pMD18-T was purchased from TaKaRa Company, with a catalog number of D101A. Plasmid pET-22b was purchased from Novagen Company, with a catalog number of 69744. pET28a(+) was purchased from Novagen. pMMB66EH was purchased from ATCC, under an accession number of ATCC 37620. Anti-His tag mouse monoclonal antibody was purchased from Sigma, with a catalog number of A7058. Anti-EPA antibody was purchased from Sigma, with a catalog number of P2318. Rabbit Anti- OPS_{*Sf*301} antiserum (*Shigella flexneri* 2a type antiserum) was purchased from DENKA SEIKEN Co., Ltd, with a catalog number of 210227. Rabbit anti-E. coli 0157 antiserum was purchased from DENKA SEIKEN Co., Ltd, with a catalog number of 210753. HRP-goat anti-rabbit IgG was purchased from TransGen Company, with a catalog number of HS-101-01. CHELATING SEPH 6 FF affinity chromatographic column was purchased from GE Healthcare, with a catalog number of 17-5203-06. G25 chromatographic packing was purchased from GE Healthcare. ProteinPak DEAE8HR cation exchange chromatography column was purchased from Waters. Superdex 75 FPLC chromatographic column was purchased from GE Healthcare, with a catalog number of 17-1047-01. Female Balb/c mice were purchased from Laboratory Animal Center of the Academy of Military Medical Sciences. The aluminium hydroxide adjuvant Rehydragel LV was purchased from General Chemical Company.

The recombinant plasmid pKD*inc*, which has been disclosed in the paper "Global Analysis of a Plasmid-Curred Shigella flexneri Strain: New Insights into the Interaction between the Chromosome and a Virulence Plasmid, Journal of Proteome Research, 2010, 9(2):843-854", and is available to the public by Biological Engineering Institute of Academy of Military Medical Sciences, is a temperature-sensitive plasmid resistant to ampicillin. The plasmid pKOBEG, which has been disclosed in the paper "A rapid method for efficient gene replacement in the filamentous fungus Aspergillus nidulans[J]. Nucleic Acids Res. 2000 Nov 15;28(22):E97", and is available to the public by Biological Engineering Institute of Academy of Military Medical Sciences, is a temperature-sensitive plasmid resistant to chloramphenicol. The plasmid pCP20, which has been disclosed in the paper "Datsenko, K.A. and B.L. Wanner .One-step inactivation of chromosomal genes in Escherichia coli K-12 using PCR products[J]. Proc.natl.Acad.Sci.U.S.A, 2000,97(12): 6640-6645", and is available to the public by Biological Engineering Institute of Academy of Military Medical Sciences, is resistant to chloramphenicol. pKD3 has been disclosed in "Datsenko, K.A. and B.L. Wanner, One-step inactivation of chromosomal genes in Escherichia coli K-12 using PCR products. Proc Natl Acad Sci U S A, 2000. 97(12): p. 6640-5. ", and is available to the public by Biological Engineering Institute of Academy of Military Medical Sciences. The plasmid pKD46 has been disclosed in the paper "Datsenko, K.A. and B.L. Wanner, One-step inactivation of chromosomal genes in Escherichia coli K-12 using PCR products. Proc Natl Acad Sci U S A, 2000. 97(12): p. 6640-5. ", and is available to the public by Biological Engineering Institute of Academy of Military Medical Sciences. The replicon of pKD46 is sensitive to temperature, and is lost when culturing at 37°C, and the plasmid comprises genes encoding the three recombinases of Red recombination system and controlled by arabinose promoter. pACYC184 has been disclosed in the paper "Yu Mei, Zhou Jianguang, Chenwei, et al., Construction of a mobile recombineering system pYM-Red [J]. PROGRESS IN BIOCHEMISTRY AND BIOPHYSICS, 2005, 32(4): 359-364.", and is available to the public by Biological Engineering Institute of Academy of Military Medical Sciences. *Shigella flexneri* 2a 301 wild-type strain (*S. flexneri* 2a 301) has been disclosed in the paper "Genome sequence of Shigella flexneri 2a: insights into pathogenicity through comparison with genomes of Escherichia coli K12 and 0157, Nucl. Acids Res 2002, 30(20):4432-4441", and is available to the public by Biological Engineering Institute of Academy of Military Medical Sciences. *Salmonella paratyphi* A 50973 strain (*S. paratyphi* CMCC50973) was purchased from National Center for Medical Culture Collections. *E*. *coli* W3110 has been disclosed in the paper "Yu Mei, Li Shanhu, Chen Wei, et al., Reconstruction of plasmid DNA with Red-mediated homologous recombination combined with restriction digestion [J]. Bulletin of the Academy of Military Medical Sciences, 2005, 29(3): 241-243.", and is available to the public by Biological Engineering Institute of Academy of Military Medical Sciences.

### Example 1. Preparation of Shigella flexneri O-glycosylated recombinant fusion protein and a vaccine thereof by one-step bioconjugate method

*Shigella spp.* is a highly infectious Gram-negative pathogenic enterobacterium, which generally invades the epithelial cells of human colon and is finally located in large intestine, resulting in typical bacillary dysentery (fever, bellyache, tenesmus, and purulent and bloody stool). The large virulence plasmid is the primary pathogenic factor, and the large virulence plasmid comprises about 32 virulence-associated genes, mainly including *mxi-spa* gene associated with type III secretion system, and virulence genes closely associated with the invasion of bacteria into epithelia, such as *ipaBCD* and *ipgC.* In order to develop it into a safe host bacterium, the large plasmid pCP encoding virulent factors has to be deleted first, and then make it be defective in O-antigen ligase gene *waaI*, so as to develop a host bacterium suitable for the invention.

### I. Host bacterium engineering

### (I) Construction of large virulence plasmid-deficient strain S. flexneri 2a 301ΔpCP

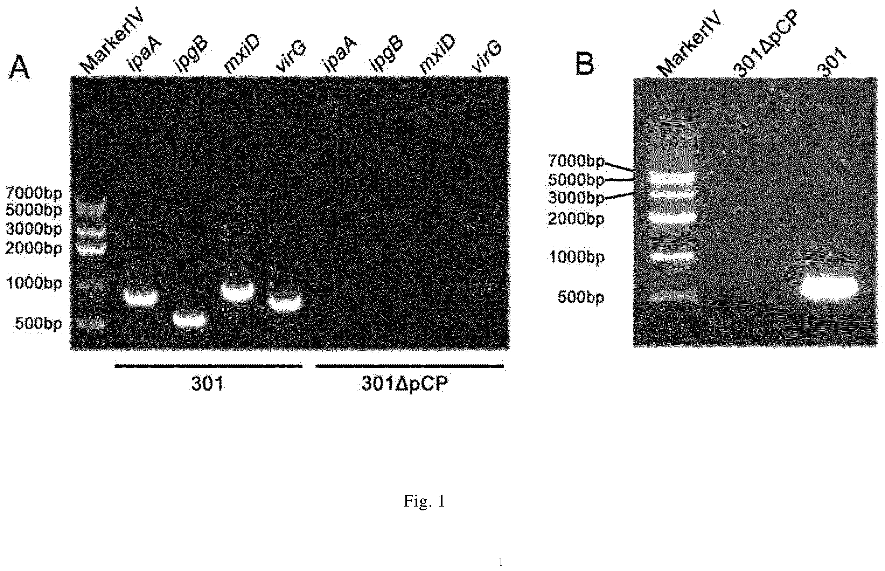

1. Preparation of competent cells of *Shigella flexneri* 2a 301 wild-type strain
   1) *Shigella flexneri* 2a 301 wild strain (*S.flexneri* 2a 301) was seeded in a LB liquid medium. After culturing at 30°C overnight, 1ml culture medium was transferred to 100mL low salt LB liquid medium, and the culture was carried out at 30°C until OD₆₀₀ reached 0.6.
   2) The bacteria were collected by centrifugation, and washed with a sterilized aqueous solution containing 10%(v/v) glycerol for four times. Finally, the bacteria were re-suspended in 400µL sterilized aqueous solution containing 10%(v/v) glycerol, to obtain *S. flexneri* 2a 301 competent cells for use in electrotransformation, and were sub-packaged for later use.
2. Deletion of large virulence plasmid
   1) *inc* was a sequence fragment associated with replication in large virulence plasmid pCP of *Shigella spp.,* and the recombinant plasmid pKD*inc* carrying the *inc* fragment was transformed into the *S. flexneri* 2a 301 competent cells prepared in Step 1 by electroporation, to obtain the intermediate recombinant bacterium, designated as *S. flexneri* 2a 301/pKD*inc*.
   2) The *S. flexneri* 2a 301/pKD*inc* was passaged serially for more than three times in LB liquid medium containing ampicillin at a concentration of 50µg/mL, to ensure the loss of large virulence plasmid, and then the cultured bacterial liquid was spread on the ampicillin-resistant LB solid medium. When monoclonal colonies were formed, the genomic DNA of the monoclonal colonies was extracted, and was used as template to carry out PCR amplification, with the primers of *ipaA, ipgB*, *mxiD*, *virG* as primers, respectively. Whether large virulence plasmid pCP was deleted or not was determined by detection of virulence genes *ipaA, ipgB*, *mxiD*, and *virG*, and meanwhile the genomic DNA of *S. flexneri* 2a 301 was used as control to carry out said PCR amplification.
      The primers *of ipaA* are as follows:
         ipaAp1:5'- AAGATTCTGCCTTTGGACC-3'(SEQ ID No.1)
         ipaAp2: 5'- GTGGTTGAAGAGTTCTGTATG-3' (SEQ ID No.2)
      The primers *of ipgB* are as follows:
         ipgB1U: 5'-TGCTTTGACGGTATACAGC-3' (SEQ ID No.3)
         ipgB1L: 5'-ACTTCCACAGGTTGAATTCG-3' (SEQ ID No.4)
      The primers *of mxiD* are as follows:
         mxiDU: 5'-AAGCAGGTTTCTTCTATTGG-3' (SEQ ID No.5)
         mxiDL: 5'-GAACACATTACCGATTACAGG-3' (SEQ ID No.6)
      The primers of *virG* are as follows:
         VirGp1: 5'-CATCAATCCGTTACTCACT-3' (SEQ ID No.7)
         VirGp2: 5'-ACTACCAGCAACAATACG-3' (SEQ ID No.8)
      The results are shown in Fig. 1A. In Fig. 1A, 301 represents *Shigella flexneri* 2a 301 wild-type strain *S. flexneri* 2a 301; and 301ΔpCP represents the monoclonal colonies screened in Step 2). Fig. 1A shows that in the *Shigella flexneri* 2a 301 wild-type strain, the target amplified fragments appeared for the genes *ipaA, ipgB*, *mxiD*, *virG*, which were the target fragment of 888bp for *ipaA,* the target fragment of 595bp for *ipgB*, the target fragment of 986bp for *mxiD*, and the target fragment of 777bp for *virG*, respectively. However, in the monoclonal colonies screened for ampicillin-resistance, no amplified fragments appeared for genes *ipaA, ipgB*, *mxiD*, *virG*, indicating successful loss of large virulence plasmid. The monoclonal colonies screened in the Step 2) (based on the plasmid incompatibility, the recombinant bacteria had the large virulence plasmid knocked out and only retained the plasmid pKD*inc*) were designated as *S. flexneri* 2a 301ΔpKD*inc*.
   3) By the utilization of the temperature sensitivity of the plasmid pKD*inc*, *S.flexneri* 2a 301ΔpCP/pKD*inc* was subjected to continuous culture for two generations in liquid LB medium free of antibiotics at 42°C, and the bacterial strain, which could grow in LB solid medium free of antibiotics, but not in ampicillin-containing LB solid medium, was designated as the bacterial strain *S.flexneri* 2a 301ΔpCP.
      The genomic DNA of *S. flexneri* 2a 301ΔpCP was extracted, the virulence genes *ipaA, ipgB*, *mxiD*, *virG* were detected in accordance with the method in Step 2), and meanwhile the genomic DNA of the *Shigella flexneri* 2a 301 wild-type strain *S. flexneri* 2a 301 was used as control to carry out the PCR amplification. The results were consistent with the results in Fig. 1A. In the *Shigella flexneri* 2a 301 wild-type strain *S. flexneri* 2a 301, the target amplified fragments appeared for the genes *ipaA, ipgB*, *mxiD*, *virG*, while in the *S. flexneri* 2a 301ΔpCP, no amplified fragments appeared for the genes *ipaA, ipgB*, *mxiD*, *virG*, indicating the absence of large virulence plasmid.
      The genomic DNA of the *S. flexneri* 2a 301ΔpCP was extracted, and the primers of *inc* were used as primers to carry out PCR amplification. Whether the plasmid pKD*inc* was succesfully deleted or not was confirmed by detection of the *inc* fragment in the plasmid pKD*inc*, and meanwhile the genomic DNA of *Shigella flexneri* 2a 301 wild-type strain *S. flexneri* 2a 301 was used as control to carry out the PCR amplification.
      The primers *of inc* are as follows:
         incF: 5'-TGCGAGAGAGAGGGGATAAC-3' (SEQ ID No.9)
         incR: 5'- CGCCTTTTCCATCAGTTTC-3' (SEQ ID No.10)
      The results are shown in Fig. IB. In Fig. 1B, 301 represents *Shigella flexneri* 2a 301 wild-type strain *S. flexneri* 2a 301; 301ΔpCP represents *S. flexneri* 2a 301ΔpCP. Fig. 1B shows that in the *Shigella flexneri* 2a 301 wild-type strain *S. flexneri* 2a 301, the target fragment of 488bp appeared for *inc* gene, while in the *S. flexneri* 2a 301ΔpCP, no target amplified fragment appeared, indicating successful loss of the plasmid pKD*inc*.
      The above results show that the *S. flexneri* 2a 301ΔpCP had the large virulence plasmid pCP lost, and also had the exogenous plasmid pKD*inc* lost, i.e., the large virulence plasmid-deleted strain *S. flexneri* 2a 301ΔpCP was constructed successfully.

### (II) The preparation of O-antigen ligase gene waaI-defective avirulent Shigella flexneri S. flexneri 2a 301ΔpCPΔwaaI

### 1. Preparation of linear targeting DNA fragments

1) Design and synthesis of PCR primers
A pair of primers were designed for each of the upstream (5'end) and the downstream (3'end) of *Shigella flexneri* 2a 301*waaI* gene, respectively, i.e., 301waaIu5/301waaIu3 and 301waaId5/301waaId3; wherein 301waaIu5 had the restriction enzyme cleavage site for *Bam*HI, 301waaIu3 had the restriction enzyme cleavage site for *Sal*I, 301waaId5 had the restriction enzyme cleavage site for *Hin*dIII, and 301waaId3 had the restriction enzyme cleavage site for *Xho*I.
In order to confirm whether the mutant was constructed successfully, a pair of primers (301waaIw5/301waaIw3) located outside the upstream and downstream homologous arms of *waaI* gene in the genome, a pair of inner primers for identification (301waaIn5/301waaIn3), a pair of primers for *kan* gene (Kan5/Kan3) and a pair of inner primers for *kan* gene (Kanf5/Kanf3), were designed to carry out PCR verification, and sequencing verification.
The primers are shown in Table 1 (in Table 1, the underlined sequences refer to the enzyme recognition sites).

**Table 1 Primer sequence listing 1**

| Primer | Sequence (5'→3') | length/bp | Use |
|---|---|---|---|
| 301waaIu5 | CGGGATCCGGGTATGGGAAGAATCAAG(SEQ ID No.11) | 628 | for amplification of up fragment |
| 301waaIu3 | GCGTCGACTCAGAAATGCTACGGTGT(SEQ ID No.12) | | |
| 301waaId5 | CCAAGCTTTGGACCTTTAGACAATCAA(SEQ ID No.13) | 680 | for amplification of down fragment |
| 301waaId3 | CCCTCGAGATGTTAGGAAGCATACCG(SEQ ID No.14) | | |
| 301waaIn5 | TGGGTGGGATTACAAGGT(SEQ ID No.15) | 562 | for identification of the mutant strain |
| 301waaIn3 | CCAATGACTAACACGGAAA(SEQ ID No.16) | | |
| 301waaIw5 | CTACAGATGCTGGCGAATA(SEQ ID No.17) | 1777 | for identification of the mutant strain |
| 301waaIw3 | TCACTACAGTTGGGATGG(SEQ ID No.18) | | |
| Kanf5 | AGCCGATTGTCTGTTGTGCC(SEQ ID No.19) | 1600 | for identification of the mutant strain |
| Kanf3 | TTGTCACTGAAGCGGGAAGG(SEQ ID No.20) | | |
| Kan5 | GCGTCGACGTGTAGGCTGGAGCTGCTTC(SEQ ID No.21) | | for identification of the mutant strain |
| Kan3 | CCAAGCTTATGGGAATTAGCCATGGTCC(SEQ ID No.22) | | |

2) Construction of linear targeting DNA fragments
(1) The genomic DNA of *S. flexneri* 2a 301ΔpCP was used as template, and 301waaIu5/301waaIu3 and 301waaId5/301waaId3 were used to amplify the upstream and downstream homologous arms of *waaI*, i.e., up and down, respectively.
   PCR program was as followed: 94°C 10min; 94°C 30s, 56°C 30s, 72°C 1min, 30 cycles; 72°C 10min.
   The nucleotide sequence of the up fragment obtained by PCR amplification was set forth in the nucleotide sequence from positions 7 to 634 starting from 5'end of SEQ ID No.23, and the nucleotide sequence of the down fragment was set forth in the nucleotide sequence from positions 2143 to 2822 starting from 5'end of SEQ ID No.23.
(2) Construction of pETKan
   The DNA molecule set forth in the nucleotide sequence from positions 641 to 2136 starting from 5'end of SEQ ID No.23 was used as template, with 5'-GCGTCGACGTGTAGGCTGGAGCTGCTTC-3'(SEQ ID No.24) and 5'-CCAAGCTTATGGGAATTAGCCATGGTCC-3'(SEQ ID No.25) as primers, to carry out PCR amplification, to obtain a PCR amplification product;
   The PCR amplification product was subjected to double enzyme digestion by *Saf*I and *Hin*dIII to obtain gene fragments; the plasmid pET-22b was subjected to double enzyme digestion by *Saf*I and *Hin*dIII to obtain a large fragment; the gene fragment was ligated to the large fragment, to obtain a recombinant plasmid, designated as pETKan. The pETKan was sequenced, and the result was correct.
(3) The up fragment was subjected to double enzyme digestion by *Bam*HI and *Sal*I, to obtain the gene fragment 1; the plasmid pETKan was subjected to double enzyme digestion by *Bam*HI and *Sal*I, to obtain the large fragment 1; and the gene fragment 1 was ligated to the large fragment 1, to obtain the intermediate vector 1;
   The down fragment was subjected to double enzyme digestion by *Hin*dIII and *Xho*I, to obtain the gene fragment 2; the intermediate vector 1 was subjected to double enzyme digestion by *Hin*dIII and *Xho*I, to obtain the large fragment 2; and the gene fragment 2 was ligated to the large fragment 2, to obtain the intermediate vector 2.
(4) The intermediate vector 2 was subjected to double enzyme digestion by *Bam*HI and *Xho*I, to obtain the targeting fragment of interest, as set forth in SEQ ID No.23. The flanking sequences of the targeting fragment are the homologous arms, and the *kan* gene was comprised in the middle region. The nucleotide sequence from positions 7 to 634 starting from 5' end of SEQ ID No.23 refer to the up fragment, the nucleotide sequence from positions 641 to 2136 starting from 5' end of SEQ ID No.23 refer to the kan gene, and the nucleotide sequence from positions 2143 to 2822 starting from 5' end of SEQ ID No.23 refer to the down fragment.

### 2. Construction of S. flexneri 2a 301ΔpCP/pKOBEG

Since the plasmid pKOBEG comprised the genes encoding the enzymes necessary for λ-Red recombination system, the plasmid pKOBEG was transformed into *S. flexneri* 2a 301ΔpCP by electroporation, and the transformed cells were spread onto the LB-plate containing chloramphenicol at a concentration of 50µg/mL, and cultured at 30°C overnight, to obtain the positive clone, designated as *S. flexneri* 2a 301ΔpCP/pKOBEG strain.

### 3. Electrotransformation of S. flexneri 2a 301ΔpCP/pKOBEG with the linear targeting DNA fragments

1) The *S.flexneri* 2a 301ΔpCP/pKOBEG was seeded in low salt LB liquid medium containing chloramphenicol at a concentration of 30µg/mL, cultured at 30°C overnight, and then passaged in low salt LB liquid medium at a volume ratio of 1:100 for furture culture.
2) When the OD₆₀₀ value of the culture medium in Step 1) reached 0.2, and L-arabinose was added at a final concentration of 1 mmol/L to induce the expression of Red recombination system.
3) When the OD₆₀₀ of the culture medium in Step 2) reached 0.6, the *S. flexneri* 2a 301ΔpCP/pKOBEG was transformed with the targeting fragments as prepared in Step 1 at a concentration of 300 ng/µL by electroporation, to obtain the transformed cells.
4) To the transformed cells, 1 mL precooled low salt LB liquid medium was added quickly. The cells were thawed at 30°C for about 2.5 h, and then were spread onto a LB plate containing kanamycin at a concentration of 50µg/mL, cultured in a 30°C incubator overnight, to screen out the positive clone.
5) The positive clone was seeded to LB liquid medium containing kanamycin at a concentration of 50µg/mL, and cultured at 42°C and passaged twice (12 h for each passage), thereby resulting in the deletion of the plasmid pKOBEG. The kanamycin-resistant, *waaI*-deleted mutant strain was obtained finally, designated as *S.flexneri* 2a 301ΔpCP *waaI::kan*1*.*

4. The plasmid pCP20 encoding a FRT site-specific recombinase was transformed into *S. flexneri* 2a 301ΔpCP *waaI::kan* by means of electroporation, and cultured in kanamycin-free LB plate containing chloramphenicol at a concentration of 50µg/mL at 30°C. The positive clone of Cm^{r}Km^{s} (chloramphenicol-resistant, kanamycin-sensitive) was screened.

5. The positive clone screened in Step 4, was transferred to liquid LB, and cultured at 42°C for 12 h, to obtain the O-antigen ligase gene *waaI*-defective, avirulent *Shigella flexneri* 2a 301, designated as *S. flexneri* 2a 301ΔpCPΔ*waaI*.
(the replicon of pCP20 was sensitive to temperature, and lost when cultured at 42°C; the plasmid comprised the DNA encoding FLP recombinase, controlled by temperature-sensitive promoter, and the expression of FLP recombinase was induced when cultured at 42°C, with the deletion of the plasmid.)

### (III) Molecular identification of S. flexneri 2a 301ΔpCPΔwaaI

The genomic DNA of *S. flexneri* 2a 301ΔpCPΔ*waaI* was used as template, and a pair of *waaI* inner primers (301waaIn5/301 waaIn3), a pair of *waaI* outer primers (301waaIw5/301waaIw3), a pair of cross primers (301waaIw5 and kanf3)and kan gene primers (kan5/kan3) were used separately to carry out PCR amplification, and meanwhile the genomic DNA of the wild-type strain *S. flexneri* 2a 301 was used as control to carry out the PCR amplification.

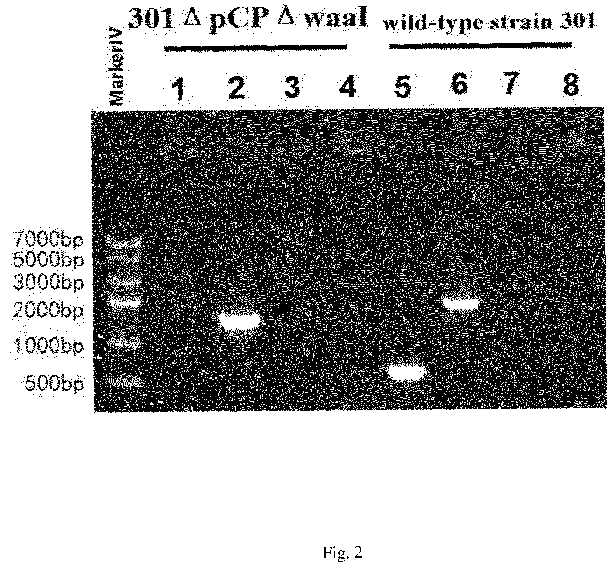

The results are shown in Fig. 2. In Fig. 2, 301ΔpCPΔwaaI represents *S. flexneri* 2a 301ΔpCPΔ*waaI*, and wild-type strain 301 represents wild-type strain *S. flexneri* 2a 301; 1,5: the PCR product when the *waaI* inner primers were used as primers; 2,6: the PCR product when the *waaI* outer primers were used as primers; 3,7: the PCR product when the *waaI*w5 and kanf3 were used as primers; 4,8: the PCR product when the Kan gene primers were used as primers. Fig. 2 shows that when the genomic DNA of *S. flexneri* 2a 301ΔpCPΔ*waaI* was used as template, and the *waaI* inner primers were used as primers to carry out PCR amplification, no target bands appeared; when the *waaI* outer primers were used as primers to carry out PCR amplification, the bands obtained were smaller than the PCR amplification bands obtained when the genomic DNA of wild-type strain was used as template; no amplification bands appeared when the cross primers and the kan gene primers were used as primers. However, when the genomic DNA of wild-type strain was used as template, and the *waaI* inner primers were used as primer to carry out PCR amplification, the target bands appeared, and no amplification bands appeared when the cross primers and the kan gene primers were used as primers.

The above results demonstrated that the *S. flexneri* 2a 301ΔpCPΔ*waaI* was *Shigella flexneri* 2a 301 that was deficient in *waaI* gene and the large virulence plasmid pCP and had no antibiotic resistance gene.

### (IV) Phenotype identification of S. flexneri 2a 301ΔpCPΔwaaI

1 mL culture of *S. flexneri* 2a 301ΔpCPΔ*waaI* cultured at 37°C overnight, was centrifuged to collect bacteria. The bacteria were washed with PBS once, and 100µL lysis buffer (the lysis buffer was prepared by well mixing 2mL 10% SDS, 0.4mL 2-mercaptoethanol, 1mL 100% glycerol, 5mL 2M pH6.8 Tris-HCl, 20 µL 10% bromophenol blue and 1.6mL ddH₂O) was added and mixed well. After heating in boiling water for 10 min, 4 µL of 20mg/mL protease K aqueous solution was added, and the reaction was carried out at 60°C for 1-2 h, to obtain lysates. 15µl of the lysates was loaded to carry out SDS-PAGE electrophoresis, the gel was placed in the fixing solution overnight, and a sensitizing solution was added and reacted for 30 min. After washing with deionized water for three times, 15 min for each time, a silver nitrate solution was added and reacted for 20 min, and deionized water was used for washing twice, 1 min for each time. A developing solution was added for color development, the reaction was stopped finally, and deionized water was used for washing.

The wild-type strain *S. flexneri* 2a 301 was also subjected to the above experiment, as control.

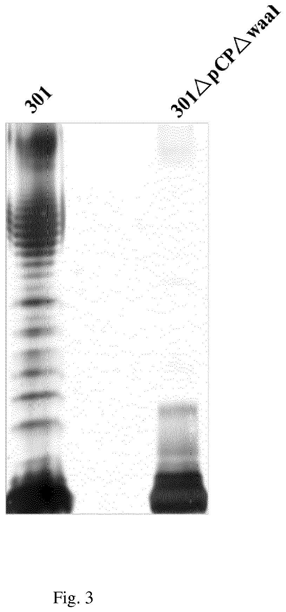

The results are shown in Fig. 3. In Fig. 3, 301ΔpCPΔwaaI represents *S. flexneri* 2a 301ΔpCPΔ*waaI*, 301 represents wild-type strain *S. flexneri* 2a 301. Fig. 3 shows that *S. flexneri* 2a 301 wild-type strain had the ladder-like bands of LPS, while *S. flexneri* 2a 301ΔpCPΔ*waaI* had no ladder-like bands of LPS due to the knockout of O-antigen ligase gene *waaI* (*waaI* functions to ligate O-antigen polysaccharide (OPS) to lipoid A-core oligosaccharide to form lipopolysaccharide(LPS)).

### II. Identification of core peptide fragments

According to the tertiary structure of *Neisseria meningitidis* pilin PilE (GeneBank: NC_003112.2), polypeptides of different lengths containing serine at position 63 (S63) of PilE (the amino acid is a glycosylation site) were prepared. To construct a series of recombinant fusion proteins, cholera toxin B subunit (CTB) (GenBank: X76390.1) was used as a carrier protein, and the polypeptides of different lengths were fused to the C-temrinus of CTB; alternatively, the nontoxic mutant of pseudomonas aeruginosa exotoxin A (rEPA) was used as a carrier protein, and the polypeptides of different lengths were fused to its N-terminus or C-terminus. The recombinant fusion protein and Neisseria meningitidis O-oligosaccharyltransferase PglL were co-expressed in S. *flexneri* 2a 301ΔpCPΔ*waaI*, whole bacterial proteins were subjected to western blot, and detected by specific antibodies, to determine the shortest polypeptide that enabled the recombinant fusion protein to be O-glycosylated. The steps were as followed:
(I) Construction of an expression vector of *Neisseria meningitidis* O-oligosaccharyltransferase PglL
   1. The amino acid sequence of *Neisseria meningitidis* O-oligosaccharyltransferase PglL (GeneBank: JN200826.1) is set forth in SEQ ID No.26, and its DNA sequence is set forth in SEQ ID No.27.
      The primers 223tac-box5':
      5'-ATCGAGATCTACTGCATAATTCGTGTCGCTCAAG-3'( SEQ ID No.28) and 223tac-box3': 5'-ATCGAGATCTGTCTCATGAGCGGATACATATTTG-3' ( SEQ ID No.29) were used for amplification to obtain the expression cassette of PglL, set forth in SEQ ID No.30.
   2. The DNA molecule set forth in SEQ ID No.30 was subjected to enzyme digestion by *Bgl*II, to obtain a gene fragment; pET28a(+)was subjected to enzyme digestion by *Bgl*II, to obtain a large fragment; the gene fragment was ligated to the large fragment, to obtain the recombinant plasmid, designated as pETtac28-*pglL*. The pETtac28-*pglL* was sequenced, and the result was correct.
(II) Construction of *S. flexneri* 2a 301 ΔpCPΔ*waaI*/pETtac28_*pglL* strain The pETtac28-*pglL* was transformed into *S. flexneri* 2a 301 ΔpCPΔ*waaI* by electroporation,
   to obtain the *S. flexneri* 2a 301 ΔpCPΔ*waaI*/pETtac28_*pglL* strain.
(III) Identification of C-terminal sequence of the core peptide fragment that enables the recombinant fusion protein to be O-glycosylated

### 1. Construction of an expression vector of recombinant CTB fusion protein

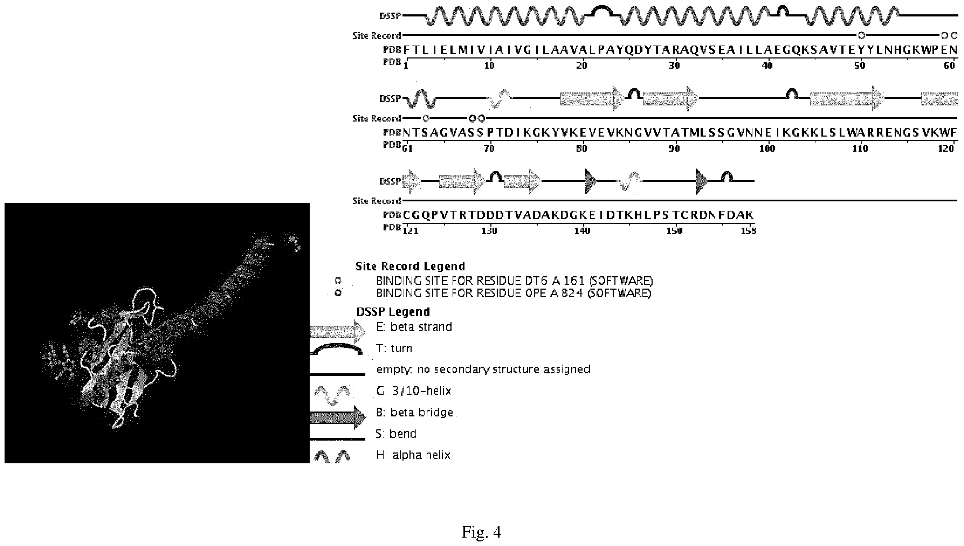

According to the tertiary structure of *Neisseria meningitidis* pilin PilE (as shown in Fig. 4), polypeptides of different lengths containing serine (S63) at position 63 of PilE were truncated, CTB was used as carrier protein, and the polypeptides of different lengths were fused to the C-terminus of CTB. The particular method was as followed:
With the N-terminus started from the serine (S45) at position 45 of PilE, and the C-terminus ended at lysine (K73) at position 73 of PilE, 29 amino acids from S45~K73 were truncated, and fused to the C-terminus of CTB; and meanwhile, with the N-terminus of the PilE peptide fragment unchanged, and the C-terminus decreased successively by 2 or 1 amino acids, i.e., the peptide fragments of S45~F71, S45~S69, S45~A67, S45~V66, S45~G65, S45~A64 were fused to the C-terminus of CTB, respectively. In order to avoid steric hindrance effect between CTB and the peptide fragment, CTB was linked to the peptide fragment via 5 amino acids. Since glycosylation occurs in periplasmic space, according to the amino acid sequence of cholera toxin B subunit (X76390.1) published on GenBank, its signal peptide (the first 21 amino acids) was replaced with DsbA signal peptide, and meanwhile the C-terminus of the recombinant fusion protein was fused to a 6×His tag, for the convenience of further detection, thereby constructing a series of recombinant CTB-recombinant fusion protein rCTB4573, rCTB4571, rCTB4569, rCTB4567, rCTB4566, rCTB4565, rCTB4564.

The sequence encoding rCTB4573 is set forth in SEQ ID No.31, wherein starting from 5' end, the sequence from positions 64 to 372 is the CTB coding sequence, the sequence from positions of 388 to 474 is the sequence encoding the amino acids from positions of 45 to 73 of PilE, and the sequence from positions of 7 to 63 is the sequence encoding DsbA signal peptide. The amino acid sequence of the protein rCTB4573 is set forth in SEQ ID No.32, wherein starting from N-terminus, the sequence from positions 20 to 122 is the CTB amino acid sequence, the sequence from positions 123 to 127 is the flexible linker, the sequence from positions 128 to 156 is the amino acids from positions 45 to 73 of PilE, the sequence from positions 157 to 166 is the flexible linker and his-tag, and the sequence from positions 1 to 19 is the DsbA signal peptide sequence.

The sequence encoding rCTB4571 is set forth in SEQ ID No.33, wherein starting from 5' end, the sequence from positions 64 to 372 is the CTB coding sequence, the sequence from positions 388 to 468 is the sequence encoding the amino acids from positions 45 to 71 of PilE, and the sequence from positions 7 to 63 is the sequence encoding DsbA signal peptide. The amino acid sequence of the protein rCTB4571 is set forth in SEQ ID No.34, wherein starting from N-terminus, the sequence from positions 20 to 122 is the CTB amino acid sequence, the sequence from positions 123 to 127 is the flexible linker, the sequence from positions 128 to 154 is the amino acids from positions 45 to 71 of PilE, the sequence from positions 155 to 163 is the flexible linker and his-tag, and the sequence from positions 1 to 19 is the DsbA signal peptide sequence.

The sequence encoding rCTB4569 is set forth in SEQ ID No.35, wherein starting from 5' end, the sequence from positions 64 to 372 is the CTB coding sequence, the sequence from positions 388 to 462 is the sequence encoding the amino acids from positions 45 to 69 of PilE, and the sequence from positions 7 to 63 is the sequence encoding DsbA signal peptide. The amino acid sequence of the protein rCTB4569 is set forth in SEQ ID No.36, wherein starting from N-terminus, the sequence from positions 20 to 122 is the CTB amino acid sequence, the sequence from positions 123 to 127 is the flexible linker, the sequence from positions 128 to 152 is the amino acids from positions 45 to 69 of PilE, the sequence from positions 153 to 161 is the flexible linker and his-tag, and the sequence from positions 1 to 19 is the DsbA signal peptide sequence.

The sequence encoding rCTB4567 is set forth in SEQ ID No.37, wherein starting from 5' end, the sequence from positions 64 to 372 is the CTB coding sequence, the sequence from positions 388 to 456 is the sequence encoding the amino acids from positions 45 to 67 of PilE, the sequence from positions 7 to 63 is the sequence encoding DsbA signal peptide. The amino acid sequence of the protein rCTB4567 is set forth in SEQ ID No.38, wherein starting from N-terminus, the sequence from positions 20 to 122 is the CTB amino acid sequence, the sequence from positions 123 to 127 is the flexible linker, the sequence from positions 128 to 150 is the amino acids from positions 45 to 67 of PilE, the sequence from positions 151 to 159 is the flexible linker and his-tag, and the sequence from positions 1 to 19 is the DsbA signal peptide sequence.

The sequence encoding rCTB4566 is set forth in SEQ ID No.39, wherein starting from 5' end, the sequence from positions 64 to 372 is the CTB coding sequence, the sequence from positions 388 to 453 is the sequence encoding the amino acids from positions 45 to 66 of PilE, and the sequence from positions 7 to 63 is the sequence encoding DsbA signal peptide. The amino acid sequence of the protein rCTB4566 is set forth in SEQ ID No.40, wherein starting from N-terminus, the sequence from positions 20 to 122 is the CTB amino acid sequence, the sequence from positions 123 to 127 is the flexible linker, the sequence from positions 128 to 149 is the amino acids from positions 45 to 66 of PilE, the sequence from positions 150 to 158 is the flexible linker and his-tag, and the sequence from positions 1 to 19 is the DsbA signal peptide sequence.

The sequence encoding rCTB4565 is set forth in SEQ ID No.41, wherein starting from 5' end, the sequence from positions 64 to 372 is the CTB coding sequence, the sequence from positions 388 to 450 is the sequence encoding the amino acids from positions 45 to 65 of PilE, and the sequence from positions 7 to 63 is the sequence encoding DsbA signal peptide. The amino acid sequence of the protein rCTB4565 is set forth in SEQ ID No.42, wherein starting from N-terminus, the sequence from positions 20 to 122 is the CTB amino acid sequence, the sequence from positions 123 to 127 is the flexible linker, the sequence from positions 128 to 148 is the amino acids from positions 45 to 65 of PilE, the sequence from positions 149 to 157 is the flexible linker and his-tag, and the sequence from positions 1 to 19 is the DsbA signal peptide sequence.

The sequence encoding rCTB4564 is set forth in SEQ ID No.43, wherein starting from 5' end, the sequence from positions 64 to 372 is the CTB coding sequence, the sequence from positions 388 to 447 is the sequence encoding the amino acids from positions 45 to 65 of PilE, and the sequence from positions 7 to 63 is the sequence encoding DsbA signal peptide. The amino acid sequence of the protein rCTB4564 is set forth in SEQ ID No.44, wherein starting from N-terminus, the sequence from positions 20 to 122 is the CTB amino acid sequence, the sequence from positions 123 to 127 is the flexible linker, the sequence from positions 128 to 147 is the amino acids from positions 45 to 64 of PilE, the sequence from positions 148 to 156 is the flexible linker and his-tag, and the sequence from positions 1 to 19 is the DsbA signal peptide sequence.

*Eco*RIand *Hin*dIII were used in double enzyme digestion of the DNA molecules set forth in SEQ ID No.31, SEQ ID No.33, SEQ ID No.35, SEQ ID No.37, SEQ ID No.39, SEQ ID No.41, and SEQ ID No.43, to obtain gene fragments, respectively; and *Eco*RI and *Hin*dIII were used in double enzyme digestion of pMMB66EH, to obtain a large fragment; each of the gene fragments was ligated to the large fragment, to obtain the recombinant plasmid, designated as pMMB66EH-rCTB4573, pMMB66EH-rCTB4571, pMMB66EH-rCTB4569, pMMB66EH-rCTB4567, pMMB66EH-rCTB4566, pMMB66EH-rCTB4565, and pMMB66EH-rCTB4564, respectively. The recombinant plasmids were sequenced, and the results were correct.

### 2. Preparation of glycoengineered Shigella flexneri

The expression vectors pMMB66EH-rCTB4573, pMMB66EH-rCTB4571, pMMB66EH-rCTB4569, pMMB66EH-rCTB4567, pMMB66EH-rCTB4566, pMMB66EH-rCTB4565, and pMMB66EH-rCTB4564 prepared in Step 1 were transformed into the *S. flexneri* 2a 301 ΔpCPΔ*waaI*/pETtac28_*pglL* strain prepared in the Step (II) by electroporation, and then the transformed cells were spread onto the LB solid medium containing kanamycin at a final concentration of 50µg/mL and ampicillin at a final concentration of 100µg/mL. The positive clones were the glycoengineered *Shigella flexneri*, i.e., *S. flexneri* 2a 301 ΔpCPΔ*waaI*/pETtac28-pglL/ pMMB66EH-rCTB4573, *S. flexneri* 2a 301 ΔpCPΔ*waaI*/pETtac28-pglL/ pMMB66EH-rCTB4571, *S. flexneri* 2a 301 ΔpCPΔ*waaI*/pETtac28-pglL/ pMMB66EH-rCTB4569, *S. flexneri* 2a 301 ΔpCPΔ*waaI*/pETtac28-pglL/ pMMB66EH-rCTB4567, *S. flexneri* 2a 301 ΔpCPΔ*waaI*/pETtac28-pglL/ pMMB66EH-rCTB4566, *S. flexneri* 2a 301 ΔpCPΔ*waaI*/pETtac28-pglL/ pMMB66EH-rCTB4565 and *S. flexneri* 2a 301 ΔpCPΔ*waaI*/pETtac28-pglL/ pMMB66EH-rCTB4564.

### 3. Glycosylation of recombinant CTB fusion protein and identification thereof

The monoclonal colonies of glycoengineered *Shigella flexneri, S.flexneri* 2a 301 ΔpCPΔ*waaI*/pETtac28-pglL/ pMMB66EH-rCTB4573, *S.flexneri* 2a 301 ΔpCPΔ*waaI*/pETtac28-pglL/ pMMB66EH-rCTB4571, *S.flexneri* 2a 301 ΔpCPΔ*waaI*/pETtac28-pglL/ pMMB66EH-rCTB4569, *S.flexneri* 2a 301 ΔpCPΔ*waaI*/pETtac28-pglL/ pMMB66EH-rCTB4567, *S.flexneri* 2a 301 ΔpCPΔ*waaI*/pETtac28-pglL/ pMMB66EH-rCTB4566, *S.flexneri* 2a 301 ΔpCPΔ*waaI*/pETtac28-pglL/ pMMB66EH-rCTB4565 and *S.flexneri* 2a 301 ΔpCPΔ*waaI*/pETtac28-pglL/ pMMB66EH-rCTB4564 were seeded in LB medium containing ampicillin at a final concentration of 100µg/mL and kanamycin a final concentration of 50µg/mL, and cultured at 37°C; when OD₆₀₀ was about 0.6, IPTG was added at a final concentration of 1mM, and the culture was cooled to 16°C and induced for 20h.

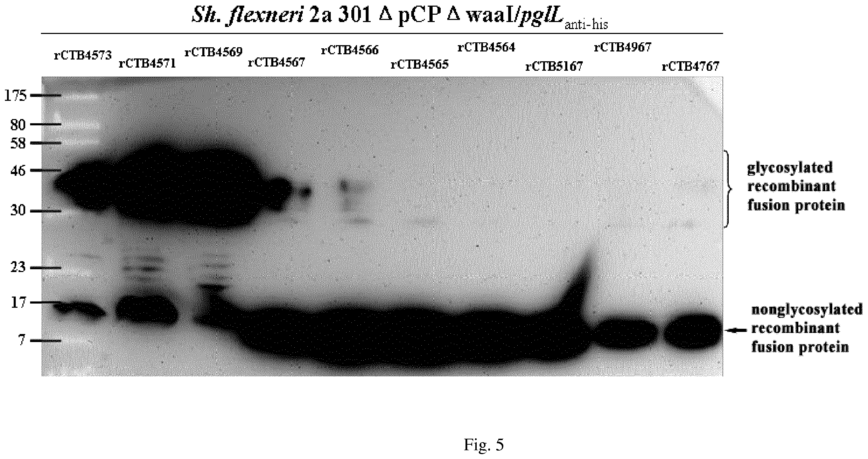

The next day, 1mL each of the bacterial liquids induced at 16°C for 20 h, was centrifuged to extract bacteria, and the extracted bacteria were slowly suspended in 1X reducing buffer (50mM pH 6.8 Tris-HCl, 1.6% SDS, 0.02% bromophenol blue, 8% glycerol, 20mM DTT), in a boiling water bath for 10min, to obtain the sample for electrophoresis. The sample was then subj ected to 15% SDS-PAGE electrophoresis. After electrophoresis, the protein was transferred onto PVDF membrane by Bio-Lab Semi-Dry Blotter, at a constant voltage of 20V for 1h, and detected by Anti-His tag mouse monoclonal antibody, and the results are shown in Fig. 5.

Fig. 5 shows that the truncated PilE peptide fragment, with the C-terminus ended at V66, still enabled the recombinant CTB fusion protein to be O-glycosylated, but the glycosylation efficiency was significantly decreased compared to other recombinant CTB fusion proteins modified by a relatively longer peptide fragment.

(IV) Identification of the N-terminus sequence of the core peptide fragment that enables the recombinant fusion protein to be O-glycosylated

### 1. Construction of an expression vector of the recombinant EPA fusion protein

According to the tertiary structure of *Neisseria meningitidis* pilin PilE (as shown in Fig. 4), polypeptides of different lengths containing serine (S63) at position 63 of PilE were truncated, rEPA was used as carrier protein, and the polypeptides of different lengths were fused to the N-terminus or C-terminus of rEPA. The particular method was as followed:
With the N-terminus started from the glycine (G55) at position 55 of PilE, and the C-terminus ended at lysine (K73) at position 73 of PilE, 19 amino acids from G55~K73 were truncated, and fused to the N-terminus of rEPA; or with the N-terminus started from the glycine (G55) at position 55 of PilE, and the C-terminus ended at serine (S69) at position 69 of PilE, 15 amino acids from G55~S69 were truncated, and fused to the N-terminus of rEPA; or with the N-terminus started from the glycine (G55) at position 55 of PilE, and the C-terminus ended at valine (V66) at position 66 of PilE, 12 amino acids from G55~ V66 were truncated, and fused to the N-terminus of rEPA; or with the N-terminus started from the proline (P58) at position 58 of PilE, and the C-terminus ended at valine (V66) at position 66 of PilE, 9 amino acids from P58~ V66 were truncated, and fused to the N-terminus of rEPA; or with the N-terminus started from the serine (S45) at position 45 of PilE, and the C-terminus ended at lysine (K73) at position 73 of PilE, 29 amino acids from S45~K73 were truncated, and fused to the C-terminus of rEPA; i.e., the peptide fragments of G55~ K73, G55~ S69, G55~ V66, P58~ V66 were fused to the N-terminus of rEPA, respectively, or the peptide fragment of S45~ K73 was fused to the C-terminus of rEPA. Since glycosylation occurs in periplasmic space, according to the amino acid sequence of Pseudomonas aeruginosa exotoxin A (AE004091.2) published on GenBank, its signal peptide (the first 25 amino acids) was replaced with DsbA signal peptide, E at position 553 was deleted, L at position 552 was mutated to V, and the C-terminus of the recombinant fusion protein was fused to a 6×His tag, for the convenience of further detection, thereby constructing a series of recombinant EPA recombinant fusion proteins rEPA5573_{N}, rEPA5569_{N}, rEPA5566_{N}, rEPA5866_{N}, and rEPA4573.

The sequence encoding rEPA4573 is set forth in SEQ ID No.45, wherein starting from 5' end, the sequence from positions 64 to 1899 is the rEPA coding sequence, the sequence from positions 1915 to 2001 is the sequence encoding the amino acids from positions 45 to 73 of PilE, and the sequence from positions 7 to 63 is the sequence encoding DsbA signal peptide. The amino acid sequence of the protein rEPA4573 is set forth in SEQ ID No.46, wherein starting from N-terminus, the sequence from positions 20 to 631 is the rEPA amino acid sequence, the sequence from positions 632 to 636 is the flexible linker, the sequence from positions 637 to 665 is the amino acids from positions 45 to 64 of PilE, the sequence from positions 666 to 674 is the flexible linker and his-tag, and the sequence from positions 1 to 19 is the DsbA signal peptide sequence.

The sequence encoding rEPA5573_{N} is set forth in SEQ ID No.47, wherein starting from 5' end, the sequence from positions 70 to 126 is the sequence encoding the amino acids from positions 55 to 73 of PilE, the sequence from positions 133 to 1962 is the rEPA coding sequence, and the sequence from positions 7 to 63 is the sequence encoding DsbA signal peptide. The amino acid sequence of the protein rEPA5573_{N} is set forth in SEQ ID No.48, wherein starting from N-terminus, the sequence from positions 22 to 40 is the amino acids from positions 55 to 73 of PilE, the sequence from positions 41 to 42 is the flexible linker, the sequence from positions 43 to 652 is the rEPA amino acid sequence, the sequence from positions 653 to 666 is the flexible linker and his-tag, and the sequence from positions 1 to 19 is the DsbA signal peptide sequence.

The sequence encoding rEPA5569_{N} is set forth in SEQ ID No.49, wherein starting from 5' end, the sequence from positions 70 to 114 is the sequence encoding the amino acids from positions 55 to 69 of PilE, the sequence from positions 121 to 1950 is the rEPA coding sequence, the sequence from positions 7 to 63 is the sequence encoding DsbA signal peptide. The amino acid sequence of the protein rEPA5569_{N} is set forth in SEQ ID No.50, wherein starting from N-terminus, the sequence from positions 22 to 36 is the amino acids from positions 55 to 69 of PilE, the sequence from positions 37 to 38 is the flexible linker, the sequence from positions 39 to 648 is the rEPA amino acid sequence, the sequence from positions 649 to 662 is the flexible linker and his-tag, and the sequence from positions 1 to 19 is the DsbA signal peptide sequence.

The sequence encoding rEPA5566_{N} is set forth in SEQ ID No.51, wherein starting from 5' end, the sequence from positions 70 to 105 is the sequence encoding the amino acids from positions 55 to 66 of PilE, the sequence from positions 112 to 1941 is the rEPA coding sequence, and the sequence from positions 7 to 63 is the sequence encoding DsbA signal peptide. The amino acid sequence of the protein rEPA5566_{N} is set forth in SEQ ID No.52, wherein starting from N-terminus, the sequence from positions 22 to 33 is the amino acids from positions 55 to 66 of PilE, the sequence from positions 34 to 35 is the flexible linker, the sequence from positions 36 to 645 is the rEPA amino acid sequence, the sequence from positions 646 to 659 is the flexible linker and his-tag, and the sequence from positions 1 to 19 is the DsbA signal peptide sequence.

The sequence encoding rEPA5866_{N} is set forth in SEQ ID No.53, wherein starting from 5' end, the sequence from positions 70 to 96 is the sequence encoding the amino acids from positions 58 to 66 of PilE, the sequence from positions 103 to 1932 is the rEPA coding sequence, and the sequence from positions 7 to 63 is the sequence encoding DsbA signal peptide. The amino acid sequence of the protein rEPA5866_{N} is set forth in SEQ ID No.54, wherein starting from N-terminus, the sequence from positions 22 to 30 is the amino acids from positions 58 to 66 of PilE, the sequence from positions 31 to 32 is the flexible linker, the sequence from positions 33 to 642 is the rEPA amino acid sequence, the sequence from positions 643 to 656 is the flexible linker and his-tag, and the sequence from positions 1 to 19 is the DsbA signal peptide sequence.

*Eco*RI and *Hin*dIII were used in double enzyme digestion of the DNA molecules set forth in SEQ ID No.45, SEQ ID No.47, SEQ ID No.49, SEQ ID No.51, and SEQ ID No.53, to obtain gene fragments, respectively; *Eco*RI and *Hin*dIII were used in double enzyme digestion of pMMB66EH, to obtain a large fragment; each of the gene fragments was ligated to the large fragment to obtain the recombinant plasmid, designated as pMMB66EH-rEPA4573, pMMB66EH-rEPA5573_{N}, pMMB66EH-rEPA5569_{N}, pMMB66EH-rEPA5566_{N}, and pMMB66EH-rEPA5866_{N}, respectively. The recombinant plasmids were sequenced, and the results were correct.

### 2. Preparation of glycoengineered Shigella flexneri

The expression vectors pMMB66EH-rEPA4573, pMMB66EH-rEPA5573_{N}, pMMB66EH-rEPA5569_{N}, pMMB66EH-rEPA5566_{N}, and pMMB66EH-rEPA5866_{N} prepared in Step 1 were electrotransformed into *S. flexneri* 2a 301 ΔpCPΔ*waaI*/pETtac28_*pglL* strain prepared in the Step (II), respectively, and the transformed cells were spread onto LB solid medium containing kanamycin at a final concentration of 50µg/mL and ampicillin at a final concentration of 100µg/mL. The positive clones were the glycoengineered *Shigella flexneri*, i.e., *S. flexneri* 2a 301 ΔpCPΔ*waaI*/pETtac28-*pglL*/ pMMB66EH-rEPA4573, *S. flexneri* 2a 301 ΔpCPΔ*waaI*/pETtac28-*pglL*/ pMMB66EH-rEPA5573_{N}, *S. flexneri* 2a 301 ΔpCPΔ*waaI*/pETtac28-*pglL*/ pMMB66EH-rEPA5569_{N}, *S. flexneri* 2a 301 ΔpCPΔ*waaI*/pETtac28-*pglL*/ pMMB66EH-rEPA5566_{N}, and *S. flexneri* 2a 301 ΔpCPΔ*waaI*/pETtac28-*pglL*/ pMMB66EH-rEPA5866_{N}.

### 3. Glycosylation of EPA fusion protein and identification thereof

The monoclonal colonies of glycoengineered bacteria *S. flexneri* 2a 301 ΔpCPΔ*waaI*/pETtac28-*pglL*/ pMMB66EH-rEPA4573, *S. flexneri* 2a 301 ΔpCPΔ*waaI*/pETtac28-*pglL*/ pMMB66EH-rEPA5573_{N}, *S. flexneri* 2a 301 ΔpCPΔ*waaI*/pETtac28-*pglL*/ pMMB66EH-rEPA5569_{N}, *S. flexneri* 2a 301 ΔpCPΔ*waaI*/pETtac28-*pglL*/ pMMB66EH-rEPA5566_{N}, and *S. flexneri* 2a 301 ΔpCPΔ*waaI*/pETtac28-*pglL*/ pMMB66EH-rEPA5866_{N}, were seeded in LB medium containing ampicillin at a final concentration of 100µg/mL and kanamycin at a final concentration of 50µg/mL, and cultured at 37°C. When the OD₆₀₀ was about 0.6, IPTG was added at a final concentration of 1mM, and the culture were cooled to 16°C and induced for 20h.

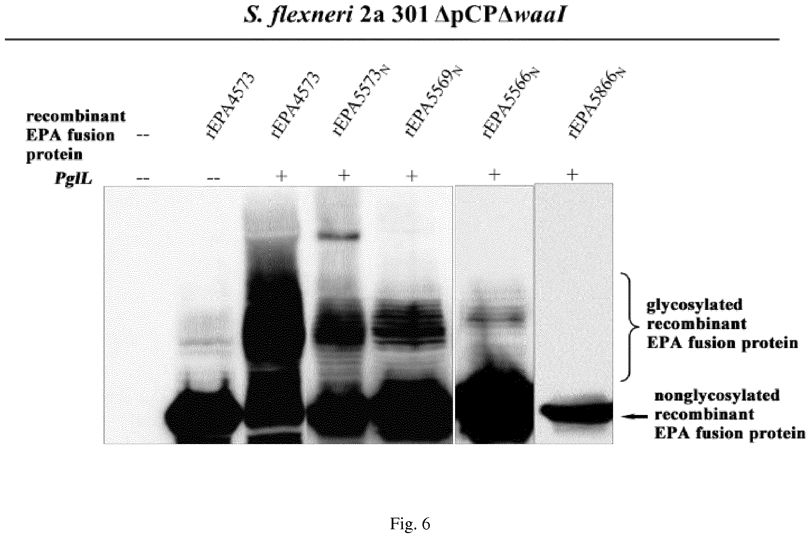

The next day, 1mL each of the bacterial liquids induced at 16°C for 20h, was centrifuged to extract the bacteria, and the extracted bacteria were slowly suspended in 1× reducing buffer, in a boiling water bath for 10min, to obtain the sample for electrophoresis. The sample was subjected to 8% SDS-PAGE electrophoresis. After electrophoresis, the protein was transferred onto PVDF membrane by Bio-Lab Semi-Dry Blotter, at a constant voltage of 20V for 1h, and detected by anti-EPA antibody, and the results are shown in Fig. 6.

Fig. 6 shows that the truncated PilE proteins, i.e., the peptide fragments of G55~ K73, G55~ S69, and G55~ V66, fused to the N-terminus of EPA protein, still successfully enabled the recombinant EPA fusion protein to be O-glycosylated, but the glycosylation efficiency of the recombinant EPA fusion protein gradually decreased with the shortening of the truncated peptide fragment.

The above results show that the core peptide fragment, which enables the recombinant fusion protein to be O-glycosylated, is G55~ V66 of PilE. However, in view of the practical application, in the invention, relevant studies are carried out by fusing the amino acid sequence of P1a (i.e., S45~ K73 of PilE) (PilE with a Genbank accession number of NC_003112.2) to a different carrier protein at a suitable position.

### III. Preparation of Shigella flexneri O-polysaccharide-recombinant CTB fusion protein conjugate by one-step bioconjugate method

(I) The monoclonal colony of the glycoengineered bacterium *S. flexneri* 2a 301 ΔpCPΔ*waaI*/pETtac28-*pglL*/ pMMB66EH-rCTB4573, was seeded to LB medium containing 100µg/mL ampicillin and 50µg/mL kanamycin, and cultured at 37°C. When OD₆₀₀ was about 0.6, IPTG was added at a final concentration of 1mM, and the culture was cooled to 16°C and induced for 20h.
(II) Purification of O-polysaccharide-recombinant CTB fusion protein conjugate rCTB4573-OPS_{*Sf*301}

### 1. Sample pretreatment

To 10g bacteria induced at 16°C for 20h in the Step (I), 100mL purified water was added. The bacteria were broken ultrasonically (ultrasonication for 3s and pause for 5s, with an accumulative period of ultrasonication for 30min), and centrifuged at 12000g. The supernatant was collected, and a sample loading buffer (20mM pH7.5 Tris-HCl, 0.2M NaCl, 10mM imidazole) was added to the supernatant. After well stirring, centrifugation was carried out at 12000g again. The supernatant was collected, and the supernatant was the crude extract containing O-polysaccharide-recombinant CTB fusion protein conjugate rCTB4573-OPS_{*Sf*301}.

### 2. Purification of a sample with Chelating affinity chromatographic column

The sample was preliminarily purified with Chelating affinity chromatographic column (Φ1.6cm^{∗}15cm).

Firstly, the column was washed with at least three column volumes of 0.5M NaOH aqueous solution, and then equilibrated with deionized water to a neutral pH. The column was then equilibrated with at least 3 column volumes of 0.5M NiSO₄ aqueous solution, further equilibrated with at least one column volume of B1 solution (20mM pH7.5 Tris-HCl, 0.5M NaCl, 500mM imidazole), and finally equilibrated with at least 3 column volumes of A1 solution (20mM pH7.5 Tris-HCl, 0.5M NaCl, 10mM imidazole), all at a flow rate of 4mL/min. The crude extract of rCTB4573- OPS_{*Sf*301} obtained in Step 1 was loaded from A pipeline, and A1 solution (20mM pH7.5 Tris-HCl, 0.5M NaCl, 10mM imidazole) was used to wash the unbound protein, until the ultraviolet absorption (280nM) was close to 0 mAU. Finally, 100% B1 (20mM pH7.5 Tris-HCl, 0.5M NaCl, 500mM imidazole) was used for elution, and 30mL eluate was collected to obtain the preliminarily purified sample.

### 3. Sample desalting

The preliminarily purified sample obtained in Step 2 was desalted with G25 fine chromatographic column (Φ1.6cm^{∗}30cm), wherein the mobile phase was A2 solution (20mM pH5.4 HAc-NaAc).

Firstly, the column was washed with at least 3 column volumes of 0.5M NaOH aqueous solution, then equilibrated with deionized water to a neutral pH, and finally equilibrated with at least 3 column volumes of A2 solution. The preliminarily purified sample obtained in Step 2 was loaded from A pipeline, wherein the mobile phase was A2 solution (20mM pH5.4 HAc-NaAc). 60mL sample was collected to obtain the desalted sample. And all the flow rates involved in each step of the above described process were 4mL/min.

### 4. Further purification of rCTB4573-OPS_{Sf301} with ProteinPak SP8HR cation exchange chromatographic column

The desalted sample obtained in Step 3 was further purified with ProteinPak SP8HR cation exchange chromatographic column.

Firstly, the column was washed with at least 3 column volumes of 0.5M NaOH aqueous solution, and then equilibrated with deionized water to a neutral pH. The column was then equilibrated with at least 3 column volumes of A2 solution (20mM pH5.4 HAc-NaAc). The sample was loaded from A pipeline, the A2 solution was used to wash the unbound glycoprotein, and linear elution from 0 to 50 % B2 solution was carried out (the A2 solution entered from A pipeline and the B2 solution entered from B pipeline, were automatically mixed by the purifier) within 30min. The eluate was collected. And all the flow rates involved in each step of the above described process were 1mL/min. The peak of the glycoprotein rCTB4573-OPS_{*Sf*301} appeared with an electric conductivity of about 35~45mS/cm, i.e., the target protein rCTB4573-OPS_{*Sf*301}.

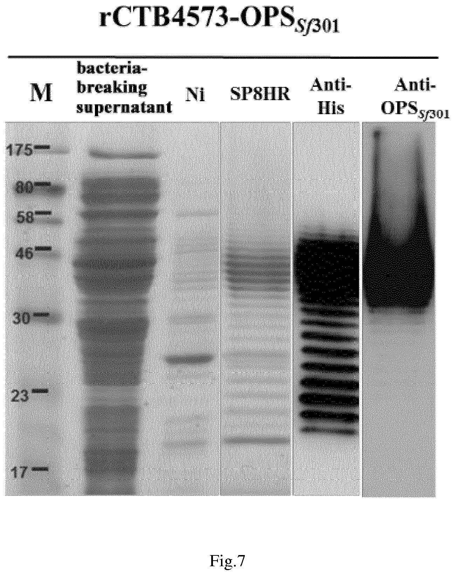

The sample was analyzed by 12% SDS-PAGE and western blot, and the results are shown in Fig. 7. In Fig. 7, M represents protein marker; bacteria-breaking supernatant represents the crude extract of rCTB4573-OPS_{*Sf*301} obtained in Step 1; Ni represents the preliminariliy purified sample obtained in Step 2; SP8HR represents the target protein rCTB4573-OPS_{*Sf*301} further purified by ProteinPak SP8HR cation exchange chromatographic column; anti-His represents the western blot result by Anti-His tag mouse monoclonal antibody; and anti-OPS_{*Sf*301} represents the western blot result by rabbit Anti- OPS_{*Sf*301} antiserum.

### IV. Preparation of Shigella flexneri O-polysaccharide-recombinant EPA fusion protein conjugate by one-step bioconjugate method

(I) The monoclonal colony of glycoengineered bacterium *S. flexneri* 2a 301 ΔpCPΔ*waaI*/pETtac28-*pglL*/ pMMB66EH-rEPA4573, was seeded to a LB medium containing 100µg/mL ampicillin and 50µg/mL kanamycin, and cultured at 37°C. When OD₆₀₀ was about 0.6, IPTG was added at a final concentration of 1mM, and the culture was cooled to 16°C and induced for 20h.
(II) Purification of O-polysaccharide-recombinant EPA fusion protein conjugate rEPA4573-OPS_{*Sf*301}

### 1. Sample pretreatment

To 10g bacteria induced at 16°C for 20h in the Step (I), 100mL purified water was added. The bacteria were broken ultrasonically (ultrasonation for 3s and pause for 5s, with an accumulative period of ultrasonation for 30min), and centrifuged at 12000g. The supernatant was collected, a sample loading buffer (20mM pH7.5 Tris-HCl, 0.2M NaCl, 10mM imidazole) was added to the supernatant. After well stirring, the centrifugation was carried out at 12000g again. The supernatant was collected, and the supernatant was the crude extract containing the O-antigen polysaccharide-recombinant EPA fusion protein conjugate rEPA4573-OPS_{*Sf*301}.

### 2. Purification of a sample with Chelating affinity chromatographic column

The sample was preliminarily purified with Chelating affinity chromatographic column (Φ1.6cm^{∗}15cm).

Firstly, the column was washed with at least three column volumes of 0.5M NaOH aqueous solution, and then equilibrated with deionized water to a neutral pH. The column was then equilibrated with at least 3 column volumes of 0.5M NiSO₄ aqueous solution, further equilibrated with at least one column volume of B1 solution (20mM pH7.5 Tris-HCl, 0.5M NaCl, 500mM imidazole), and finally equilibrated with at least 3 column volumes of A1 solution (20mM pH7.5 Tris-HCl, 0.5M NaCl, 10mM imidazole), all at a flow rate of 4mL/min. The crude extract of rEPA4573-OPS_{*Sf*301} obtained in Step 1 was loaded from A pipeline, and A1 solution (20mM pH7.5 Tris-HCl, 0.5M NaCl, 10mM imidazole) was used to wash the unbound protein. Finally, 100% B1 (20mM pH7.5 Tris-HCl, 0.5M NaCl, 500mM imidazole) was used for elution, and 30mL eluate was collected to obtain the preliminarily purified sample.

### 3. Sample desalting

The sample preliminarily purified with Chelating affinity chromatographic column was desalted with G25 fine chromatographic column (Φ1.6cm^{∗}30cm), wherein the mobile phase was A3 solution (20mM pH7.5 Tris-HCl).

Firstly, the column was washed with at least 3 column volumes of 0.5M NaOH aqueous solution, then equilibrated with deionized water to a neutral pH, and finally equilibrated with at least 3 column volumes of A3 solution. The preliminarily purified sample obtained in Step 2 was loaded from A pipeline, and 60mL sample was collected to obtain the desalted sample, wherein the mobile phase was A3 solution (20mM pH7.5 Tris-HCl). And all the flow rates involved in each step of the above described process were 4mL/min.

### 4. Further purification of rEPA4573-OPS_{Sf301} with ProteinPak DEAE8HR anion exchange chromatographic column

The desalted sample obtained in Step 3 was further purified with ProteinPak DEAE8HR anion exchange chromatographic column (waters).

Firstly, the column was washed with at least 3 column volumes of 0.5M NaOH aqueous solution, and then equilibrated with deionized water to a neutral pH. The column was then equilibrated with at least 3 column volumes of A3 solution (20mM pH7.5 Tris-HCl). The sample was loaded from A pipeline, the A3 solution was used to wash the unbound glycoprotein, and linear elution from 0 to 50% B3 solution was carried out (the A3 solution entered from A pipeline, and the B3 solution entered from B pipeline, were automatically mixed by the purifier) within 30min. The eluate was collected. And all the flow rates involved in each step of the above described process were 1mL/min. The peak of the glycoprotein rEPA4573-OPS_{*Sf*301} appeared with an electric conductivity of about 8~18mS/cm, i.e., the crude extract of the target protein rEPA4573-OPS_{*Sf*301}.

### 5. Fine purification of rEPA4573-OPS_{Sf301} with Superdex 75 chromatographic column

The sample purified with ProteinPak DEAE8HR anion exchange chromatographic column was further purified with Superdex 75 FPLC(Φ1cm^{∗}30cm, GE Company).

Firstly, the column was washed with at least 3 column volumes of 0.5M NaOH aqueous solution, and then equilibrated with deionized water to a neutral pH. The columen was then equilibrated with at least 3 column volumes of A4 solution (20mM pH7.5 PB, 0.9g/100ml NaCl), and 1mL of the crude extract of the protein rEPA4573-OPS_{*Sf*301} was loaded via a loading loop. 8~11mL effluent sample was collected. The sample was the finely purified rEPA4573-OPS_{*Sf*301}, i.e., the target protein rEPA4573-OPS_{*Sf*301}.

The sample was analyzed by 8% SDS-PAGE and western blot, and the *S.flexneri* 2a 301 ΔpCPΔ*waaI*/ pMMB66EH-rEPA4573, which was transformed only with pMMB66EH-rEPA4573, but not with pETtac28-*pglL*, was used as control.

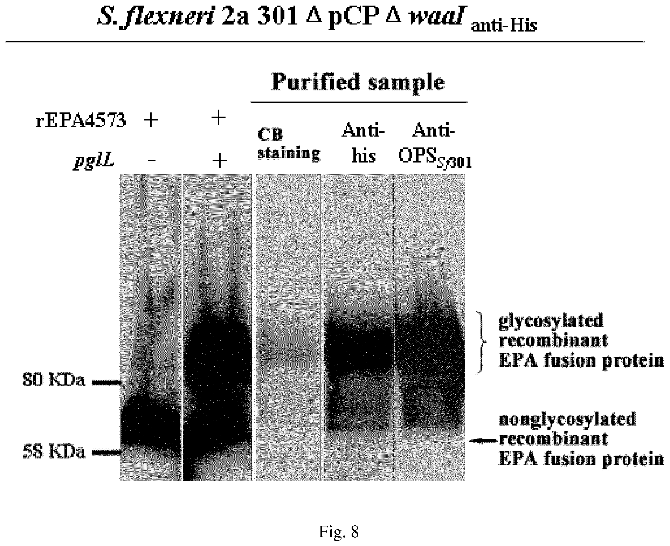

The results are shown in Fig. 8. In Fig. 8, from left to right, Lane 1 represents the control protein; Lane 2 represents the crude extract of rEPA4573-OPS_{*Sf*301} obtained in Step 1; the purified sample represents the target protein rEPA4573-OPS_{*Sf*301} finely purified in Step 5; CB straining represents the Commassie Blue staining result of the purified sample; Anti-his represents the western blot result by Anti-His tag mouse monoclonal antibody; Anti-OPS_{*Sf*301} represents the western blot result by rabbit Anti- OPS_{*Sf*301} anti-serum.

### V. Preparation and animal experiment evaluation of rCTB4573-OPS_{Sf301} and rEPA4573-OPS_{Sf301} polysaccharide-protein conjugate vaccines

(I) Preparation of rCTB4573-OPS_{*Sf*301} and rEPA4573-OPS_{*Sf*301} polysaccharide-protein conjugate vaccines
   The rCTB4573-OPS_{*Sf*301} purified in Step III and the rEPA4573-OPS_{*Sf*301} purified in Step IV were sterilized by filtration, and separately mixed with aluminium hydroxide adjuvant in a volume ratio of 9:1, to obtain the samples of rCTB4573-OPS_{*Sf*301} and rEPA4573-OPS_{*Sf*301} for intraperitoneal injection.
(II) Animal immunization with rCTB4573-OPS_{*Sf*301} and rEPA4573-OPS_{*Sf*301} and effect evaluation thereof

### 1. Preparation of O-antigen (OPS_{Sf301})

LPS (see, the paper "Sun Yang, Feng Shuzhang, Zhu Lingwei, et al., Preparation and identification of monoclonal antibodies against LPS of entero-hemorrhagic E. coli 0157 [J]. Chinese Journal of Zoonoses, 2007, 23(10): 971-973.") was extracted by means of hot phenol-water extraction, and lyophilized. The lyophilized sample was dissolved in 1% glacial acetic acid at a concentration of 10mg/ml, in a boiling water bath for 90min, and then cooled to room temperature. pH was adjusted to 7.0. After centrifugation at 64000×g for 5h, the supernatant was collected, and was lyophilized after extensive dialysis with deionized water.

### 2. Mouse experiment

40 6-week-old female Balb/c mice, were randomly divided into the following four groups: the aluminium hydroxide group, the OPS_{*Sf*301} group, the rCTB4573-OPS_{*Sf*301} group and the rEPA4573-OPS_{*Sf*301} group, wherein the aluminium hydroxide group was used as negative control, and each of the other three groups was injected with 2.5µg of polysaccharide as calculated based on the polysaccharide content of the conjugate vaccine as used. Each of the groups was immunized at Day1, 14, 28, and eyeball was removed to collect blood 10 days after the last immunization.

Indirect ELISA was used to determine the titer of antibody against the *Shigella flexneri* 2a type O-antigen polysaccharide in the serum of each group of mice. The assay plates were coated with the extracted LPS of the *Shigella flexneri* 2a 301 strain, and each well was coated with 10µg LPS. As to the other operating steps, please refer to Short Protocols in Molecular Biology [M]. Science Press, 2008.

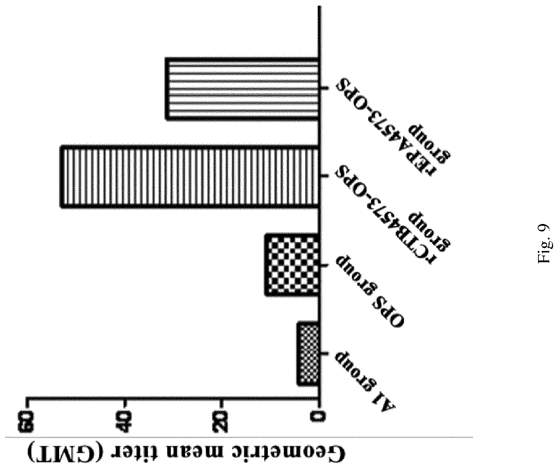

The results are shown in Fig. 9. In Fig. 9, A1 group represents the aluminium hydroxide group; OPS group represents the OPS_{*Sf*301} group; rCTB-OPS group represents the rCTB4573- OPS_{*Sf*301} group; rEPA4573-OPS group represents the rEPA4573-OPS_{*Sf*301} group. Fig. 9 shows that as compared with OPS_{*Sf*301}, the rCTB4573-OPS_{*Sf*301} purified in Step III and the rEPA4573-OPS_{*Sf*301} purified in Step IV could significantly induce the generation of specific antibodies against OPS (O-polysaccharide) of *Shigella flexneri* 2a 301 in mice.

### VI. Increasing the ratio of polysaccharides/protein by connection of O-glycosylation sites in tandem

### (I) Construction of an expression vector of the recombinant CTB fusion protein rCTB4573₂ containing two P1a sequences

In order to increase the percentage of polysaccharides in a glycoprotein, the amino acid sequences of two P1a (i.e., S45~ K73 of PilE) were fused in tandem to the C-terminus of CTB, to synthesize rCTB4573₂.

The sequence encoding rCTB4573₂ is set forth in SEQ ID No.55, wherein starting from 5' end, the sequence from positions 64 to 372 is the CTB coding sequence, the sequence from positions 388 to 474, and the sequence from positions 487 to 573 each are the sequence encoding the amino acids from positions 45 to 67 of PilE, and the sequence from positions 7 to 63 is the sequence encoding DsbA signal peptide. The amino acid sequence of the protein rCTB4573₂ is set forth in SEQ ID No.56, wherein starting from N-terminus, the sequence from positions 20 to 122 is the CTB amino acid sequence, the sequence from positions 123 to 127, and the sequence from positions 157 to 160 is the flexible linker, the sequence from positions 128 to 156 and the sequence from positions 161 to 189 each are the amino acids from positions 45 to 73 of PilE, the sequence from positions 190 to 199 is the flexible linker and his-tag, and the sequence from positions 1 to 19 is the DsbA signal peptide sequence.

*Eco*RI and *Hind*III were used in double enzyme digestion of the DNA molecule set forth in SEQ ID No.55, to obtain a gene fragment; *Eco*RI and *Hind*III were used in double enzyme digestion of pMMB66EH, to obtain a large fragment; the gene fragment was ligated to the large fragment, to obtain the recombinant plasmid, designated as pMMB66EH-rCTB4573₂. The recombinant plasmid pMMB66EH-rCTB4573₂ was sequenced, and the results were correct.

### (II) Construction of an expression vector of the recombinant EPA fusion protein (rEPA4573_{NMC}) containing three P1a sequences

In order to increase the percentage of polysaccharides in a glycoprotein, according to the steric structure of EPA, three P1a sequences (i.e., S45~ K73 of PilE) was introduced on the molecular surface. The particular method was as followed: two P1a polypeptides were fused at the N-terminus and C-terminus of rEPA, respectively; and one P1a polypeptide was fused between K240 and P241 of rEPA, so as to construct rEPA4573_{NMC}.

The sequence encoding rEPA4573_{NMC} is set forth in SEQ ID No.57, wherein starting from 5' end, the sequence from positions 70 to 156, the sequence from positions 877 to 963, and the sequence from positions 2101 to 2187 each are the sequence encoding the amino acids from positions 45 to 67 of PilE, the sequence from positions 2188 to 2217 is the sequence encoding the flexible linker and his-tag, and the sequence from positions 7 to 63 is the sequence encoding DsbA signal peptide. The amino acid sequence of the protein rEPA4573_{NMC} is set forth SEQ ID No.58. The sequence from positions 1 to 19 is the DsbA signal peptide; the sequence from positions 22 to 50, the sequence from positions 291 to 319, and the sequence from positions 699 to 727 each are the amino acids from positions 45 to 73 of PilE; the sequence from positions 694 to 698 is the flexible linker, and the sequence from positions 728 to 736 is the flexible linker and his-tag sequence.

*Eco*RI and *Hind*III were used in double enzyme digestion of the DNA molecule set forth in SEQ ID No.57, to obtain a gene fragment; *Eco*RI and *Hind*III were used in double enzyme digestion of pMMB66EH, to obtain a large fragment; the gene fragment was ligated to the large fragment, to obtain the recombinant plasmid, designated as pMMB66EH-rEPA4573_{NMC}. The recombinant plasmid pMMB66EH-rEPA4573_{NMC} was sequenced, and the results were correct.

### (III) Preparation of glycoengineered Shigella flexneri

In accordance with the method for preparing glycoengineered Shigella flexneri as disclosed in Step II (III), the glycoengineered bacteria *S. flexneri* 2a 301 ΔpCPΔ*waaI* /pETtac28-*pglL* / pMMB66EH- rCTB4573₂ and *S. flexneri* 2a 301 ΔpCPΔ*waaI* /pETtac28-*pglL* / pMMB66EH- rEPA4573_{NMC} were constructed, respectively.

### (IV) Glycosylation of rCTB4573₂ and rEPA4573_{NMC} in the glycoengineered Shigella flexneri and identification thereof

The monoclonal colonies of glycoengineered bacteria *S. flexneri* 2a 301 ΔpCPΔ*waaI* /pETtac28-*pglL* / pMMB66EH- rCTB4573₂ and *S. flexneri* 2a 301 ΔpCPΔ*waaI* /pETtac28-*pglL* / pMMB66EH- rEPA4573_{NMC}, were seeded in LB medium containing ampicillin at a final concentration of 100µg/mL and kanamycin at a final concentration of 50µg/mL, respectively, and cultured at 37°C. When OD₆₀₀ was about 0.6, IPTG was added at a final concentration of 1mM. The culture was cooled to 16°C and induced for 20h.

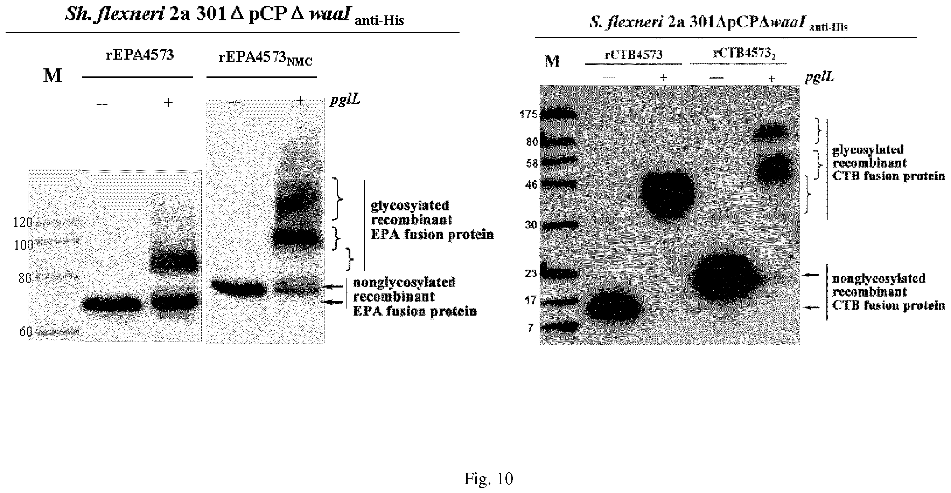

The next day, 1mL bacterial liquid induced at 16°C for 20h, was centrifuged to extract bacteria, and the extracted bacteria were slowly suspended in 1X reducing buffer, in a boiling water bath for 10min, to obtain the sample for electrophoresis. The sample was then subjected to SDS-PAGE electrophoresis. After electrophoresis, the protein was transferred onto PVDF membrane by Bio-Lab Semi-Dry Blotter, at a constant voltage of 20V for 1h, and detected by Anti-His tag mouse monoclonal antibody. The results are shown in Fig. 10.

Meanwhile, *S. flexneri* 2a 301 ΔpCPΔ*waaI* / pMMB66EH- rCTB4573₂, *S. flexneri* 2a 301 ΔpCPΔ*waaI* / pMMB66EH- rEPA4573_{NMC}, *S. flexneri* 2a 301 ΔpCPΔ*waaI* /pETtac28-*pglL*/ pMMB66EH- rCTB4573, *S. flexneri* 2a 301 ΔpCPΔ*waaI* /pETtac28-*pglL*/ pMMB66EH- rEPA4573, *S. flexneri* 2a 301 ΔpCPΔ*waaI* / pMMB66EH- rCTB4573, and *S. flexneri* 2a 301 ΔpCPΔ*waaI* / pMMB66EH- rEPA4573 were used as control.

Fig. 10 shows that the recombinant fusion proteins rCTB4573, rEPA4573 expressed in the glycoengineered bacteria *S. flexneri* 2a 301 ΔpCPΔ*waaI* /pETtac28-*pglL* / pMMB66EH- rCTB4573₂ and *S. flexneri* 2a 301 ΔpCPΔ*waaI* /pETtac28-*pglL* / pMMB66EH- rEPA4573_{NMC}, were O-glycosylated, and after the introduction of multiple glycosylation sites, the appearance of several groups of glycosylated bands indicated that the proteins were effectively glycosylated at multiple sites.

### VII. Preparation of Shigella flexneri O-polysaccharide-recombinant TTC fusion protein conjugate by one-step bioconjugate method

### (I) Construction of an expression vector of the TTC fusion protein

According to the amino acid sequence of fragment C of tetanus toxin (AF154828.1) published on GenBank, DsbA signal peptide is fused to its N- terminus, the polypeptide of P1a (i.e., S45~ K73 of PilE) and a 6×His tag were fused to its C-terminus, so as to construct the recombinant fragment C of tetanus toxin, rTTC4573.

The sequence encoding rTTC4573 is set forth in SEQ ID No.59, wherein starting from 5' end, the sequence from positions 64 to 1371 is the TTC coding sequence, the sequence from positions 1387 to 1473 is the sequence encoding the amino acids from positions 45 to 67 of PilE, and the sequence from positions 7 to 63 is the sequence encoding DsbA signal peptide.

The amino acid sequence of the protein rTTC4573 is set forth in SEQ ID No.60, wherein starting from N-terminus, the sequence from positions 20 to 455 is the TTC amino acid sequence, the sequence from positions 461 to 489 is the amino acids from positions 45 to 73 of PilE, the sequence from positions 490 to 498 is the flexible linker and his-tag, and the sequence from positions 1 to 19 is the DsbA signal peptide sequence.

EcoRI and HindIII were used in double enzyme digestion of the DNA molecule set forth in SEQ ID No.59, to obtain a gene fragment; *Eco*RI and *Hind*III were used in double enzyme digestion of pMMB66EH, to obtain a large fragment; and the gene fragment was ligated to the large fragment, to obtain the recombinant plasmid, designated as pMMB66EH-rTTC4573. The pMMB66EH-rTTC4573 was sequenced, and the result was correct.

### (II) Preparation of glycoengineered Shigella flexneri

*S.flexneri* 2a 301 ΔpCPΔ*waaI*/pETtac28-*pglL* was transformed with the pMMB66EH- rTTC4573, to construct the glycoengineered *Shigella flexneri, S.flexneri* 2a 301 ΔpCPΔ*waaI*/pETtac28-pglL/pMMB66EH- rTTC4573.

### (III) Glycosylation of rTTC4573 in the glycoengineered Shigella flexneri and identification thereof

The monoclonal colony of glycoengineered bacterium *S. flexneri* 2a 301 ΔpCPΔ*waaI*/pETtac28-pglL/ pMMB66EH- rTTC4573 was seeded to LB medium containing 100µg/mL ampicillin and 50µg/mL kanamycin, and cultured at 37°C. When OD₆₀₀ was about 0.6, IPTG was added at a final concentration of 1mM. The culuture was cooled to 25°C and induced for 20h.

Meanwhile, *S.flexneri* 2a 301 ΔpCPΔ*waaI*/pMMB66EH- rTTC4573 was subjected to said induction experiment, as control.

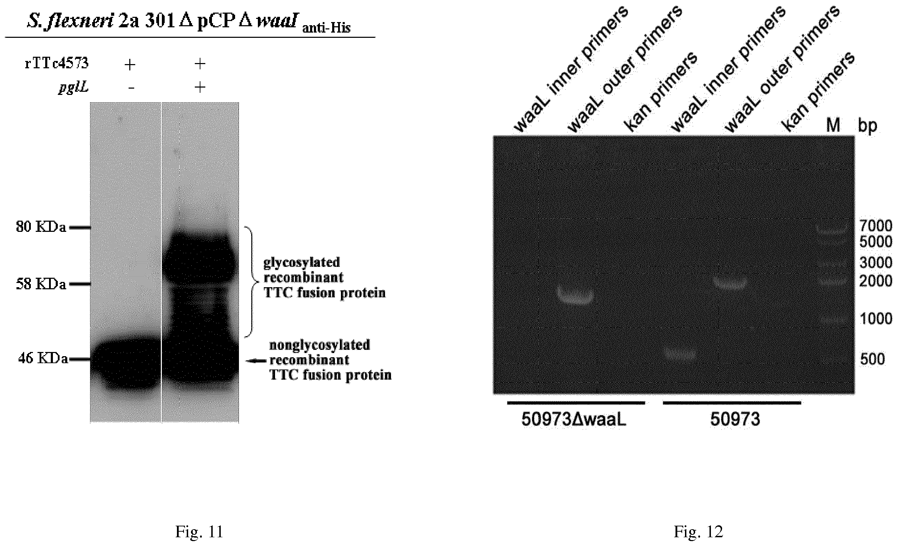

The next day, 1mL of the bacterial liquid induced at 25°C for 20h, was centrifuged to extract bacteria, and the extracted bacteria were slowly suspended in 1X reducing buffer, in a boiling water bath for 10min, to obtain the sample for electrophoresis. The sample was then subjected to SDS-PAGE electrophoresis. After electrophoresis, the protein was transferred onto PVDF membrane by Bio-Lab Semi-Dry Blotter, at a constant voltage of 20V for 1h, and detected Anti-His tag mouse monoclonal antibody. The results are shown in Fig. 11.

Fig. 11 shows that the substrate protein rTTC4573, in the presence of oligosaccharyltransferase PglL, was O-glycosylated.

Example 2. Preparation of *Salmonella paratyphi* A O-polysaccharide-recombinant CTB fusion protein by one-step bioconjugate method

### I. Preparation of O-antigen ligase gene waaL-defective Salmonella paratyphi A

### (I) Preparation of linear targeting DNA fragment

### 1. Design and synthesis of PCR primers

According to the *waaL* gene (GenBank number of CP000026, from positions 3696236 to 3697450) listed in the complete genomic sequence (NC_006511) of *Salmonella paratyphi* A 50973 strain (*S. paratyphi* CMCC50973) on NCBI, as well as upstream and downstream sequences thereof, a pair of primers, i.e., 73waaLu1/73waaLu2 and 73waaLd1/73waaLd2, were designed for each of the upstream (5'end) and the downstream (3'end) of *Salmonella paratyphi* A 50973 *waaI* gene. For the convenience of operation, the *Bam*HI and *Sal*I restriction enzyme cleavage sites were added to the end of the primer for the upstream homologous arm "up", and the *Hin*dIII and *Xho*I restriction enzyme cleavage sites were added to the end of the primer for the downstream homologous arm "down".

In addition, in order to confirm whether the mutant was constructed successfully, a pair of primers (73waaLw1/73waaLw2) located outside the regions of the upstream and downstream homologous arms of *waal* gene in the genome, a pair of internal primers for identification (73waaLn1/73waaLn2) and a pair of primers for kan gene (Kan1/Kan2) were designed to carry out PCR verification, and sequencing verification.

The primers are shown in Table 2 (in Table 2, the underlined sequences refer to the enzyme recognition sites).

**Table 2 Primer sequence listing 2**

| Primer | Sequence (5'→3') | Use |
|---|---|---|
| 73waaLu1 | CGGGATCCAGGCTTTGACTATGTGGA (SEQ ID No.61) | for amplification of up fragment |
| 73waaLu2 | GCGTCGACATCTGGCGATATGAGTATG (SEQ ID No.62) | |
| 73waaLd1 | CCAAGCTTTAGTGCAGGCATATTGGG (SEQ ID No.63) | for amplification of down fragment |
| 73waaLd2 | CCCTCGAGTATCACCTCGCAGAACCT (SEQ ID No.64) | |
| 73waaLw1 | AACACCGGATTACGGATAA (SEQ ID No.65) | for identification of the mutant strain |
| 73waaLw2 | TGCATGGTGGCTGTAGAA (SEQ ID No.66) | |
| 73waaLn1 | AGAAACGGTTGCGAAAAT (SEQ ID No.67) | for identification of the mutant strain |
| 73waaLn2 | ATAGCCGTAGCCCTTGAT (SEQ ID No.68) | |
| Kan1 | GCGTCGACGTGTAGGCTGGAGCTGCTTC (SEQ ID No.69) | for identification of the mutant strain |
| Kan2 | CCAAGCTTATGGGAATTAGCCATGGTCC (SEQ ID No.70) | |

### 2. Construction of linear targeting DNA fragments

1) The genomic DNA of *Salmonella paratyphi* A 50973 strain was used as template, and 73waaLu1/73waaLu2 and 73waaLd1/73waaLd2 were used to amplify the upstream and downstream homologous arms of waaL gene, i.e., up and down, respectively.
   PCR program was as followed: 94°C 10min; 94°C 30s, 50°C 30s, 72°C 30s, 30 cycles; 72°C 10min.
2) Construction of pETKan was performed as disclosed in Example 1.
*3) Bam*HI and SalI were used in double enzyme digestion of the up fragment, to obtain the gene fragment 3; *Bam*HI and *Sal*I were used in double enzyme digestion of the plasmid pETKan, to obtain the large fragment 3; and the gene fragment 3 was ligated to the large fragment 3, to obtain the intermediate vector 3;
   *Hind*III and *Xho*I were used in double enzyme digestion of the down fragment, to obtain the gene fragment 4; *Hind*III and *Xho*I were used in double enzyme digestion of the intermediate vector 3, to obtain the large fragment 4; and the gene fragment 4 was ligated to the large fragment 4, to obtain the intermediate vector 4.
*4) Bam*HI and *Xho*I were used in double enzyme digestion of the intermediate vector 4, to obtain the targeting DNA fragment of interest, flanked by the homologous arms, and having the *kan* gene in the middle region. The nucleotide sequence of the fragment is set forth in SEQ ID No.71.

In SEQ ID No.71, starting from 5' end, the nucleotides from positions 7 to 571 refer to the up fragment, the nucleotides from positions 578 to 2073 refer to the kan gene, and the nucleotides from positions 2080 to 2543 refer to the down fragment.

The DNA fragment set forth in SEQ ID No.71 was used as template, and 73waaLu1 and 73waaLd2 were used as primers, to further amplify the targeting fragment by PCR, to obtain the linear targeting DNA fragment at a concentration of up to 300 ng/µL.

### (II) Construction of S. paratyphi CMCC50973/pKOBEG

Since the plasmid pKOBEG comprised the genes encoding the enzymes necessary for λ-Red recombination system, the plasmid pKOBEG was transformed into *Salmonella paratyphi* A 50973 competent cells by electroporation, and the transformed cells were spread onto LB-plate containing chloramphenicol (the plasmid pKOBEG is chloramphenicol-resistant), and cultured at 30°C overnight, to obtain the positive clone, designated as *S. paratyphi* 50973/pKOBEG strain.

### (III) Transformation of S. paratyphi 50973/pKOBEG with the linear targeting DNA fragments by electroporation

1. The *S.paratyphi* CMCC50973 /pKOBEG was seeded to low salt LB liquid medium containing chloramphenicol at a final concentration of 30µg/mL, cultured at 30°C overnight, and then passaged in low salt LB liquid medium at a volume ratio of 1:100 for furture culture.
2. L-arabinose was added at a final concentration of 1 mmol/L to induce the expression of Red recombination system at 1 h before the OD₆₀₀ value of the culture medium in Step 1 reached 0.6.
3. When the OD₆₀₀ of the culture medium in Step 2 reached 0.6, the *S. paratyphi* CMCC50973 /pKOBEG was transformed with 5µL the targeting DNA fragments as prepared in Step (I) at 300 ng/µL by electroporation.
4. To the transformed cells, 1 mL precooled low salt LB liquid medium was added. The cells were thawed at 30°C for about 2.5 h, and then were spread onto a LB plate containing kanamycin at a concentration of 50µg/mL, and cultured in a 30°C incubator overnight; and the positive clone was screened out.
5. The positive clone was seeded to LB liquid medium containing kanamycin at a concentration of 50µg/mL, and cultured at 42°C and passaged twice (12 h for each passage), thereby resulting in the deletion of the plasmid pKOBEG. The kanamycin-resistant, *waaI*-deleted mutant strain was obtained finally, designated as *S.paratyphi* CMCC50973 *waaL::kan.*

### (IV) The plasmid pCP20 encoding a FRT site-specific recombinase was transformed into S. paratyphi CMCC50973 waaL::kan by electroporation, and cultured in kanamycin-free LB plate containing chloramphenicol at a concentration of 50µg/mL at 30°C. The positive clone of Cm^{r}Km^{s} (chloramphenicol-resistant, kanamycin-sensitive) was screened.

### (V) The positive clone screened in Step (IV), was transferred to liquid LB, and cultured at 42°C for 12 h, to obtain the target gene-deleted mutant free of the kanamycin resistance gene and the plasmid pCP20, designated as S. paratyphi CMCC50973ΔwaaL, i.e., lipopolysaccharide synthesis-deficient Salmonella paratyphi A.

### II. Molecular identification of S. paratyphi CMCC50973ΔwaaL

The genomic DNA of *S. paratyphi* CMCC50973 and *S. paratyphi* CMCC50973Δ*waaL* were used as template, respectively, and a pair of *waaL* inner primers (73waaLn1 / 73waaLn2), a pair of *waaL* outer primers (73waaLw1/73waaLw2) and a pair of kan primers (kan1/kan2) were used to carry out PCR verification, respectively.

The results are shown in Fig. 12. In Fig. 12, 50973Δ*waaL* represents *S. paratyphi* CMCC50973Δ*waaL*; and 50973 represents *S. paratyphi* CMCC50973. Fig. 12 shows that when the genomic DNA of *S. paratyphi* CMCC50973Δ*waaL* was used as template, and the *waaL* inner primers were used as primers to carry out PCR amplification, no target bands appeared; when the genomic DNA of *S. paratyphi* CMCC50973 was used as template, and the *waaL* inner primers were used as primers to carry out PCR amplification, target bands appeared. Moreover, since *S. paratyphi* CMCC50973Δ*waaL* had the *waaL* gene knocked out, the band, obtained when the genomic DNA of *S. paratyphi* CMCC50973Δ*waaL* was used as template, and the *waaL* outer primers were used as primers to carry out PCR amplification, is smaller than the band, obtained when the genomic DNA of *S. paratyphi* CMCC50973 was used as template, and the *waaL* outer primers were used as primers to carry out PCR amplification. Since the Kan resistance gene was finally deleted, when the genomic DNA of *S. paratyphi* CMCC50973Δ*waaL* was used as template, and the kan primers were used as primers to carry out PCR amplification, no target bands appeared.

The above results demonstrate the successful construction of a *waaL* gene-deleted *Salmonella paratyphi* A 50973 mutant with no antibiotic resistance gene, i.e. *S. paratyphi* CMCC50973Δ*waaL*.

### III. Phenotype identification of S. paratyphi CMCC50973ΔwaaL

1 mL culture of *S. paratyphi* CMCC50973Δ*waaL* cultured at 37°C overnight, was centrifuged to collect bacteria. The bacteria were washed with PBS once, and 100µL lysis buffer (the lysis buffer was prepared by well mixing 2mL 10% SDS, 0.4mL 2-mercaptoethanol, 1mL 100% glycerol, 5mL 2M pH6.8 Tris-HCl, 20 µL 10% bromophenol blue and 1.6mL ddH₂O) was added and mixed well. After heating in boiling water for 10 min, 4 µL of 20mg/mL protease K aqueous solution was added, and the reaction was carried out at 60°C for 1-2 h, to obtain lysates. 15µl of the lysates was loaded to carry out SDS-PAGE electrophoresis, the gel was placed in the fixing solution overnight, and a sensitizing solution was added and reacted for 30 min. After washing with deionized water for three times, 15 min for each time, a silver nitrate solution was added and reacted for 20 min, and deionized water was used for washing twice, 1 min for each time. A developing solution was added for color development, the reaction was stopped finally, and deionized water was used for washing.

*S. paratyphi* CMCC50973 was also subjected to the above experiment, as control.

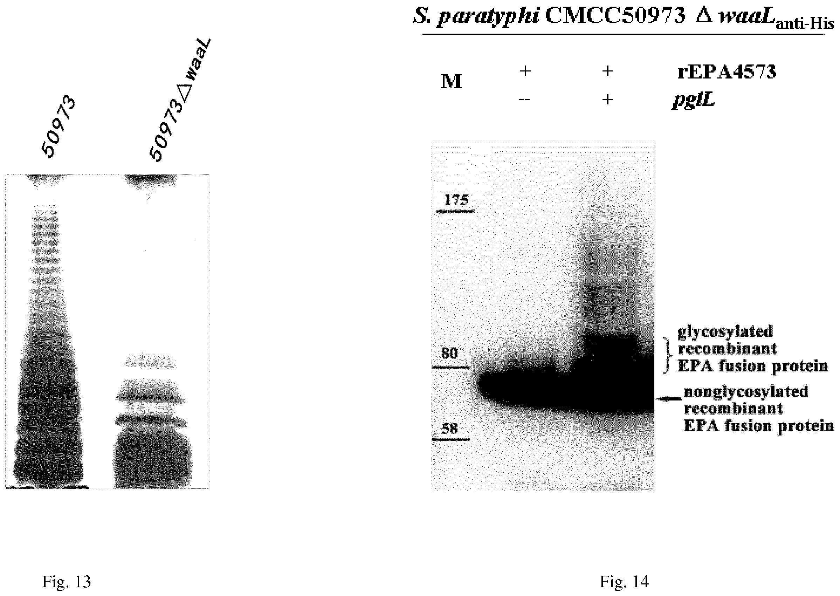

The results are shown in Fig. 13. In Fig. 13, 50973Δ*waaL* represents *S. paratyphi* CMCC50973Δ*waaL*; and 50973 represents *S. paratyphi* CMCC50973. Fig. 13 shows that the wild-type strain *S. paratyphi* CMCC50973 had ladder-like bands, while the mutant strain had no ladder-like bands due to the knockout of O-antigen ligase gene *waaL* (*waaL* functions to ligate O-antigen polysaccharide (OPS) to lipoid A-core oligosaccharide to form lipopolysaccharide(LPS)).

### IV Preparation of Salmonella paratyphi A O-polysaccharide-recombinant EPA fusion protein conjugate by one-step bioconjugate method

### (I) Construction of glycoengineered Salmonella paratyphi A, S. paratyphi CMCC50973ΔwaaL/pETtac28-pglL / pMMB66EH-rEPA4573

The host bacterium *S. paratyphi* CMCC50973Δ*waaL* was transformed with the vectors pETtac28-*pglL* and pMMB66EH-rEPA4573 sequentially by electroporation, and the transformed cells were spread onto LB solid medium containing kanamycinat a final concentration of 50µg/mL and ampicillin a final concentration of 100µg/mL, and the clone formed was the glycoengineered *Salmonella paratyphi* A, *S. paratyphi* CMCC50973Δ*waaL*/pETtac28-*pglL* / pMMB66EH-rEPA4573.

### (II) Glycosylation of the EPA fusion protein and identification thereof

The monoclonal colony of glycoengineered bacterium *S. paratyphi* CMCC50973Δ*waaL*/pETtac28-*pglL* / pMMB66EH-rEPA4573, was seeded in LB medium containing ampicillin at a final concentration of 100µg/mL and kanamycin at a final concentration of 50µg/mL, and cultured at 37°C. When OD₆₀₀ was about 0.6, IPTG was added at a final concentration of 1mM. The culture was cooled to 25°C and induced for 20h, to obtain the glycoprotein rEPA-OPS_{*Spty*50973}.

The next day, 1mL of the bacterial liquid induced at 25°Cfor 20h, was centrifuged to extract bacteria, and the extracted bacteria were slowly suspended in 1X reducing buffer, in a boiling water bath for 10min, to obtain the sample for electrophoresis. The sample was then subjected to 8% SDS-PAGE electrophoresis. After electrophoresis, the protein was transferred onto PVDF membrane by Bio-Lab Semi-Dry Blotter, at a constant voltage of 20V for 1h, and was subjected to western-blot detection using anti-EPA antibody.

Meanwhile, the *S. paratyphi* CMCC50973Δ*waaL* / pMMB66EH-rEPA4573, constructed by transforming *S. paratyphi* CMCC50973Δ*waaL* with pMMB66EH-rEPA4573 but not with pETtac28-*pglL*, was used as control, to carry out the above induction experiment.

The results are shown in Fig. 14. Fig. 14 shows that the substrate protein rEPA4573, in the presence of oligosaccharyltransferase PglL, was O-glycosylated.

### V Preparation of Salmonella paratyphi A O-polysaccharide-recombinant CTB fusion protein conjugate by one-step bioconjugate method

### (I) Construction of glycoengineered Salmonella paratyphi A, S. paratyphi CMCC50973ΔwaaL/pETtac28-pglL / pMMB66EH-rCTB4573

The host bacterium *S. paratyphi* CMCC50973Δ*waaL* was transformed with the vectors pETtac28-*pglL* and pMMB66EH-rCTB4573 sequentially, and spread onto LB solid medium containing kanamycin at a final concentration of 50µg/mL and ampicillin at a final concentration of 100µg/mL, and the clone formed was the glycoengineered *Salmonella paratyphi* A, *S. paratyphi* CMCC50973Δ*waaL*/pETtac28-*pglL* / pMMB66EH-rCTB4573.

### (II) Glycosylation of the recombinant CTB fusion protein and identification thereof

The monoclonal colony of glycoengineered bacterium *S. paratyphi* CMCC50973Δ*waaL*/pETtac28-*pglL* / pMMB66EH-rCTB4573, was seeded in LB medium containing ampicillin at a final concentration of 100µg/mL and kanamycin at a final concentration of 50µg/mL, and cultured at 37°C.When OD₆₀₀ was about 0.6, IPTG was added at a final concentration of 1mM. The culture was cooled to 16°C and induced for 20h, to obtain the glycoprotein rCTB-OPS_{*Spty*50973}.

The next day, 1mL of the bacterial liquid induced at 16°C for 20h, was centrifuged to extract bacteria, and the extracted bacteria were slowly suspended in 1X reducing buffer, in a boiling water bath for 10min, to obtain the sample for electrophoresis. The sample was then subjected to 8% SDS-PAGE electrophoresis. After electrophoresis, the protein was transferred onto PVDF membrane by Bio-Lab Semi-Dry Blotter, at a constant voltage of 20V for 1h, and subjected to western-blot detection using Anti-His tag mouse monoclonal antibody.

The *S. paratyphi* CMCC50973Δ*waaL* / pMMB66EH-rCTB4573, constructed by transforming *S. paratyphi* CMCC50973Δ*waaL* with pMMB66EH-rCTB4573 but not with pETtac28-*pg*/*L*, was used as control, to carry out the induction experiment.

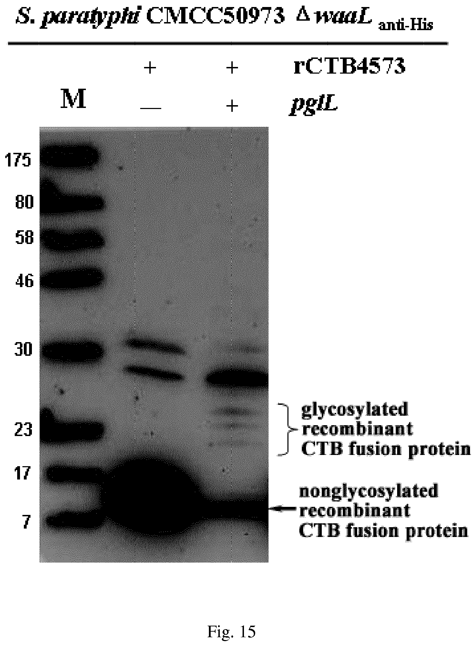

The results are shown in Fig. 15. Fig. 15 shows that the substrate protein rCTB4573, in the presence of O-oligosaccharyltransferase PglL, was O-glycosylated.

Example 3. Modification of the recombinant CTB fusion protein with exogenous O-polysaccharides of *E*. *coli* 0157

When a recombinant fusion protein is modified by an exogenous polysaccharide, it needs a bacterium defective in both O-antigen ligase gene and host O-antigen synthesis, as a host. When O-antigen ligase gene is deleted, no matter the polysaccharides expressed in the host bacterium are endogenous or exogenous, they cannot be utilized in LPS synthesis pathway in the host, while the O-antigen synthesis deficiency in the host ensures that the glycosylation system only utilizes exogenous polysaccharides to modify the recombinant fusion protein, thereby avoiding the contamination due to the modification of the recombinant fusion protein with the endogenous O-antigen polysaccharides in the host. In a host bacterium, a recombinant fusion protein gene, *Neisseria meningitidis* O-oligosaccharyltransferase PglL gene, a gene cluster for synthesis of exogenous polysaccharide, are co-expressed in the host bacterium; and in the presence of O-oligosaccharyltransferase PglL as catalyst, the exogenous polysaccharide is transferred to the recombinant fusion protein.

In the Example, *E*. *coli* K12 W3110 strain is used as host bacterium; for naturally occurring insertional inactivation of the *wbbL* gene of the key glycosyltransferase, rhamnosyl transferase, in the O-antigen synthesis pathway in K12 strain, endogenous O-antigen cannot be synthesized; therefore only the O-antigen ligase gene *waaL* needs to be knocked out to construct a host bacterium defective in both O-antigen ligase and O-antigen synthesis.

### I. Construction of a host bacterium defective in both O-antigen ligase and O-antigen synthesis

### (I) Preparation of linear targeting DNA fragments

### 1. Design and synthesis of PCR primers

According to the genomic sequence (AC_000091.1) of the E. *coli* W3110 strain published on GenBank, the targeting fragment for the *waaL* gene (from positions 3696236 to 3697450) was designed. Two fragments of 41 bp were selected from the upstream and downstream of the *waaL* gene, respectively, and used as the homologous arms, and 3' end of each homologous arm was linked with one of the primers for amplification of chloramphenicol-resistant gene flanked by FRT sites, thereby obtaining the chloramphenicol-resistant gene fragment flanked by the 41 bp upstream and downstream homologous arms of the *waaL* gene and FRT sites, by PCR amplification; and the primers 3110waaL up 5' and 3110waaL down 3' located outside the *waaL* gene region were designed to identify whether the *waaL* gene was knocked out or not.

The primers are shown in Table 3.

**Table 3 Primer sequence listing 3**

| Primer name | Primer sequence (5'→3') | Note | Use of the primer pair |
|---|---|---|---|
| 3110waaLcat 5' | | the waaL homologous arms were underlined | For amplification of chloramphenicol-resistant gene fragment comprising upstream and downstream homologous arms of the *waaL* gene |
| 3110waaLcat 3' | | | |
| 3110waaL up 5' | TACAAATAGTATCCCCAAC(SEQ ID No.74) | - | primers for identifying *waaL* knock-out |
| 3110waaL down 3' | AATTAACCTCAACAGTCAA(SEQ ID No.75) | | |

### 2. Construction of linear targeting DNA fragments

The plasmid pKD3 was used as a template, and 3110waaLcat 5' and 3110waaLcat 3' were used as primers to carry out PCR amplification, to obtain the chloramphenicol-resistant gene fragment flanked by the 41 bp upstream and downstream homologous arms of the *waaL* gene and FRT sites. The fragment was set forth in SEQ ID No.76.

### (II) Obtainment of O-antigen ligase gene waaL-defective E. coli W3110 (E.coli W3110ΔwaaL)

### 1. Preparation of E. coli W3110/pKD46

1) *E*. *coli* W3110 was seeded in LB liquid medium, after incubating at 37°C overnight, the culture was transferred to 100mL LB liquid medium at a volume ratio of 1:100, and cultured at 37°C until OD₆₀₀ reached 0.6.
2) The bacteria were collected by centrifugation, and washed with the autoclaved 10% glycerol (v/v) for four times, and finally re-suspended in 400µL 10% glycerol, to obtain *E*. *coli* W3110 competent cells for use in electrotransformation, sub-packaged for later use.
3) The plasmid pKD46 (the replicon of pKD46 is sensitive to temperature, and lost when cultured at 37°C, and the plasmid comprises the genes encoding three recombinases of Red recombination system and controlled by arabinose promoter) was transformed into the prepared *E*. *coli* W3110 competent cell by electroporation, and the transformed cells were spread onto the LB plate containing ampicillin at a final concentration of 100µg/mL. After culturing at 30°C overnight, the obtained positive clone was the *E*. *coli* W31 10/pKD46 strain.

### 2. Preparation of E. coli W3110 waaL::cat

1) The *E*. *coli* W3110/pKD46 was cultured at 30°C overnight, and then passaged in LB liquid medium containing 100µg/mL ampicillin at a volume ratio of 1:100 for furture culture.
2) When the OD₆₀₀ value was about 0.2, L-arabinose was added at a final concentration of 0.2g/100ml to induce the expression of Red recombination system; when the OD₆₀₀ value was about 0.6, the *E*. *coli* W3110/pKD46 competent cell was prepared.
3) 10µL of the linear targeting DNA fragments prepared in the step (I) was transformed into the *E*. *coli* W3110/pKD46 competent cell by electroporation.
4) 1 mL precooled low salt LB liquid medium was added quickly. The cells were thawed at 30°C for about 2.5 h, and then were spread onto a LB plate containing chloramphenicol at a concentration of 30µg/mL, and cultured in a 30°C incubator overnight.
5) The positive clone was screened out. Its genomic DNA was extracted and used as template, and 3110waaL up 5' and 3110waaL down 3' were used as primers to carry out PCR identification, to obtain PCR amplification product. Meanwhile, the genomic DNA of *E*. *coli* W3110 was used as template, to carry out the PCR amplification, as control.
   The PCR amplification product of *E*. *coli* W3110 was of 1444bp, while the PCR amplification product of the positive clone in Step 5) was of 1246bp when the *waaL* gene was replaced with the *cat* gene, the positive clone was designated as *E*. *coli* W3110 *waaL::cat*/pKD46*.*
6) the *E*. *coli* W3110 *waaL::cat*/pKD46 was seeded in LB liquid medium containing chloramphenicol at a final concentration of 30µg/mL, and passaged for three times at 37°C (culturing for 12 h for each passage), resulting in the deletion of the plasmid pKD46, to obtain *E*. *coli* W3110 *waaL::cat* strain.

### 3. Obtainment of E. coli W3110ΔwaaL strain

1) The pCP20 was transformed into *E*. *coli* W3110 *waaL::cat* cell by electroporation, and the transformed cells were spread onto LB plate containing 100µg/mL ampicillin, to obtain W3110 *waaL*::*cat*/pCP20 strain.
2) The monoclonal colony of E. *coli* W3110 *waaL::cat*/pCP20 was seeded in LB liquid medium, cultured at 30°C until OD₆₀₀ was about 0.6, and then cultured at 42°C overnight.
   (the replicon of pCP20 is sensitive to temperature, and lost when cultured at 42°C, the plasmid comprises the DNA encoding FLP recombinase, controlled by temperature-sensitive promoter, and the expression of FLP recombinase was induced when cultured at 42°C, with the loss of the plasmid.)
3) The monoclonal colony was isolated by streak plate method from the bacterial liquid cultured at 42°C overnight on LB plate, and the colonies were grown on LB plate with or without chloramphenicol. The colonies, which grew on LB plate but not on LB plate containing chloramphenicol, were picked and designated as *E*. *coli* W3110Δ*waaL.* Its genomic DNA was used as template, and 3110waaL up 5' and 3110waaL down 3' were used as primers to carry out PCR identification, to obtain the PCR amplification product. The genomic DNA of *E*. *coli* W3110 and E. *coli* W3110 *waaL::cat* were separately used as template, to carry out the PCR amplification, as control.

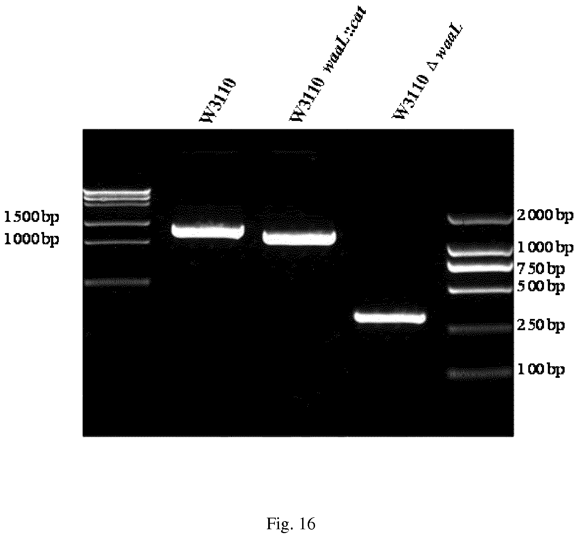

The results are shown in Fig. 16. The PCR amplification fragment of E. *coli* W3110 was of 1444bp, and the PCR amplification fragment of W3110 *waaL::cat* resulted from the replacement of the *waaL* gene with the *cat* gene was of 1246bp, and the PCR amplification fragment *of E. coli* W3110Δ*waaL* with the cat gene deleted, was of 316bp. Fig. 16 shows the successful construction of *E. coli* W3110Δ*waaL*.
II. The construction of the recombinant CTB fusion protein expression vector pMMB66EH-rCTB4573 was performed as disclosed in Step II (III) in Example 1.
III. Construction of *Neisseria meningitidis* O-oligosaccharyltransferase PglL expression vector pETtac28-*pglL* was performed as disclosed in Step II (I) in Example 1.
IV. Construction of expression vector pACYC184-O157 comprising a gene cluster for synthesis of the exogenous bacterial polysaccharide

(I) According to the gene sequence of 0157 (GenBank:AF061251.1) on NCBI website, the following primers were designed:
   O157-5' (the underlined sequence is the *Asc*I enzyme recognition site):
   O157-3'(the underlined sequence is the *Not*I enzyme recognition site):
      5'-ATATGCGGCCGCTTAATCCAGCCATTCGGTATGGAACACACCTTCTTT-3'( SE Q ID No.78)
(II) The genomic DNA ofE. *coli* 0157 was used as template, and O157-5' and O157-3' were used as primers to carry out PCR amplification, to obtain PCR amplification product, i.e., the gene cluster for 0157 polysaccharide synthesis, as set forth in SEQ ID No.79.
(III) According to the gene sequences of the plasmid pACYC184, the following primers were deisgned:
   p184-5': 5'-ATATGGCGCGCCTCTGAGTTACAACAGTCCGC-3'( SEQ ID No.80) (the underlined sequence is the *Asc*I enzyme recognition site)
   p184-3': 5'-ATAAGCGGCCGCTTCAGGTGCTACATTTGAAG-3'( SEQ ID No.81) (the underlined sequence is the *Not*I enzyme recognition site)
(IV) pACYC184 was used as template, and p184-5' and p184-3' were used as primers to carry out PCR amplification, to obtain the PCR amplification product, i.e., the linear pACYC184 vector fragment, set forth in SEQ ID No. 82.
(V) *Asc*I and *Not*I were used in double enzyme digestion of the DNA molecule set forth in SEQ ID No.79, to obtain a gene fragment; *Asc*I and *Not*I were used in double enzyme digestion of the DNA molecule set forth in SEQ ID No.82, to obtain a large fragment; the gene fragment was ligated to the large fragment, to obtain the recombinant plasmid, designated as pACYC184-O157. The pACYC184-O157 was sequenced, and the result was correct.

### V. Construction of glycoengineered E. coli

The three recombinant plasmids pMMB66EH-rCTB4573, pETtac28-*pglL* and pACYC184-O157 were transformed into the host bacterium *E.coli* W3110Δ*waaL* by electroporation, and the transformed cells were spread onto LB plate containing ampicillin, kanamycin and chloramphenicol, and the formed clone was the glycoengineered *E*. *coli, E*. *coli* W3110Δ*waaL*/ pMMB66EH-rCTB4573/pETtac28-*pglL*/pACYC184-O157.

### VI. Glycosylation of the recombinant CTB fusion protein r CTB4573-OPS_{EcO157} and identification thereof

The monoclonal colony of glycoengineered bacterium *E*. *coli* W3110Δ*waaL*/ pMMB66EH-rCTB4573/pETtac28-*pglL*/ pACYC184-O157, was seeded to LB medium containing 100µg/mL ampicillin, 50µg/mL kanamycin and 30µg/mL chloramphenicol, and cultured at 37°C. When OD₆₀₀ was about 0.6, IPTG was added at a final concentration of 1mM. The culture was cooled to 16°C and induced for 20h, to express rCTB4573-OPS_{*Ec*O157}.

1mL of the bacterial liquid induced at 16°C for 20h, was centrifuged to extract bacteria, and the the extracted bacteria were slowly suspended in 1X reducing buffer, in a boiling water bath for 10min, to obtain the sample for electrophoresis. The sample was then subjected to 15% SDS-PAGE electrophoresis. After electrophoresis, the protein was transferred onto PVDF membrane by Bio-Lab Semi-Dry Blotter, at a constant voltage of 20V for 1h, and detected by rabbit anti-*E*. *coli* 0157 antiserum.

*E*. *coli* W3110Δ*waaL*/ pMMB66EH-rCTB4573/pETtac28-*pglL* was used as control.

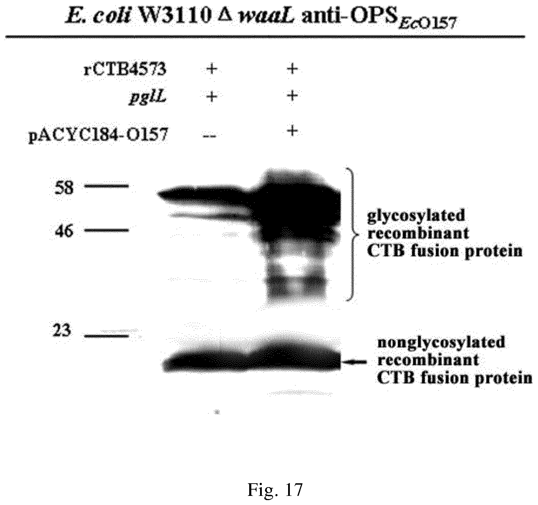

The results are shown in Fig. 17. Fig. 17 shows that the substrate protein rCTB4573, in the presence of oligosaccharyltransferase PglL and a gene cluster for synthesis of exogenous polysaccharide, was O-glycosylated.

### Industrial application

As compared with the polysaccharide proteins prepared by the existing chemical crosslinking methods, the bacterial polysaccharide-modified recombinant fusion proteins prepared by the method of the invention have the advantages such as homogeneous polysaccharide binding sites, and controllable quality; and the preparation of bacterial polysaccharide-modified recombinant fusion proteins by culturing engineering bacteria, has the advantages such as high-efficient production, low production cost, and good biosafety, as it does not comprises the steps such as the preparation of polysaccharides, the preparation of carrier protein, and chemical crosslinking. Specific antibodies against bacterial polysaccharides can be obtained by immunizing a mouse with the bacterial polysaccharide-modified recombinant fusion protein prepared by the method of the invention. The use of the bacterial polysaccharide-modified recombinant fusion protein in the preparation of a bacterial polysaccharide-protein conjugate vaccine, can avoid a variety of problems concerning the culture of pathogenic bacteria, enhance the homogeneity of vaccines and production efficiency, and reduce the cost for vaccine preparation. The invention has a wide application prospect in the preparation of polysaccharide-modified protein vaccines.

## Claims

1. A method for preparing a bacterial polysaccharide-modified recombinant fusion protein, comprising co-expressing a recombinant fusion protein and *Neisseria meningitidis* O-oligosaccharyltransferase PgIL in a O-antigen ligase gene-defective bacterium, and linking a polysaccharide endogenous or exogenous for the bacterium to the recombinant fusion protein by the *Neisseria meningitidis* O-oligosaccharyltransferase PgIL, to obtain the bacterial polysaccharide-modified recombinant fusion protein;
wherein, the recombinant fusion protein comprises an N-terminal signal peptide, a peptide fragment having a glycosylation site of *Neisseria meningitidis* O-oligosaccharyltransferase PgIL which is the serine at position 63 starting from N-terminus of *Neisseria meningitidis* pilin PilE, and a carrier protein;
the carrier protein is a nontoxic mutant of Pseudomonas aeruginosa exotoxin A or a fragment of a bacterial toxin protein, wherein the fragment of a bacterial toxin protein is cholera toxin B subunit or fragment C of tetanus toxin;
and
the peptide fragment having a glycosylation site of *Neisseria meningitidis* O-oligosaccharyltransferase PgIL is a peptide fragment comprising at least the amino acids from positions 55 to 66 starting from N-terminus of *Neisseria meningitidis* pilin PilE and is any one of the following peptide fragments:
(1) the peptide fragment set forth in the amino acid sequence from positions 128 to 156 starting from N-terminus of SEQ ID No.32;
(2) the peptide fragment set forth in the amino acid sequence from positions 128 to 154 starting from N-terminus of SEQ ID No.34;
(3) the peptide fragment set forth in the amino acid sequence from positions 128 to 152 starting from N-terminus of SEQ ID No.36;
(4) the peptide fragment set forth in the amino acid sequence from positions 128 to 150 starting from N-terminus of SEQ ID No.38;
(5) the peptide fragment set forth in the amino acid sequence from positions 22 to 40 starting from N-terminus of SEQ ID No.48;
(6) the peptide fragment set forth in the amino acid sequence from positions 22 to 36 starting from N-terminus of SEQ ID No.50.

2. The method according to claim 1, **characterized in that** the nontoxic mutant of the bacterial toxin protein is a nontoxic mutant of *Pseudomonas aeruginosa* exotoxin A; the nontoxic mutant of *Pseudomonas aeruginosa exotoxin* A is set forth in the amino acid sequence from positions 20 to 631 starting from N-terminus of SEQ ID No.46.

3. The method according to claim 1, **characterized in that** the fragment of the bacterial toxin protein is cholera toxin B subunit or fragment C of tetanus toxin; the cholera toxin B subunit is set forth in the amino acid sequence from positions 20 to 122 starting from N-terminus of SEQ ID No.32; the fragment C of tetanus toxin is set forth in the amino acid sequence from positions 20 to 455 starting from N-terminus of SEQ ID No.60.

4. The method according to claim 1, **characterized in that** the recombinant fusion protein has an amino acid sequence set forth in any one of:
(1) the amino acid sequence set forth in SEQ ID No.32;
(2) the amino acid sequence set forth in SEQ ID No.34;
(3) the amino acid sequence set forth in SEQ ID No.36;
(4) the amino acid sequence set forth in SEQ ID No.38;
(6) the amino acid sequence set forth in SEQ ID No.46;
(7) the amino acid sequence set forth in SEQ ID No.48;
(8) the amino acid sequence set forth in SEQ ID No.50;
(10) the amino acid sequence set forth in SEQ ID No.56;
(11) the amino acid sequence set forth in SEQ ID No.58; and
(12) the amino acid sequence set forth in SEQ ID No.60.

5. The method according to claim 1, **characterized in that** the recombinant fusion protein is introduced into the bacterium by a recombinant expression vector, the recombinant expression vector is obtained by inserting a gene encoding the recombinant fusion protein into the multiple cloning site of pMMB66EH.

6. The method according to claim 1, **characterized in that** the *Neisseria meningitidis* O-oligosaccharyltransferase PgIL is introduced into the bacterium by a recombinant expression vector, the recombinant expression vector is obtained by inserting an expression cassette of the *Neisseria meningitidis* O-oligosaccharyltransferase PgIL into the multiple cloning site of pET28a(+); and the expression cassette of the *Neisseria meningitidis* O-oligosaccharyltransferase PgIL is set forth in SEQ ID No.30.

7. The method according to claim 1, **characterized in that** the polysaccharide exogenous for the bacterium is obtained by introducing a recombinant expression vector comprising a gene cluster for polysaccharide synthesis into the bacterium; the gene cluster for polysaccharide synthesis is set forth in SEQ ID No.79.

8. The method according to claim 7, wherein, the recombinant expression vector comprising a gene cluster for polysaccharide synthesis is prepared by the following method: subjecting the DNA molecule set forth in SEQ ID No.79 to double enzyme digestion by *Asc*l and *Not*l*,* to obtain a gene fragment; subjecting the DNA molecule set forth in SEQ ID No.82 to double enzyme digestion by *Asc*l and *Not*l*,* to obtain a large vector fragment; and ligating the gene fragment to the large vector fragment to obtain the recombinant expression vector comprising a gene cluster for polysaccharide synthesis.

9. The method according to claim 1, **characterized in that** the O-antigen ligase gene-defective bacterium is a O-antigen ligase gene-defective *Shigella flexneri,* a O-antigen ligase gene-defective *Salmonella paratyphi* A or a O-antigen ligase gene-defective *Escherichia coli;* preferably, the O-antigen ligase gene-defective *Escherichia coli* is a strain of *E. coli* K12.

10. The method according to claim 1, **characterized by** further comprising the steps of cell lysis, centrifugation and collection of supernatant, desalting and/or separation and purification, after the expression.

11. A bacterial polysaccharide-modified recombinant fusion protein prepared by the method according to claim 1.

12. A vaccine comprising the bacterial polysaccharide-modified recombinant fusion protein according to claim 11.

13. The bacterial polysaccharide-modified recombinant fusion protein according to claim 11, for use in (a) inducing generation of antibodies against O-polysaccharides in animal; and/or
(b) preventing and/or treating a disease caused by a bacterium.

## Patentansprüche

1. Verfahren zur Herstellung eines mit einem bakterielles Polysaccharid modifizierten rekombinanten Fusionsprotein, umfassend Co-Expression eines rekombinanten Fusionsproteins und *Neisseria meningitidis* O-Oligosaccharyltransferase PgIL in einem Bakterium mit defektem O-Antigen-Ligase-Gen, und Anbinden eines für das Bakterium endogenen oder exogenen Polysaccharids an das rekombinante Fusionsprotein durch die *Neisseria meningitidis* O-Oligosaccharyltransferase PgIL, unter Erhalt des mit dem bakteriellen Polysaccharid modifizierten rekombinanten Fusionsproteins;
wobei das rekombinante Fusionsprotein ein N-terminales Signalpeptid, ein Peptidfragment, welches eine Glykosylierungsstelle von *Neisseria meningitidis O-*Oligosaccharyltransferase PgIL aufweist, welche das Serin an Position 63 ausgehend vom N-Terminus von *Neisseria meningitidis* Pilin PilE ist, und ein Trägerprotein umfasst;
das Trägerprotein eine nicht-toxische Mutante von *Pseudomonas aeruginosa Exotoxin A* oder ein Fragment eines bakteriellen Toxinproteins ist, wobei das Fragment eines bakteriellen Toxinproteins Cholera-Toxin-B-Untereinheit oder Fragment C von Tetanus-Toxin ist;
und
das Peptidfragment, welches eine Glykosylierungsstelle von *Neisseria meningitidis* O-Oligosaccharyltransferase PgIL aufweist, ein Peptidfragment ist, welches mindestens die Aminosäuren von Position 55 bis 66 ausgehend vom N-Terminus von *Neisseria meningitidis* pilin PilE umfasst und eines der folgenden Peptidfragmente ist:
(1) das Peptidfragment wie dargestellt in der Aminosäuresequenz von Positionen 128 bis 156 ausgehend vom N-Terminus von SEQ ID Nr. 32;
(2) das Peptidfragment wie dargestellt in der Aminosäuresequenz von Positionen 128 bis 154 ausgehend vom N-Terminus von SEQ ID Nr. 34;
(3) das Peptidfragment wie dargestellt in der Aminosäuresequenz von Positionen 128 bis 152 ausgehend vom N-Terminus von SEQ ID Nr. 36;
(4) das Peptidfragment wie dargestellt in der Aminosäuresequenz von Positionen 128 bis 150 ausgehend vom N-Terminus von SEQ ID Nr. 38;
(5) das Peptidfragment wie dargestellt in der Aminosäuresequenz von Positionen 22 bis 40 ausgehend vom N-Terminus von SEQ ID Nr. 48;
(6) das Peptidfragment wie dargestellt in der Aminosäuresequenz von Positionen 22 bis 36 ausgehend vom N-Terminus von SEQ ID Nr. 50.

2. Verfahren gemäß Anspruch 1, **dadurch gekennzeichnet, dass** die nicht-toxische Mutante des bakteriellen Toxinproteins eine nicht-toxische Mutante von *Pseudomonas aeruginosa Exotoxin A* ist, wobei die nicht-toxische Mutante von *Pseudomonas aeruginosa Exotoxin A* dargestellt ist in der Aminosäuresequenz von Position 20 bis 631 ausgehend vom N-Terminus von SEQ ID Nr. 46.

3. Verfahren gemäß Anspruch 1, **dadurch gekennzeichnet, dass** das Fragment des bakteriellen Toxinproteins Cholera-Toxin-B-Untereinheit oder Fragment C von Tetanus-Toxin ist; wobei die Cholera-Toxin-B-Untereinheit dargestellt ist in der Aminosäuresequenz von Positionen 20 bis 122 ausgehend vom N-Terminus von SEQ ID Nr. 32; das Fragment C von Tetanustoxin dargestellt ist in der Aminosäuresequenz von Positionen 20 bis 455 ausgehend vom N-Terminus von SEQ ID Nr. 60.

4. Verfahren nach Anspruch 1, **dadurch gekennzeichnet, dass** das rekombinante Fusionsprotein eine Aminosäuresequenz wie dargestellt in einer der folgenden aufweist:
(1) der Aminosäuresequenz dargestellt in SEQ ID Nr. 32;
(2) der Aminosäuresequenz dargestellt in SEQ ID Nr. 34;
(3) der Aminosäuresequenz dargestellt in SEQ ID Nr. 36;
(4) der Aminosäuresequenz dargestellt in SEQ ID Nr. 38;
(6) der Aminosäuresequenz dargestellt in SEQ ID Nr. 46;
(7) der Aminosäuresequenz dargestellt in SEQ ID Nr. 48;
(8) der Aminosäuresequenz dargestellt in SEQ ID Nr. 50;
(10) der Aminosäuresequenz dargestellt in SEQ ID Nr. 56;
(11) der Aminosäuresequenz dargestellt in SEQ ID Nr. 58; und
(12) der Aminosäuresequenz dargestellt in SEQ ID Nr. 60.

5. Verfahren gemäß Anspruch 1, **dadurch gekennzeichnet, dass** das rekombinante Fusionsprotein in das Bakterium mittels eines rekombinanten Expressionsvektors eingeführt wird, wobei der rekombinante Expressionsvektor erhalten wurde durch Einfügen eines das rekombinante Fusionsprotein kodierenden Gens in die multiple Klonierungsstelle von pMMB66EH.

6. Verfahren nach Anspruch 1, **dadurch gekennzeichnet, dass** die *Neisseria meningitidis* O-Oligosaccharyltransferase PgIL in das Bakterium mittels eines rekombinanten Expressionsvektors eingeführt wird, wobei der rekombinante Expressionsvektor erhalten wird durch Einfügen einer Expressionskassette für das *Neisseria meningitidis* O-Oligosaccharyltransferase PgIL in die multiple Klonierungsstelle von pET28a(+); und die Expressionskassette der *Neisseria meningitidis* O-Oligosaccharyltransferase PgIL in SEQ ID Nr. 30 dargestellt ist.

7. Verfahren gemäß Anspruch 1, **dadurch gekennzeichnet, dass** das für das Bakterium exogene Polysaccharid erhalten wird durch Einführen eines rekombinanten Expressionsvektors umfassend ein Gencluster für Polysaccharidsynthese in das Bakterium, wobei das Gencluster für Polysaccharidsynthese in SEQ ID Nr. 79 dargestellt ist.

8. Verfahren gemäß Anspruch 7, worin der rekombinante Expressionsvektor umfassend ein Gencluster für Polysaccharidsynthese hergestellt wird durch die folgende Methode: Unterwerfen des in SEQ ID Nr. 79 dargestellten DNA-Moleküls einer doppelten Enzymverdauung durch *Asc*l und *Not*l unter Erhalt eines Genfragments; Unterwerfen des in SEQ ID Nr. 82 dargestellten DNA-Moleküls einer doppelten Enzymverdauung durch *Asc*l und *Not*l unter Erhalt eines großen Vektorfragments; und Ligieren des Genfragments an das große Vektorfragment unter Erhalt des rekombinanten Expressionsvektors umfassend ein Gencluster für Polysaccharidsynthese.

9. Verfahren gemäß Anspruch 1, **dadurch gekennzeichnet, dass** das Bakterium mit defektem O-Antigen-Ligase-Gen ein O-Antigen-Ligase gendefektes *Shigella flexneri,* ein O-Antigen-Ligase-gendefektes *Salmonella paratyphi* A oder ein O-Antigen-Ligase gendefektes *Escherichia coli* ist;
wobei vorzugsweise das O-Antigen-Ligase gendefekte *Escherichia coli* ein Stamm von *E. coli* K12 ist.

10. Verfahren gemäß Anspruch 1, **dadurch gekennzeichnet, dass** es nach der Expression weiter umfasst die Schritte der Zelllyse, Zentrifugation und Sammeln des Überstandes, Entsalzen und/oder Trennen und Reinigen.

11. Ein mit einem bakteriellen Polysaccharid modifiziertes rekombinantes Fusionsprotein, welches durch das Verfahren gemäß Anspruch 1 hergestellt ist.

12. Eine Vakzine umfassend das mit einem bakteriellen Polysaccharid modifizierte rekombinante Fusionsprotein gemäß Anspruch 11.

13. Das mit einem bakteriellen Polysaccharid modifizierte rekombinante Fusionsprotein gemäß Anspruch 11 zur Verwendung bei
(a) dem Induzieren der Erzeugung von Antikörpern gegen O-Polysaccharide in einem Tier; und/oder
(b) Verhindern und/oder Behandeln einer Erkrankung, die durch ein Bakterium verursacht ist.

## Revendications

1. Procédé pour préparer une protéine de fusion recombinante modifiée par un polysaccharide bactérien, comprenant la co-expression d'une protéine de fusion recombinante et de la O-oligosaccharyltransférase PgIL de *Neisseria meningitidis* dans une bactérie déficiente en gène de O-antigène ligase, et la liaison d'un polysaccharide endogène ou exogène pour la bactérie à la protéine de fusion recombinante par la O-oligosaccharyltransférase PgIL de *Neisseria meningitidis,* pour obtenir la protéine de fusion recombinante modifiée par un polysaccharide bactérien;
où, la protéine de fusion recombinante comprend un peptide signal N-terminal, un fragment peptidique ayant un site de glycosylation de la O-oligosaccharyltransférase PgIL de *Neisseria meningitidis* qui est la sérine en position 63 à partir de l'extrémité N-terminale de la piline PilE de *Neisseria meningitidis,* et une protéine porteuse;
la protéine porteuse est un mutant non toxique de l'exotoxine A de Pseudomonas aeruginosa ou un fragment d'une protéine toxine bactérienne, où le fragment d'une protéine toxine bactérienne est la sous-unité B de la toxine cholérique ou le fragment C de la toxine tétanique;
et
le fragment peptidique ayant un site de glycosylation de la O-oligosaccharyltransférase PgIL de *Neisseria meningitidis* est un fragment peptidique comprenant au moins les acides aminés des positions 55 à 66 à partir de l'extrémité N-terminale de la piline PilE de *Neisseria meningitidis* et est l'un quelconque des fragments peptidiques suivants:
(1) le fragment peptidique présenté dans la séquence d'acides aminés des positions 128 à 156 à partir de l'extrémité N-terminale de SEQ ID N°32;
(2) le fragment peptidique présenté dans la séquence d'acides aminés des positions 128 à 154 à partir de l'extrémité N-terminale de SEQ ID N°34;
(3) le fragment peptidique présenté dans la séquence d'acides aminés des positions 128 à 152 à partir de l'extrémité N-terminale de SEQ ID N°36;
(4) le fragment peptidique présenté dans la séquence d'acides aminés des positions 128 à 150 à partir de l'extrémité N-terminale de SEQ ID N°38;
(5) le fragment peptidique présenté dans la séquence d'acides aminés des positions 22 à 40 à partir de l'extrémité N-terminale de SEQ ID N°48;
(6) le fragment peptidique présenté dans la séquence d'acides aminés des positions 22 à 36 à partir de l'extrémité N-terminale de SEQ ID N°50.

2. Procédé selon la revendication 1, **caractérisé en ce que** le mutant non toxique de la protéine toxine bactérienne est un mutant non toxique de l'exotoxine A de *Pseudomonas aeruginosa;* le mutant non toxique de l'exotoxine A de *Pseudomonas aeruginosa* est présenté dans la séquence d'acides aminés des positions 20 à 631 à partir de l'extrémité N-terminale de SEQ ID N°46.

3. Procédé selon la revendication 1, **caractérisé en ce que** le fragment de la protéine toxine bactérienne est la sous-unité B de la toxine cholérique ou le fragment C de la toxine tétanique; la sous-unité B de la toxine cholérique est présentée dans la séquence d'acides aminés des positions 20 à 122 à partir de l'extrémité N-terminale de SEQ ID N°32; le fragment C de la toxine tétanique est présenté dans la séquence d'acides aminés des positions 20 à 455 à partir de l'extrémité N-terminale de SEQ ID N°60.

4. Procédé selon la revendication 1, **caractérisé en ce que** la protéine de fusion recombinante a une séquence d'acides aminés présentée dans l'une quelconque de:
(1) la séquence d'acides aminés présentée dans SEQ ID N°32;
(2) la séquence d'acides aminés présentée dans SEQ ID N°34;
(3) la séquence d'acides aminés présentée dans SEQ ID N°36;
(4) la séquence d'acides aminés présentée dans SEQ ID N°38;
(6) la séquence d'acides aminés présentée dans SEQ ID N°46;
(7) la séquence d'acides aminés présentée dans SEQ ID N°48;
(8) la séquence d'acides aminés présentée dans SEQ ID N°50;
(10) la séquence d'acides aminés présentée dans SEQ ID N°56;
(11) la séquence d'acides aminés présentée dans SEQ ID N°58; et
(12) la séquence d'acides aminés présentée dans SEQ ID N°60.

5. Procédé selon la revendication 1, **caractérisé en ce que** la protéine de fusion recombinante est introduite dans la bactérie par un vecteur d'expression recombinant, le vecteur d'expression recombinant est obtenu par insertion d'un gène codant la protéine de fusion recombinante dans le site de clonage multiple de pMMB66EH.

6. Procédé selon la revendication 1, **caractérisé en ce que** la O-oligosaccharyltransférase PgIL de *Neisseria meningitidis* est introduite dans la bactérie par un vecteur d'expression recombinant, le vecteur d'expression recombinant est obtenu par insertion d'une cassette d'expression de la O-oligosaccharyltransférase PgIL de *Neisseria meningitidis* dans le site de clonage multiple de pET28A(+); et la cassette d'expression de la O-oligosaccharyltransférase PgIL de *Neisseria meningitidis* est présentée dans SEQ ID N°30.

7. Procédé selon la revendication 1, **caractérisé en ce que** le polysaccharide exogène pour la bactérie est obtenu par introduction d'un vecteur d'expression recombinant comprenant un groupe de gènes pour la synthèse de polysaccharides dans la bactérie; le groupe de gènes pour la synthèse de polysaccharides est présenté dans SEQ ID N°79.

8. Procédé selon la revendication 7, où le vecteur d'expression recombinant comprenant un groupe de gènes pour la synthèse de polysaccharides est préparé par le procédé suivant: exposition de la molécule d'ADN présentée dans SEQ ID N°79 à une double digestion enzymatique par *Asc*l et *Not*l*,* pour obtenir un fragment de gène; exposition de la molécule d'ADN présentée dans SEQ ID N° 82 à une double digestion enzymatique par *Asc*l et *Not*l*,* pour obtenir un grand fragment de vecteur; et ligature du fragment de gène au grand fragment de vecteur pour obtenir le vecteur d'expression recombinant comprenant un groupe de gènes pour la synthèse de polysaccharides.

9. Procédé selon la revendication 1, **caractérisée en ce que** la bactérie déficiente en gène de O-antigène ligase est une *Shigella flexneri* déficiente en gène de O-antigène ligase, une *Salmonella paratyphi* A déficiente en gène de O-antigène ligase ou une *Escherichia coli* déficiente en gène de O-antigène ligase;
de préférence, la *Escherichia coli* déficiente en gène de O-antigène ligase est une souche de *E. coli* K12.

10. Procédé selon la revendication 1, **caractérisé en ce qu'**il comprend en outre les étapes de lyse cellulaire, de centrifugation et de collecte de surnageant, de retrait des sels et/ou de séparation et de purification, après l'expression.

11. Protéine de fusion recombinante modifiée par un polysaccharide bactérien préparée par le procédé selon la revendication 1.

12. Vaccin comprenant la protéine de fusion recombinante modifiée par un polysaccharide bactérien selon la revendication 11.

13. Protéine de fusion recombinante modifiée par un polysaccharide bactérien selon la revendication 11, destinée à être utilisée pour (a) induire la production d'anticorps contre les O-polysaccharides chez un animal; et/ou
(b) prévenir et/ou traiter une maladie causée par une bactérie.


### SEQUENCE LISTING

<110> INSTITUTE OF BIOTECHNOLOGY, ACADEMY OF MILITARY MEDICAL SCIENCES, CHINA
<120> Method for preparing a bacterial polysaccharide-modified recombinant fusion protein and use of the protein
<130> 64136P EP-WO
<150> CN201410709118.1
   <151> 2014-11-27
<160> 82
<170> PatentIn version 3.5
<210> 1
   <211> 19
   <212> DNA
   <213> artificial
<220>
   <223> primer sequence
<400> 1
   aagattctgc ctttggacc 19
<210> 2
   <211> 21
   <212> DNA
   <213> artificial
<220>
   <223> primer sequence
<400> 2
   gtggttgaag agttctgtat g 21
<210> 3
   <211> 19
   <212> DNA
   <213> artificial
<220>
   <223> primer sequence
<400> 3
   tgctttgacg gtatacagc 19
<210> 4
   <211> 20
   <212> DNA
   <213> artificial
<220>
   <223> primer sequence
<400> 4
   acttccacag gttgaattcg 20
<210> 5
   <211> 20
   <212> DNA
   <213> artificial
<220>
   <223> primer sequence
<400> 5
   aagcaggttt cttctattgg 20
<210> 6
   <211> 21
   <212> DNA
   <213> artificial
<220>
   <223> primer sequence
<400> 6
   gaacacatta ccgattacag g 21
<210> 7
   <211> 19
   <212> DNA
   <213> artificial
<220>
   <223> primer sequence
<400> 7
   catcaatccg ttactcact 19
<210> 8
   <211> 18
   <212> DNA
   <213> artificial
<220>
   <223> primer sequence
<400> 8
   actaccagca acaatacg 18
<210> 9
   <211> 20
   <212> DNA
   <213> artificial
<220>
   <223> primer sequence
<400> 9
   tgcgagagag aggggataac 20
<210> 10
   <211> 19
   <212> DNA
   <213> artificial
<220>
   <223> primer sequence
<400> 10
   cgccttttcc atcagtttc 19
<210> 11
   <211> 27
   <212> DNA
   <213> artificial
<220>
   <223> primer sequence
<400> 11
   cgggatccgg gtatgggaag aatcaag 27
<210> 12
   <211> 26
   <212> DNA
   <213> artificial
<220>
   <223> primer sequence
<400> 12
   gcgtcgactc agaaatgcta cggtgt 26
<210> 13
   <211> 27
   <212> DNA
   <213> artificial
<220>
   <223> primer sequence
<400> 13
   ccaagctttg gacctttaga caatcaa 27
<210> 14
   <211> 26
   <212> DNA
   <213> artificial
<220>
   <223> primer sequence
<400> 14
   ccctcgagat gttaggaagc ataccg 26
<210> 15
   <211> 18
   <212> DNA
   <213> artificial
<220>
   <223> primer sequence
<400> 15
   tgggtgggat tacaaggt 18
<210> 16
   <211> 19
   <212> DNA
   <213> artificial
<220>
   <223> primer sequence
<400> 16
   ccaatgacta acacggaaa 19
<210> 17
   <211> 19
   <212> DNA
   <213> artificial
<220>
   <223> primer sequence
<400> 17
   ctacagatgc tggcgaata 19
<210> 18
   <211> 18
   <212> DNA
   <213> artificial
<220>
   <223> primer sequence
<400> 18
   tcactacagt tgggatgg 18
<210> 19
   <211> 20
   <212> DNA
   <213> artificial
<220>
   <223> primer sequence
<400> 19
   agccgattgt ctgttgtgcc 20
<210> 20
   <211> 20
   <212> DNA
   <213> artificial
<220>
   <223> primer sequence
<400> 20
   ttgtcactga agcgggaagg 20
<210> 21
   <211> 28
   <212> DNA
   <213> artificial
<220>
   <223> primer sequence
<400> 21
   gcgtcgacgt gtaggctgga gctgcttc 28
<210> 22
   <211> 28
   <212> DNA
   <213> artificial
<220>
   <223> primer sequence
<400> 22
   ccaagcttat gggaattagc catggtcc 28
<210> 23
   <211> 2828
   <212> DNA
   <213> artificial
<220>
   <223> linear targeting DNA fragment
<400> 23
   ggatccgggt atgggaagaa tcaagtagag tgtcgttcta caagtatgtc tcttgcagat      60
   ttgccagctc aaaccgtttt tcaaaattta aatcttgaaa taataaccaa taagttgaca     120
   tcggagataa gatgacctca acattatttt tctctctcga gaaaaaaaac tggatagcgt     180
   actggaacag agctctcgta ttcttattca ttaccaccta ttttttgggt gggattacaa     240
   ggtataaaca tcttattgtt attcttatga caataacgac aatcgtctat ctctgcaaac     300
   ggccaaaaca ctatctctca ctatttaaaa catttctttt tggtagtgtt gccatattaa     360
   ctattgctgc attgctgtca cttcttcaat cccctgatgc aggtgctagc atgaaggaag     420
   ttttcaaagc tattattgag aatactttac tatgcacaat agcaataccg gtcatattga     480
   gagacgagaa aagagaagat gtcgaaaaaa tcgttttctt ctcatttatt agtgcgttgg     540
   gcttacgctg tttttctgaa ttgattacct attataagga ctatcaacaa gggataatgc     600
   cattcgcaga ttatagacac cgtagcattt ctgagtcgac gtgtaggctg gagctgcttc     660
   gaagttccta tactttctag agaataggaa cttcggaata ggaacttcaa gatcccctca     720
   cgctgccgca agcactcagg gcgcaagggc tgctaaagga agcggaacac gtagaaagcc     780
   agtccgcaga aacggtgctg accccggatg aatgtcagct actgggctat ctggacaagg 840
   gaaaacgcaa gcgcaaagag aaagcaggta gcttgcagtg ggcttacatg gcgatagcta 900
   gactgggcgg ttttatggac agcaagcgaa ccggaattgc cagctggggc gccctctggt 960
   aaggttggga agccctgcaa agtaaactgg atggctttct tgccgccaag gatctgatgg 1020
   cgcaggggat caagatctga tcaagagaca ggatgaggat cgtttcgcat gattgaacaa 1080
   gatggattgc acgcaggttc tccggccgct tgggtggaga ggctattcgg ctatgactgg 1140
   gcacaacaga caatcggctg ctctgatgcc gccgtgttcc ggctgtcagc gcaggggcgc 1200
   ccggttcttt ttgtcaagac cgacctgtcc ggtgccctga atgaactgca ggacgaggca 1260
   gcgcggctat cgtggctggc cacgacgggc gttccttgcg cagctgtgct cgacgttgtc 1320
   actgaagcgg gaagggactg gctgctattg ggcgaagtgc cggggcagga tctcctgtca 1380
   tctcaccttg ctcctgccga gaaagtatcc atcatggctg atgcaatgcg gcggctgcat 1440
   acgcttgatc cggctacctg cccattcgac caccaagcga aacatcgcat cgagcgagca 1500
   cgtactcgga tggaagccgg tcttgtcgat caggatgatc tggacgaaga gcatcagggg 1560
   ctcgcgccag ccgaactgtt cgccaggctc aaggcgcgca tgcccgacgg cgaggatctc 1620
   gtcgtgaccc atggcgatgc ctgcttgccg aatatcatgg tggaaaatgg ccgcttttct 1680
   ggattcatcg actgtggccg gctgggtgtg gcggaccgct atcaggacat agcgttggct 1740
   acccgtgata ttgctgaaga gcttggcggc gaatgggctg accgcttcct cgtgctttac 1800
   ggtatcgccg ctcccgattc gcagcgcatc gccttctatc gccttcttga cgagttcttc 1860
   tgagcgggac tctggggttc gaaatgaccg accaagcgac gcccaacctg ccatcacgag 1920
   atttcgattc caccgccgcc ttctatgaaa ggttgggctt cggaatcgtt ttccgggacg 1980
   ccggctggat gatcctccag cgcggggatc tcatgctgga gttcttcgcc caccccagct 2040
   tcaaaagcgc tctgaagttc ctatactttc tagagaatag gaacttcgga ataggaacta 2100
   aggaggatat tcatatggac catggctaat tcccataagc tttggacctt tagacaatca 2160
   atagggccac ataattttgc gctattcatc tggtttggca ctggtttatt agggctggta 2220
   agtcttatga tgctatactg tgcaatattg aaagagtgta taaaaaatgg cgtcaagaat 2280
   aaatatcgct caccatataa tgcatattat ataatcttac tatcttttat aggttatttt 2340
   gttatccgtg gaaacgtaga acaaattgaa ccaaatttat taggcgttta cgccggctta 2400
   ttattagcga tgaaaaacaa gtaaaaaata aaaaaggctg cataatgcgg cctttttatt 2460
   tattcaaacc aattatgaat aacctcttcg aatctttgag ttacgccatc ccaactgtag 2520
   tgatcaaaca caaagtcctg accttgcttt gcaactgctg ttaattcagg attcgctaac 2580
   gtttttaaaa tatcgctgct tatggagtct gcagtcatag gttcttttaa atggaatccc 2640
   gtagtatttt ctttgacaaa ttccgtcatc cccccacgcg tactcactaa aacaggctta 2700
   ccagcaccca tagcttcaat tgccaccatg caaaatggtt cctggaactg ggaaggaatg 2760
   acgactaaat ctgccagtgg gtaataacaa tacatctttt caggcggtat gcttcctaac 2820
   atctcgag 2828
<210> 24
   <211> 28
   <212> DNA
   <213> artificial
<220>
   <223> primer sequence
<400> 24
   gcgtcgacgt gtaggctgga gctgcttc 28
<210> 25
   <211> 28
   <212> DNA
   <213> artificial
<220>
   <223> primer sequence
<400> 25
   ccaagcttat gggaattagc catggtcc 28
<210> 26
   <211> 604
   <212> PRT
   <213> Neisseria meningitidis
<400> 26
   Met Pro Ala Glu Thr Thr Val Ser Gly Ala His Pro Ala Ala Lys Leu
   1 5 10 15
   Pro Ile Tyr Ile Leu Pro Cys Phe Leu Trp Ile Gly Ile Val Pro Phe
   20 25 30
   Thr Phe Ala Leu Lys Leu Lys Pro Ser Pro Asp Phe Tyr His Asp Ala
   35 40 45
   Ala Ala Ala Ala Gly Leu Ile Val Leu Leu Phe Leu Thr Ala Gly Lys
   50 55 60
   Lys Leu Phe Asp Val Lys Ile Pro Ala Ile Ser Phe Leu Leu Phe Ala
   65 70 75 80
   Met Ala Ala Phe Trp Tyr Leu Gln Ala Arg Leu Met Asn Leu Ile Tyr
   85 90 95
   Pro Gly Met Asn Asp Ile Val Ser Trp Ile Phe Ile Leu Leu Ala Val
               100                 105                 110
   Ser Ala Trp Ala Cys Arg Ser Leu Val Ala His Phe Gly Gln Glu Arg
           115                 120                 125
   Ile Val Thr Leu Phe Ala Trp Ser Leu Leu Ile Gly Ser Leu Leu Gln
       130                 135                 140
   Ser Cys Ile Val Val Ile Gln Phe Ala Gly Trp Glu Asp Thr Pro Leu
   145                 150                 155                 160
   Phe Gln Asn Ile Ile Val Tyr Ser Gly Gln Gly Val Ile Gly His Ile
                   165                 170                 175
   Gly Gln Arg Asn Asn Leu Gly His Tyr Leu Met Trp Gly Ile Leu Ala
               180                 185                 190
   Ala Ala Tyr Leu Asn Gly Gln Arg Lys Ile Pro Ala Ala Leu Gly Val
           195                 200                 205
   Ile Cys Leu Ile Met Gln Thr Ala Val Leu Gly Leu Val Asn Ser Arg
       210                 215                 220
   Thr Ile Leu Thr Tyr Ile Ala Ala Ile Ala Leu Ile Leu Pro Phe Trp
   225                 230                 235                 240
   Tyr Phe Arg Ser Asp Lys Ser Asn Arg Arg Thr Met Leu Gly Ile Ala
                   245                 250                 255
   Ala Ala Val Phe Leu Thr Ala Leu Phe Gln Phe Ser Met Asn Thr Ile
               260                 265                 270
   Leu Glu Thr Phe Thr Gly Ile Arg Tyr Glu Thr Ala Val Glu Arg Val
           275                 280                 285
   Ala Asn Gly Gly Phe Thr Asp Leu Pro Arg Gln Ile Glu Trp Asn Lys
       290                 295                 300
   Ala Leu Ala Ala Phe Gln Ser Ala Pro Ile Phe Gly His Gly Trp Asn
   305                 310                 315                 320
   Ser Phe Ala Gln Gln Thr Phe Leu Ile Asn Ala Glu Gln His Asn Ile
                   325                 330                 335
   Tyr Asp Asn Leu Leu Ser Asn Leu Phe Thr His Ser His Asn Ile Val
               340                 345                 350
   Leu Gln Leu Leu Ala Glu Met Gly Ile Ser Gly Thr Leu Leu Val Ala
           355                 360                 365
   Ala Thr Leu Leu Thr Gly Ile Ala Gly Leu Leu Lys Arg Pro Leu Thr
       370                 375                 380
   Pro Ala Ser Leu Phe Leu Ile Cys Thr Leu Ala Val Ser Met Cys His
   385                 390                 395                 400
   Ser Met Leu Glu Tyr Pro Leu Trp Tyr Val Tyr Phe Leu Ile Pro Phe
                   405                 410                 415
   Gly Leu Met Leu Phe Leu Ser Pro Ala Glu Ala Ser Asp Gly Ile Ala
               420                 425                 430
   Phe Lys Lys Ala Ala Asn Leu Gly Ile Leu Thr Ala Ser Ala Ala Ile
           435                 440                 445
   Phe Ala Gly Leu Leu His Leu Asp Trp Thr Tyr Thr Arg Leu Val Asn
       450                 455                 460
   Ala Phe Ser Pro Ala Thr Asp Asp Ser Ala Lys Thr Leu Asn Arg Lys
   465                 470                 475                 480
   Ile Asn Glu Leu Arg Tyr Ile Ser Ala Asn Ser Pro Met Leu Ser Phe
                   485                 490                 495
   Tyr Ala Asp Phe Ser Leu Val Asn Phe Ala Leu Pro Glu Tyr Pro Glu
               500                 505                 510
   Thr Gln Thr Trp Ala Glu Glu Ala Thr Leu Lys Ser Leu Lys Tyr Arg
           515                 520                 525
   Pro His Ser Ala Thr Tyr Arg Ile Ala Leu Tyr Leu Met Arg Gln Gly
       530                 535                 540
   Lys Val Ala Glu Ala Lys Gln Trp Met Arg Ala Thr Gln Ser Tyr Tyr
   545                 550                 555                 560
   Pro Tyr Leu Met Pro Arg Tyr Ala Asp Glu Ile Arg Lys Leu Pro Val
                   565                 570                 575
   Trp Ala Pro Leu Leu Pro Glu Leu Leu Lys Asp Cys Lys Ala Phe Ala
               580                 585                 590
   Ala Ala Pro Gly His Pro Glu Ala Lys Pro Cys Lys
   595 600
<210> 27
   <211> 1815
   <212> DNA
   <213> Neisseria meningitidis
<400> 27
   atgcccgctg aaacgaccgt atccggcgcg caccccgccg ccaaactgcc gatttacatc      60
   ctgccctgct tcctttggat aggcatcgtc ccctttacct tcgcgctcaa actgaaaccg     120
   tcgcccgact tttaccacga tgccgccgcc gcagccggcc tgattgtcct gttgttcctc     180
   acggcaggaa aaaaactgtt tgatgtcaaa atccccgcca tcagcttcct tctgtttgca     240
   atggcggcgt tttggtatct tcaggcacgc ctgatgaacc tgatttaccc cggtatgaac     300
   gacatcgtct cttggatttt catcttgctc gccgtcagcg cgtgggcctg ccggagcttg     360
   gtcgcacact tcggacaaga acgcatcgtg accctgtttg cctggtcgct gcttatcggc     420
   tccctgcttc aatcctgcat cgtcgtcatc cagtttgccg gctgggaaga cacccctctg     480
   tttcaaaaca tcatcgttta cagcgggcaa ggcgtaatcg gacacatcgg gcagcgcaac     540
   aacctcggac actacctcat gtggggcata ctcgccgccg cctacctcaa cggacaacga     600
   aaaatccccg ccgccctcgg cgtaatctgc ctgattatgc agaccgccgt tttaggtttg     660
   gtcaactcgc gcaccatctt gacctacata gccgccatcg ccctcatcct tcccttctgg     720
   tatttccgtt cggacaaatc caacaggcgg acgatgctcg gcatagccgc agccgtattc     780
   cttaccgcgc tgttccaatt ttccatgaac accattctgg aaacctttac tggcatccgc     840
   tacgaaactg ccgtcgaacg cgtcgccaac ggcggtttca cagacttgcc gcgccaaatc     900
   gaatggaata aagcccttgc cgccttccag tccgccccga tattcgggca cggctggaac     960
   agttttgccc aacaaacctt cctcatcaat gccgaacagc acaacatata cgacaacctc    1020
   ctcagcaact tgttcaccca ttcccacaac atcgtcctcc aactccttgc agagatggga    1080
   atcagcggca cgcttctggt tgccgcaacc ctgctgacgg gcattgccgg gctgcttaaa    1140
   cgccccctga cccccgcatc gcttttccta atctgcacgc ttgccgtcag tatgtgccac    1200
   agtatgctcg aatatccttt gtggtatgtc tatttcctca tccctttcgg actgatgctc    1260
   ttcctgtccc ccgcagaggc ttcagacggc atcgccttca aaaaagccgc caatctcggc    1320
   atactgaccg cctccgccgc catattcgca ggattgctgc acttggactg gacatacacc    1380
   cggctggtta acgccttttc ccccgccact gacgacagtg ccaaaaccct caaccggaaa    1440
   atcaacgagt tgcgctatat ttccgcaaac agtccgatgc tgtcctttta tgccgacttc    1500
   tccctcgtaa acttcgccct gccggaatac cccgaaaccc agacttgggc ggaagaagca    1560
   accctcaaat cactaaaata ccgcccccac tccgccacct accgcatcgc cctctacctg    1620
   atgcggcaag gcaaagttgc agaagcaaaa caatggatgc gggcgacaca gtcctattac    1680
   ccctacctga tgccccgata cgccgacgaa atccgcaaac tgcccgtatg ggcgccgctg    1740
   ctacccgaac tgctcaaaga ctgcaaagcc ttcgccgccg cgcccggtca tccggaagca    1800
   aaaccctgca aatga                                                     1815
<210> 28
   <211> 34
   <212> DNA
   <213> artificial
<220>
   <223> primer sequence
<400> 28
   atcgagatct actgcataat tcgtgtcgct caag 34
<210> 29
   <211> 34
   <212> DNA
   <213> artificial
<220>
   <223> primer sequence
<400> 29
   atcgagatct gtctcatgag cggatacata tttg 34
<210> 30
   <211> 2488
   <212> DNA
   <213> artificial
<220>
   <223> expression cassette of PglL
<400> 30
   atcgagatct actgcataat tcgtgtcgct caaggcgcac tcccgttctg gataatgttt      60
   tttgcgccga catcataacg gttctggcaa atattctgaa atgagctgtt gacaattaat     120
   catcggctcg tataatgtgt ggaattgtga gcggataaca atttcacaca ggaaacagaa     180
   ttcatgcccg ctgaaacgac cgtatccggc gcgcaccccg ccgccaaact gccgatttac     240
   atcctgccct gcttcctttg gataggcatc gtccccttta ccttcgcgct caaactgaaa      300
   ccgtcgcccg acttttacca cgatgccgcc gccgcagccg gcctgattgt cctgttgttc     360
   ctcacggcag gaaaaaaact gtttgatgtc aaaatccccg ccatcagctt ccttctgttt     420
   gcaatggcgg cgttttggta tcttcaggca cgcctgatga acctgattta ccccggtatg     480
   aacgacatcg tctcttggat tttcatcttg ctcgccgtca gcgcgtgggc ctgccggagc     540
   ttggtcgcac acttcggaca agaacgcatc gtgaccctgt ttgcctggtc gctgcttatc     600
   ggctccctgc ttcaatcctg catcgtcgtc atccagtttg ccggctggga agacacccct     660
   ctgtttcaaa acatcatcgt ttacagcggg caaggcgtaa tcggacacat cgggcagcgc 720
   aacaacctcg gacactacct catgtggggc atactcgccg ccgcctacct caacggacaa 780
   cgaaaaatcc ccgccgccct cggcgtaatc tgcctgatta tgcagaccgc cgttttaggt 840
   ttggtcaact cgcgcaccat cttgacctac atagccgcca tcgccctcat ccttcccttc 900
   tggtatttcc gttcggacaa atccaacagg cggacgatgc tcggcatagc cgcagccgta 960
   ttccttaccg cgctgttcca attttccatg aacaccattc tggaaacctt tactggcatc 1020
   cgctacgaaa ctgccgtcga acgcgtcgcc aacggcggtt tcacagactt gccgcgccaa 1080
   atcgaatgga ataaagccct tgccgccttc cagtccgccc cgatattcgg gcacggctgg 1140
   aacagttttg cccaacaaac cttcctcatc aatgccgaac agcacaacat atacgacaac 1200
   ctcctcagca acttgttcac ccattcccac aacatcgtcc tccaactcct tgcagagatg 1260
   ggaatcagcg gcacgcttct ggttgccgca accctgctga cgggcattgc cgggctgctt 1320
   aaacgccccc tgacccccgc atcgcttttc ctaatctgca cgcttgccgt cagtatgtgc 1380
   cacagtatgc tcgaatatcc tttgtggtat gtctatttcc tcatcccttt cggactgatg 1440
   ctcttcctgt cccccgcaga ggcttcagac ggcatcgcct tcaaaaaagc cgccaatctc 1500
   ggcatactga ccgcctccgc cgccatattc gcaggattgc tgcacttgga ctggacatac 1560
   acccggctgg ttaacgcctt ttcccccgcc actgacgaca gtgccaaaac cctcaaccgg 1620
   aaaatcaacg agttgcgcta tatttccgca aacagtccga tgctgtcctt ttatgccgac 1680
   ttctccctcg taaacttcgc cctgccggaa taccccgaaa cccagacttg ggcggaagaa 1740
   gcaaccctca aatcactaaa ataccgcccc cactccgcca cctaccgcat cgccctctac 1800
   ctgatgcggc aaggcaaagt tgcagaagca aaacaatgga tgcgggcgac acagtcctat 1860
   tacccctacc tgatgccccg atacgccgac gaaatccgca aactgcccgt atgggcgccg 1920
   ctgctacccg aactgctcaa agactgcaaa gccttcgccg ccgcgcccgg tcatccggaa 1980
   gcaaaaccct gcaaatgaaa gcttggctgt tttggcggat gagagaagat tttcagcctg 2040
   atacagatta aatcagaacg cagaagcggt ctgataaaac agaatttgcc tggcggcagt 2100
   agcgcggtgg tcccacctga ccccatgccg aactcagaag tgaaacgccg tagcgccgat 2160
   ggtagtgtgg ggtctcccca tgcgagagta gggaactgcc aggcatcaaa taaaacgaaa 2220
   ggctcagtcg aaagactggg cctttcgttt tatctgttgt ttgtcggtga acgctctcct 2280
   gagtaggaca aatccgccgg gagcggattt gaacgttgcg aagcaacggc ccggagggtg 2340
   gcgggcagga cgcccgccat aaactgccag gcatcaaatt aagcagaagg ccatcctgac 2400
   ggatggcctt tttgcgtttc tacaaactct tttgtttatt tttctaaata cattcaaata 2460
   tgtatccgct catgagacag atctcgat 2488
<210> 31
   <211> 513
   <212> DNA
   <213> artificial
<220>
   <223> sequence encoding rCTB4573
<400> 31
   gaattcatga agaaaatttg gctggcctta gccggcctgg ttctggcatt cagcgccagc      60
   gcaaccccgc agaacatcac cgacctgtgc gccgagtacc acaacaccca aatttatacc     120
   ctgaacgaca aaatttttag ctacaccgag agcctggcag gcaagcgcga gatggccatc     180
   atcaccttca agaacggcgc cattttccag gtggaggtgc cgggcagcca gcacatcgac     240
   agtcagaaga aggccatcga gcgcatgaag gacaccctgc gcatcgccta cctgaccgag     300
   gccaaggtgg agaagctgtg cgtgtggaac aacaagaccc cgcacgccat cgccgcaatc     360
   agcatggcca acgaccagaa cgccaccagc gccgtgaccg agtactatct gaaccatggc     420
   gagtggccgg gtaataacac cagcgccggc gtggccacaa gcagtgagat caagggcggc     480
   ggatcccacc atcaccacca ccattaaaag ctt                                 513
<210> 32
   <211> 166
   <212> PRT
   <213> artificial
<220>
   <223> recombinant fusion protein rCTB4573
<400> 32
   Met Lys Lys Ile Trp Leu Ala Leu Ala Gly Leu Val Leu Ala Phe Ser
   1               5                   10                  15
   Ala Ser Ala Thr Pro Gln Asn Ile Thr Asp Leu Cys Ala Glu Tyr His
               20                  25                  30
   Asn Thr Gln Ile Tyr Thr Leu Asn Asp Lys Ile Phe Ser Tyr Thr Glu
           35                  40                  45
   Ser Leu Ala Gly Lys Arg Glu Met Ala Ile Ile Thr Phe Lys Asn Gly
       50                  55                  60
   Ala Ile Phe Gln Val Glu Val Pro Gly Ser Gln His Ile Asp Ser Gln
   65                  70                  75                  80
   Lys Lys Ala Ile Glu Arg Met Lys Asp Thr Leu Arg Ile Ala Tyr Leu
                   85                  90                  95
   Thr Glu Ala Lys Val Glu Lys Leu Cys Val Trp Asn Asn Lys Thr Pro
               100                 105                 110
   His Ala Ile Ala Ala Ile Ser Met Ala Asn Asp Gln Asn Ala Thr Ser
           115                 120                 125
   Ala Val Thr Glu Tyr Tyr Leu Asn His Gly Glu Trp Pro Gly Asn Asn
       130                 135                 140
   Thr Ser Ala Gly Val Ala Thr Ser Ser Glu Ile Lys Gly Gly Gly Ser
   145                 150                 155                 160
   His His His His His His
                   165
<210> 33
   <211> 504
   <212> DNA
   <213> artificial
<220>
   <223> sequence encoding rCTB4571
<400> 33
   gaattcatga agaaaatttg gctggcctta gccggcctgg ttctggcatt cagcgccagc       60
   gcaaccccgc agaacatcac cgacctgtgc gccgagtacc acaacaccca aatttatacc      120
   ctgaacgaca aaatttttag ctacaccgag agcctggcag gcaagcgcga gatggccatc      180
   atcaccttca agaacggcgc cattttccag gtggaggtgc cgggcagcca gcacatcgac      240
   agtcagaaga aggccatcga gcgcatgaag gacaccctgc gcatcgccta cctgaccgag      300
   gccaaggtgg agaagctgtg cgtgtggaac aacaagaccc cgcacgccat cgccgcaatc      360
   agcatggcca acgaccagaa cgccaccagc gccgtgaccg agtactatct gaaccatggc      420
   gagtggccgg gtaataacac cagcgccggc gtggccacaa gcagtgaggg cggcggccac      480
   catcaccacc accattaaaa gctt                                             504
<210> 34
   <211> 163
   <212> PRT
   <213> artificial
<220>
   <223> recombinant fusion protein rCTB4571
<400> 34
   Met Lys Lys Ile Trp Leu Ala Leu Ala Gly Leu Val Leu Ala Phe Ser
   1               5                   10                  15
   Ala Ser Ala Thr Pro Gln Asn Ile Thr Asp Leu Cys Ala Glu Tyr His
   20 25 30
   Asn Thr Gln Ile Tyr Thr Leu Asn Asp Lys Ile Phe Ser Tyr Thr Glu
   35 40 45
   Ser Leu Ala Gly Lys Arg Glu Met Ala Ile Ile Thr Phe Lys Asn Gly
   50 55 60
   Ala Ile Phe Gln Val Glu Val Pro Gly Ser Gln His Ile Asp Ser Gln
   65 70 75 80
   Lys Lys Ala Ile Glu Arg Met Lys Asp Thr Leu Arg Ile Ala Tyr Leu
   85 90 95
   Thr Glu Ala Lys Val Glu Lys Leu Cys Val Trp Asn Asn Lys Thr Pro
   100 105 110
   His Ala Ile Ala Ala Ile Ser Met Ala Asn Asp Gln Asn Ala Thr Ser
   115 120 125
   Ala Val Thr Glu Tyr Tyr Leu Asn His Gly Glu Trp Pro Gly Asn Asn
   130 135 140
   Thr Ser Ala Gly Val Ala Thr Ser Ser Glu Gly Gly Gly His His His
   145 150 155 160
   His His His
<210> 35
   <211> 498
   <212> DNA
   <213> artificial
<220>
   <223> sequence encoding rCTB4569
<400> 35
   gaattcatga agaaaatttg gctggcctta gccggcctgg ttctggcatt cagcgccagc      60
   gcaaccccgc agaacatcac cgacctgtgc gccgagtacc acaacaccca aatttatacc     120
   ctgaacgaca aaatttttag ctacaccgag agcctggcag gcaagcgcga gatggccatc     180
   atcaccttca agaacggcgc cattttccag gtggaggtgc cgggcagcca gcacatcgac     240
   agtcagaaga aggccatcga gcgcatgaag gacaccctgc gcatcgccta cctgaccgag     300
   gccaaggtgg agaagctgtg cgtgtggaac aacaagaccc cgcacgccat cgccgcaatc     360
   agcatggcca acgaccagaa cgccaccagc gccgtgaccg agtactatct gaaccatggc     420
   gagtggccgg gtaataacac cagcgccggc gtggccacaa gcggcggcgg ccaccatcac     480
   caccaccatt aaaagctt                                                   498
<210> 36
   <211> 161
   <212> PRT
   <213> artificial
<220>
   <223> recombinant fusion protein rCTB4569
<400> 36
   Met Lys Lys Ile Trp Leu Ala Leu Ala Gly Leu Val Leu Ala Phe Ser
   1               5                   10                  15
   Ala Ser Ala Thr Pro Gln Asn Ile Thr Asp Leu Cys Ala Glu Tyr His
               20                  25                  30
   Asn Thr Gln Ile Tyr Thr Leu Asn Asp Lys Ile Phe Ser Tyr Thr Glu
           35                  40                  45
   Ser Leu Ala Gly Lys Arg Glu Met Ala Ile Ile Thr Phe Lys Asn Gly
       50                  55                  60
   Ala Ile Phe Gln Val Glu Val Pro Gly Ser Gln His Ile Asp Ser Gln
   65                  70                  75                  80
   Lys Lys Ala Ile Glu Arg Met Lys Asp Thr Leu Arg Ile Ala Tyr Leu
                   85                  90                  95
   Thr Glu Ala Lys Val Glu Lys Leu Cys Val Trp Asn Asn Lys Thr Pro
               100                 105                 110
   His Ala Ile Ala Ala Ile Ser Met Ala Asn Asp Gln Asn Ala Thr Ser
           115                 120                 125
   Ala Val Thr Glu Tyr Tyr Leu Asn His Gly Glu Trp Pro Gly Asn Asn
       130                 135                 140
   Thr Ser Ala Gly Val Ala Thr Ser Gly Gly Gly His His His His His
   145                 150                 155                 160
   His
<210> 37
   <211> 492
   <212> DNA
   <213> artificial
<220>
   <223> sequence encoding rCTB4567
<400> 37
   gaattcatga agaaaatttg gctggcctta gccggcctgg ttctggcatt cagcgccagc        60
   gcaaccccgc agaacatcac cgacctgtgc gccgagtacc acaacaccca aatttatacc       120
   ctgaacgaca aaatttttag ctacaccgag agcctggcag gcaagcgcga gatggccatc       180
   atcaccttca agaacggcgc cattttccag gtggaggtgc cgggcagcca gcacatcgac       240
   agtcagaaga aggccatcga gcgcatgaag gacaccctgc gcatcgccta cctgaccgag       300
   gccaaggtgg agaagctgtg cgtgtggaac aacaagaccc cgcacgccat cgccgcaatc       360
   agcatggcca acgaccagaa cgccaccagc gccgtgaccg agtactatct gaaccatggc       420
   gagtggccgg gtaataacac cagcgccggc gtggccggcg gcggccacca tcaccaccac       480
   cattaaaagc tt                                                           492
<210> 38
   <211> 159
   <212> **PRT**
   <213> artificial
<220>
   <223> recombinant fusion protein rCTB4567
<400> 38
   Met Lys Lys Ile Trp Leu Ala Leu Ala Gly Leu Val Leu Ala Phe Ser
   1               5                   10                  15
   Ala Ser Ala Thr Pro Gln Asn Ile Thr Asp Leu Cys Ala Glu Tyr His
               20                  25                  30
   Asn Thr Gln Ile Tyr Thr Leu Asn Asp Lys Ile Phe Ser Tyr Thr Glu
           35                  40                  45
   Ser Leu Ala Gly Lys Arg Glu Met Ala Ile Ile Thr Phe Lys Asn Gly
       50                  55                  60
   Ala Ile Phe Gln Val Glu Val Pro Gly Ser Gln His Ile Asp Ser Gln
   65                  70                  75                  80
   Lys Lys Ala Ile Glu Arg Met Lys Asp Thr Leu Arg Ile Ala Tyr Leu
                   85                  90                  95
   Thr Glu Ala Lys Val Glu Lys Leu Cys Val Trp Asn Asn Lys Thr Pro
               100                 105                 110
   His Ala Ile Ala Ala Ile Ser Met Ala Asn Asp Gln Asn Ala Thr Ser
           115                 120                 125
   Ala Val Thr Glu Tyr Tyr Leu Asn His Gly Glu Trp Pro Gly Asn Asn
       130                 135                 140
   Thr Ser Ala Gly Val Ala Gly Gly Gly His His His His His His
   145                 150                 155
<210> 39
   <211> 489
   <212> DNA
   <213> artificial
<220>
   <223> sequence encoding rCTB4566
<400> 39
   gaattcatga agaaaatttg gctggcctta gccggcctgg ttctggcatt cagcgccagc        60
   gcaaccccgc agaacatcac cgacctgtgc gccgagtacc acaacaccca aatttatacc       120
   ctgaacgaca aaatttttag ctacaccgag agcctggcag gcaagcgcga gatggccatc       180
   atcaccttca agaacggcgc cattttccag gtggaggtgc cgggcagcca gcacatcgac       240
   agtcagaaga aggccatcga gcgcatgaag gacaccctgc gcatcgccta cctgaccgag       300
   gccaaggtgg agaagctgtg cgtgtggaac aacaagaccc cgcacgccat cgccgcaatc       360
   agcatggcca acgaccagaa cgccaccagc gccgtgaccg agtactatct gaaccatggc       420
   gagtggccgg gtaataacac cagcgccggc gtgggcggcg gccaccatca ccaccaccat       480
   taaaagctt                                                               489
<210> 40
   <211> 158
   <212> PRT
   <213> artificial
<220>
   <223> recombinant fusion protein rCTB4566
<400> 40
   Met Lys Lys Ile Trp Leu Ala Leu Ala Gly Leu Val Leu Ala Phe Ser
   1               5                   10                  15
   Ala Ser Ala Thr Pro Gln Asn Ile Thr Asp Leu Cys Ala Glu Tyr His
               20                  25                  30
   Asn Thr Gln Ile Tyr Thr Leu Asn Asp Lys Ile Phe Ser Tyr Thr Glu
           35                  40                  45
   Ser Leu Ala Gly Lys Arg Glu Met Ala Ile Ile Thr Phe Lys Asn Gly
       50                  55                  60
   Ala Ile Phe Gln Val Glu Val Pro Gly Ser Gln His Ile Asp Ser Gln
   65                  70                  75                  80
   Lys Lys Ala Ile Glu Arg Met Lys Asp Thr Leu Arg Ile Ala Tyr Leu
                   85                  90                  95
   Thr Glu Ala Lys Val Glu Lys Leu Cys Val Trp Asn Asn Lys Thr Pro
               100                 105                 110
   His Ala Ile Ala Ala Ile Ser Met Ala Asn Asp Gln Asn Ala Thr Ser
           115                 120                 125
   Ala Val Thr Glu Tyr Tyr Leu Asn His Gly Glu Trp Pro Gly Asn Asn
       130                 135                 140
   Thr Ser Ala Gly Val Gly Gly Gly His His His His His His
   145                 150                 155
<210> 41
   <211> 486
   <212> DNA
   <213> artificial
<220>
   <223> sequence encoding rCTB4565
<400> 41
   gaattcatga agaaaatttg gctggcctta gccggcctgg ttctggcatt cagcgccagc        60
   gcaaccccgc agaacatcac cgacctgtgc gccgagtacc acaacaccca aatttatacc       120
   ctgaacgaca aaatttttag ctacaccgag agcctggcag gcaagcgcga gatggccatc       180
   atcaccttca agaacggcgc cattttccag gtggaggtgc cgggcagcca gcacatcgac       240
   agtcagaaga aggccatcga gcgcatgaag gacaccctgc gcatcgccta cctgaccgag       300
   gccaaggtgg agaagctgtg cgtgtggaac aacaagaccc cgcacgccat cgccgcaatc       360
   agcatggcca acgaccagaa cgccaccagc gccgtgaccg agtactatct gaaccatggc       420
   gagtggccgg gtaataacac cagcgccggc ggcggcggcc accatcacca ccaccattaa       480
   aagctt                                                                  486
<210> 42
   <211> 157
   <212> PRT
   <213> artificial
<220>
   <223> recombinant fusion protein rCTB4565
<400> 42
   Met Lys Lys Ile Trp Leu Ala Leu Ala Gly Leu Val Leu Ala Phe Ser
   1               5                   10                  15
   Ala Ser Ala Thr Pro Gln Asn Ile Thr Asp Leu Cys Ala Glu Tyr His
               20                  25                  30
   Asn Thr Gln Ile Tyr Thr Leu Asn Asp Lys Ile Phe Ser Tyr Thr Glu
           35                  40                  45
   Ser Leu Ala Gly Lys Arg Glu Met Ala Ile Ile Thr Phe Lys Asn Gly
       50                  55                  60
   Ala Ile Phe Gln Val Glu Val Pro Gly Ser Gln His Ile Asp Ser Gln
   65                  70                  75                  80
   Lys Lys Ala Ile Glu Arg Met Lys Asp Thr Leu Arg Ile Ala Tyr Leu
                   85                  90                  95
   Thr Glu Ala Lys Val Glu Lys Leu Cys Val Trp Asn Asn Lys Thr Pro
               100                 105                 110
   His Ala Ile Ala Ala Ile Ser Met Ala Asn Asp Gln Asn Ala Thr Ser
           115                 120                 125
   Ala Val Thr Glu Tyr Tyr Leu Asn His Gly Glu Trp Pro Gly Asn Asn
       130                 135                 140
   Thr Ser Ala Gly Gly Gly Gly His His His His His His
   145                 150                 155
<210> 43
   <211> 483
   <212> DNA
   <213> artificial
<220>
   <223> sequence encoding rCTB4564
<400> 43
   gaattcatga agaaaatttg gctggcctta gccggcctgg ttctggcatt cagcgccagc        60
   gcaaccccgc agaacatcac cgacctgtgc gccgagtacc acaacaccca aatttatacc       120
   ctgaacgaca aaatttttag ctacaccgag agcctggcag gcaagcgcga gatggccatc       180
   atcaccttca agaacggcgc cattttccag gtggaggtgc cgggcagcca gcacatcgac       240
   agtcagaaga aggccatcga gcgcatgaag gacaccctgc gcatcgccta cctgaccgag       300
   gccaaggtgg agaagctgtg cgtgtggaac aacaagaccc cgcacgccat cgccgcaatc       360
   agcatggcca acgaccagaa cgccaccagc gccgtgaccg agtactatct gaaccatggc       420
   gagtggccgg gtaataacac cagcgccggc ggcggccacc atcaccacca ccattaaaag       480
   ctt                                                                     483
<210> 44
   <211> 156
   <212> PRT
   <213> artificial
<220>
   <223> recombinant fusion protein rCTB4564
<400> 44
   Met Lys Lys Ile Trp Leu Ala Leu Ala Gly Leu Val Leu Ala Phe Ser
   1               5                   10                  15
   Ala Ser Ala Thr Pro Gln Asn Ile Thr Asp Leu Cys Ala Glu Tyr His
               20                  25                  30
   Asn Thr Gln Ile Tyr Thr Leu Asn Asp Lys Ile Phe Ser Tyr Thr Glu
           35                  40                  45
   Ser Leu Ala Gly Lys Arg Glu Met Ala Ile Ile Thr Phe Lys Asn Gly
       50                  55                  60
   Ala Ile Phe Gln Val Glu Val Pro Gly Ser Gln His Ile Asp Ser Gln
   65                  70                  75                  80
   Lys Lys Ala Ile Glu Arg Met Lys Asp Thr Leu Arg Ile Ala Tyr Leu
                   85                  90                  95
   Thr Glu Ala Lys Val Glu Lys Leu Cys Val Trp Asn Asn Lys Thr Pro
               100                 105                 110
   His Ala Ile Ala Ala Ile Ser Met Ala Asn Asp Gln Asn Ala Thr Ser
           115                 120                 125
   Ala Val Thr Glu Tyr Tyr Leu Asn His Gly Glu Trp Pro Gly Asn Asn
       130                 135                 140
   Thr Ser Ala Gly Gly Gly His His His His His His
   145                 150                 155
<210> 45
   <211> 2037
   <212> DNA
   <213> artificial
<220>
   <223> sequence encoding rEPA4573
<400> 45
   gaattcatga aaaagatttg gctggcgctg gctggtttag ttttagcgtt tagcgcatcg 60
   gcggccgagg aagccttcga cctctggaac gaatgcgcca aagcctgcgt gctcgacctc 120
   aaggacggcg tgcgttccag ccgcatgagc gtcgacccgg ccatcgccga caccaacggc 180
   cagggcgtgc tgcactactc catggtcctg gagggcggca acgacgcgct caagctggcc 240
   atcgacaacg ccctcagcat caccagcgac ggcctgacca tccgcctcga aggcggcgtc 300
   gagccgaaca agccggtgcg ctacagctac acgcgccagg cgcgcggcag ttggtcgctg 360
   aactggctgg taccgatcgg ccacgagaag ccctcgaaca tcaaggtgtt catccacgaa 420
   ctgaacgccg gtaaccagct cagccacatg tcgccgatct acaccatcga gatgggcgac 480
   gagttgctgg cgaagctggc gcgcgatgcc accttcttcg tcagggcgca cgagagcaac 540
   gagatgcagc cgacgctcgc catcagccat gccggggtca gcgtggtcat ggcccaggcc 600
   cagccgcgcc gggaaaagcg ctggagcgaa tgggccagcg gcaaggtgtt gtgcctgctc 660
   gacccgctgg acggggtcta caactacctc gcccagcagc gctgcaacct cgacgatacc 720
   tgggaaggca agatctaccg ggtgctcgcc ggcaacccgg cgaagcatga cctggacatc 780
   aagcccacgg tcatcagtca tcgcctgcac ttccccgagg gcggcagcct ggccgcgctg 840
   accgcgcacc aggcttgcca cctgccgctg gagactttca cccgtcatcg ccagccgcgc 900
   ggctgggaac aactggagca gtgcggctat ccggtgcagc ggctggtcgc cctctacctg 960
   gcggcgcggc tgtcgtggaa ccaggtcgac caggtgatcg gcaacgccct ggccagcccc 1020
   ggcagcggcg gcgacctggg cgaagcgatc cgcgagcagc cggagcaggc ccgtctggcc 1080
   ctgaccctgg ccgccgccga gagcgagcgc ttcgtccggc agggcaccgg caacgacgag 1140
   gccggcgcgg ccagcgccga cgtggtgagc ctgacctgcc cggtcgccgc cggtgaatgc 1200
   gcgggcccgg cggacagcgg cgacgccctg ctggagcgca actatcccac tggcgcggag 1260
   ttcctcggcg acggcggcga catcagcttc agcacccgcg gcacgcagaa ctggacggtg 1320
   gagcggctgc tccaggcgca ccgccaactg gaggagcgcg gctatgtgtt cgtcggctac 1380
   cacggcacct tcctcgaagc ggcgcaaagc atcgtcttcg gcggggtgcg cgcgcgcagc 1440
   caggacctcg acgcgatctg gcgcggtttc tatatcgccg gcgatccggc gctggcctac 1500
   ggctacgccc aggaccagga acccgacgcg cgcggccgga tccgcaacgg tgccctgctg 1560
   cgggtctatg tgccgcgctc gagcctgccg ggcttctacc gcaccggcct gaccctggcc 1620
   gcgccggagg cggcgggcga ggtcgaacgg ctgatcggcc atccgctgcc gctgcgcctg 1680
   gacgccatca ccggccccga ggaggaaggc gggcgcgtga ccattctcgg ctggccgctg 1740
   gccgagcgca ccgtggtgat tccctcggcg atccccaccg acccgcgcaa cgtcggcggc 1800
   gacctcgacc cgtccagcat ccccgacaag gaacaggcga tcagcgccct gccggactac 1860
   gccagccagc ccggcaaacc gccgcgcgag gacctgaagg atcagaacgc gacctcagcc     1920
   gttaccgagt attacctgaa tcacggcgaa tggcccggca acaacacttc tgccggcgtg     1980
   gcaacctcct ctgaaatcaa aggaggtgga caccaccacc accaccacta aaagctt        2037
<210> 46
   <211> 674
   <212> PRT
   <213> artificial
<220>
   <223> recombinant fusion protein rEPA4573
<400> 46
   Met Lys Lys Ile Trp Leu Ala Leu Ala Gly Leu Val Leu Ala Phe Ser
   1               5                   10                  15
   Ala Ser Ala Ala Glu Glu Ala Phe Asp Leu Trp Asn Glu Cys Ala Lys
               20                  25                  30
   Ala Cys Val Leu Asp Leu Lys Asp Gly Val Arg Ser Ser Arg Met Ser
           35                  40                  45
   Val Asp Pro Ala Ile Ala Asp Thr Asn Gly Gln Gly Val Leu His Tyr
       50                  55                  60
   Ser Met Val Leu Glu Gly Gly Asn Asp Ala Leu Lys Leu Ala Ile Asp
   65                  70                  75                  80
   Asn Ala Leu Ser Ile Thr Ser Asp Gly Leu Thr Ile Arg Leu Glu Gly
                   85                  90                  95
   Gly Val Glu Pro Asn Lys Pro Val Arg Tyr Ser Tyr Thr Arg Gln Ala
               100                 105                 110
   Arg Gly Ser Trp Ser Leu Asn Trp Leu Val Pro Ile Gly His Glu Lys
           115                 120                 125
   Pro Ser Asn Ile Lys Val Phe Ile His Glu Leu Asn Ala Gly Asn Gln
       130                 135                 140
   Leu Ser His Met Ser Pro Ile Tyr Thr Ile Glu Met Gly Asp Glu Leu
   145                 150                 155                 160
   Leu Ala Lys Leu Ala Arg Asp Ala Thr Phe Phe Val Arg Ala His Glu
                   165                 170                 175
   Ser Asn Glu Met Gln Pro Thr Leu Ala Ile Ser His Ala Gly Val Ser
               180                 185                 190
   Val Val Met Ala Gln Ala Gln Pro Arg Arg Glu Lys Arg Trp Ser Glu
           195                 200                 205
   Trp Ala Ser Gly Lys Val Leu Cys Leu Leu Asp Pro Leu Asp Gly Val
       210                 215                 220
   Tyr Asn Tyr Leu Ala Gln Gln Arg Cys Asn Leu Asp Asp Thr Trp Glu
   225                 230                 235                 240
   Gly Lys Ile Tyr Arg Val Leu Ala Gly Asn Pro Ala Lys His Asp Leu
                   245                 250                 255
   Asp Ile Lys Pro Thr Val Ile Ser His Arg Leu His Phe Pro Glu Gly
               260                 265                 270
   Gly Ser Leu Ala Ala Leu Thr Ala His Gln Ala Cys His Leu Pro Leu
           275                 280                 285
   Glu Thr Phe Thr Arg His Arg Gln Pro Arg Gly Trp Glu Gln Leu Glu
       290                 295                 300
   Gln Cys Gly Tyr Pro Val Gln Arg Leu Val Ala Leu Tyr Leu Ala Ala
   305                 310                 315                 320
   Arg Leu Ser Trp Asn Gln Val Asp Gln Val Ile Gly Asn Ala Leu Ala
                   325                 330                 335
   Ser Pro Gly Ser Gly Gly Asp Leu Gly Glu Ala Ile Arg Glu Gln Pro
               340                 345                 350
   Glu Gln Ala Arg Leu Ala Leu Thr Leu Ala Ala Ala Glu Ser Glu Arg
           355                 360                 365
   Phe Val Arg Gln Gly Thr Gly Asn Asp Glu Ala Gly Ala Ala Ser Ala
       370                 375                 380
   Asp Val Val Ser Leu Thr Cys Pro Val Ala Ala Gly Glu Cys Ala Gly
   385                 390                 395                 400
   Pro Ala Asp Ser Gly Asp Ala Leu Leu Glu Arg Asn Tyr Pro Thr Gly
                   405                 410                 415
   Ala Glu Phe Leu Gly Asp Gly Gly Asp Ile Ser Phe Ser Thr Arg Gly
               420                 425                 430
   Thr Gln Asn Trp Thr Val Glu Arg Leu Leu Gln Ala His Arg Gln Leu
           435                 440                 445
   Glu Glu Arg Gly Tyr Val Phe Val Gly Tyr His Gly Thr Phe Leu Glu
       450                 455                 460
   Ala Ala Gln Ser Ile Val Phe Gly Gly Val Arg Ala Arg Ser Gln Asp
   465                 470                 475                 480
   Leu Asp Ala Ile Trp Arg Gly Phe Tyr Ile Ala Gly Asp Pro Ala Leu
                   485                 490                 495
   Ala Tyr Gly Tyr Ala Gln Asp Gln Glu Pro Asp Ala Arg Gly Arg Ile
               500                 505                 510
   Arg Asn Gly Ala Leu Leu Arg Val Tyr Val Pro Arg Ser Ser Leu Pro
           515                 520                 525
   Gly Phe Tyr Arg Thr Gly Leu Thr Leu Ala Ala Pro Glu Ala Ala Gly
       530                 535                 540
   Glu Val Glu Arg Leu Ile Gly His Pro Leu Pro Leu Arg Leu Asp Ala
   545                 550                 555                 560
   Ile Thr Gly Pro Glu Glu Glu Gly Gly Arg Val Thr Ile Leu Gly Trp
                   565                 570                 575
   Pro Leu Ala Glu Arg Thr Val Val Ile Pro Ser Ala Ile Pro Thr Asp
               580                 585                 590
   Pro Arg Asn Val Gly Gly Asp Leu Asp Pro Ser Ser Ile Pro Asp Lys
           595                 600                 605
   Glu Gln Ala Ile Ser Ala Leu Pro Asp Tyr Ala Ser Gln Pro Gly Lys
       610                 615                 620
   Pro Pro Arg Glu Asp Leu Lys Asp Gln Asn Ala Thr Ser Ala Val Thr
   625                 630                 635                 640
   Glu Tyr Tyr Leu Asn His Gly Glu Trp Pro Gly Asn Asn Thr Ser Ala
                   645                 650                 655
   Gly Val Ala Thr Ser Ser Glu Ile Lys Gly Gly Gly His His His His
               660                 665                 670
   His His
<210> 47
   <211> 2013
   <212> DNA
   <213> artificial
<220>
   <223> sequence encoding rEPA5573N
<400> 47
   gaattcatga aaaagatttg gctggcgctg gctggtttag ttttagcgtt tagcgcatcg      60
   gcggccgagg gcgaatggcc cggcaacaac acttctgccg gcgtggcaac ctcctctgaa     120
   atcaaaacta gtgaagcctt cgacctctgg aacgaatgcg ccaaagcctg cgtgctcgac     180
   ctcaaggacg gcgtgcgttc cagccgcatg agcgtcgacc cggccatcgc cgacaccaac     240
   ggccagggcg tgctgcacta ctccatggtc ctggagggcg gcaacgacgc gctcaagctg     300
   gccatcgaca acgccctcag catcaccagc gacggcctga ccatccgcct cgaaggcggc     360
   gtcgagccga acaagccggt gcgctacagc tacacgcgcc aggcgcgcgg cagttggtcg     420
   ctgaactggc tggtaccgat cggccacgag aagccctcga acatcaaggt gttcatccac     480
   gaactgaacg ccggtaacca gctcagccac atgtcgccga tctacaccat cgagatgggc     540
   gacgagttgc tggcgaagct ggcgcgcgat gccaccttct tcgtcagggc gcacgagagc     600
   aacgagatgc agccgacgct cgccatcagc catgccgggg tcagcgtggt catggcccag     660
   gcccagccgc gccgggaaaa gcgctggagc gaatgggcca gcggcaaggt gttgtgcctg     720
   ctcgacccgc tggacggggt ctacaactac ctcgcccagc agcgctgcaa cctcgacgat     780
   acctgggaag gcaagatcta ccgggtgctc gccggcaacc cggcgaagca tgacctggac     840
   atcaagccca cggtcatcag tcatcgcctg cacttccccg agggcggcag cctggccgcg     900
   ctgaccgcgc accaggcttg ccacctgccg ctggagactt tcacccgtca tcgccagccg     960
   cgcggctggg aacaactgga gcagtgcggc tatccggtgc agcggctggt cgccctctac    1020
   ctggcggcgc ggctgtcgtg gaaccaggtc gaccaggtga tcggcaacgc cctggccagc    1080
   cccggcagcg gcggcgacct gggcgaagcg atccgcgagc agccggagca ggcccgtctg    1140
   gccctgaccc tggccgccgc cgagagcgag cgcttcgtcc ggcagggcac cggcaacgac    1200
   gaggccggcg cggccagcgc cgacgtggtg agcctgacct gcccggtcgc cgccggtgaa    1260
   tgcgcgggcc cggcggacag cggcgacgcc ctgctggagc gcaactatcc cactggcgcg    1320
   gagttcctcg gcgacggcgg cgacatcagc ttcagcaccc gcggcacgca gaactggacg    1380
   gtggagcggc tgctccaggc gcaccgccaa ctggaggagc gcggctatgt gttcgtcggc    1440
   taccacggca ccttcctcga agcggcgcaa agcatcgtct tcggcggggt gcgcgcgcgc    1500
   agccaggacc tcgacgcgat ctggcgcggt ttctatatcg ccggcgatcc ggcgctggcc    1560
   tacggctacg cccaggacca ggaacccgac gcgcgcggcc ggatccgcaa cggtgccctg    1620
   ctgcgggtct atgtgccgcg ctcgagcctg ccgggcttct accgcaccgg cctgaccctg 1680
   gccgcgccgg aggcggcggg cgaggtcgaa cggctgatcg gccatccgct gccgctgcgc 1740
   ctggacgcca tcaccggccc cgaggaggaa ggcgggcgcg tgaccattct cggctggccg 1800
   ctggccgagc gcaccgtggt gattccctcg gcgatcccca ccgacccgcg caacgtcggc 1860
   ggcgacctcg acccgtccag catccccgac aaggaacagg cgatcagcgc cctgccggac 1920
   tacgccagcc agcccggcaa accgccgcgc gaggacctga aggatcagaa cgcgaccgga 1980
   ggtggacacc accaccacca ccactaaaag ctt 2013
<210> 48
   <211> 666
   <212> PRT
   <213> artificial
<220>
   <223> recombinant fusion protein rEPA5573N
<400> 48
   Met Lys Lys Ile Trp Leu Ala Leu Ala Gly Leu Val Leu Ala Phe Ser
   1               5                   10                  15
   Ala Ser Ala Ala Glu Gly Glu Trp Pro Gly Asn Asn Thr Ser Ala Gly
               20                  25                  30
   Val Ala Thr Ser Ser Glu Ile Lys Thr Ser Glu Ala Phe Asp Leu Trp
           35                  40                  45
   Asn Glu Cys Ala Lys Ala Cys Val Leu Asp Leu Lys Asp Gly Val Arg
       50                  55                  60
   Ser Ser Arg Met Ser Val Asp Pro Ala Ile Ala Asp Thr Asn Gly Gln
   65                  70                  75                  80
   Gly Val Leu His Tyr Ser Met Val Leu Glu Gly Gly Asn Asp Ala Leu
                   85                  90                  95
   Lys Leu Ala Ile Asp Asn Ala Leu Ser Ile Thr Ser Asp Gly Leu Thr
               100                 105                 110
   Ile Arg Leu Glu Gly Gly Val Glu Pro Asn Lys Pro Val Arg Tyr Ser
           115                 120                 125
   Tyr Thr Arg Gln Ala Arg Gly Ser Trp Ser Leu Asn Trp Leu Val Pro
       130                 135                 140
   Ile Gly His Glu Lys Pro Ser Asn Ile Lys Val Phe Ile His Glu Leu
   145                 150                 155                 160
   Asn Ala Gly Asn Gln Leu Ser His Met Ser Pro Ile Tyr Thr Ile Glu
   165 170 175
   Met Gly Asp Glu Leu Leu Ala Lys Leu Ala Arg Asp Ala Thr Phe Phe
   180 185 190
   Val Arg Ala His Glu Ser Asn Glu Met Gln Pro Thr Leu Ala Ile Ser
   195 200 205
   His Ala Gly Val Ser Val Val Met Ala Gln Ala Gln Pro Arg Arg Glu
   210 215 220
   Lys Arg Trp Ser Glu Trp Ala Ser Gly Lys Val Leu Cys Leu Leu Asp
   225 230 235 240
   Pro Leu Asp Gly Val Tyr Asn Tyr Leu Ala Gln Gln Arg Cys Asn Leu
   245 250 255
   Asp Asp Thr Trp Glu Gly Lys Ile Tyr Arg Val Leu Ala Gly Asn Pro
   260 265 270
   Ala Lys His Asp Leu Asp Ile Lys Pro Thr Val Ile Ser His Arg Leu
   275 280 285
   His Phe Pro Glu Gly Gly Ser Leu Ala Ala Leu Thr Ala His Gln Ala
   290 295 300
   Cys His Leu Pro Leu Glu Thr Phe Thr Arg His Arg Gln Pro Arg Gly
   305 310 315 320
   Trp Glu Gln Leu Glu Gln Cys Gly Tyr Pro Val Gln Arg Leu Val Ala
   325 330 335
   Leu Tyr Leu Ala Ala Arg Leu Ser Trp Asn Gln Val Asp Gln Val Ile
   340 345 350
   Gly Asn Ala Leu Ala Ser Pro Gly Ser Gly Gly Asp Leu Gly Glu Ala
   355 360 365
   Ile Arg Glu Gln Pro Glu Gln Ala Arg Leu Ala Leu Thr Leu Ala Ala
   370 375 380
   Ala Glu Ser Glu Arg Phe Val Arg Gln Gly Thr Gly Asn Asp Glu Ala
   385 390 395 400
   Gly Ala Ala Ser Ala Asp Val Val Ser Leu Thr Cys Pro Val Ala Ala
   405 410 415
   Gly Glu Cys Ala Gly Pro Ala Asp Ser Gly Asp Ala Leu Leu Glu Arg
   420 425 430
   Asn Tyr Pro Thr Gly Ala Glu Phe Leu Gly Asp Gly Gly Asp Ile Ser
   435 440 445
   Phe Ser Thr Arg Gly Thr Gln Asn Trp Thr Val Glu Arg Leu Leu Gln
   450 455 460
   Ala His Arg Gln Leu Glu Glu Arg Gly Tyr Val Phe Val Gly Tyr His
   465 470 475 480
   Gly Thr Phe Leu Glu Ala Ala Gln Ser Ile Val Phe Gly Gly Val Arg
   485 490 495
   Ala Arg Ser Gln Asp Leu Asp Ala Ile Trp Arg Gly Phe Tyr Ile Ala
   500 505 510
   Gly Asp Pro Ala Leu Ala Tyr Gly Tyr Ala Gln Asp Gln Glu Pro Asp
   515 520 525
   Ala Arg Gly Arg Ile Arg Asn Gly Ala Leu Leu Arg Val Tyr Val Pro
   530 535 540
   Arg Ser Ser Leu Pro Gly Phe Tyr Arg Thr Gly Leu Thr Leu Ala Ala
   545 550 555 560
   Pro Glu Ala Ala Gly Glu Val Glu Arg Leu Ile Gly His Pro Leu Pro
   565 570 575
   Leu Arg Leu Asp Ala Ile Thr Gly Pro Glu Glu Glu Gly Gly Arg Val
   580 585 590
   Thr Ile Leu Gly Trp Pro Leu Ala Glu Arg Thr Val Val Ile Pro Ser
   595 600 605
   Ala Ile Pro Thr Asp Pro Arg Asn Val Gly Gly Asp Leu Asp Pro Ser
   610 615 620
   Ser Ile Pro Asp Lys Glu Gln Ala Ile Ser Ala Leu Pro Asp Tyr Ala
   625 630 635 640
   Ser Gln Pro Gly Lys Pro Pro Arg Glu Asp Leu Lys Asp Gln Asn Ala
   645 650 655
   Thr Gly Gly Gly His His His His His His
   660 665
<210> 49
   <211> 2001
   <212> DNA
   <213> artificial
<220>
   <223> sequence encoding rEPA5569N
<400> 49
   gaattcatga aaaagatttg gctggcgctg gctggtttag ttttagcgtt tagcgcatcg 60
   gcggccgagg gcgaatggcc cggcaacaac acttctgccg gcgtggcaac ctccactagt 120
   gaagccttcg acctctggaa cgaatgcgcc aaagcctgcg tgctcgacct caaggacggc 180
   gtgcgttcca gccgcatgag cgtcgacccg gccatcgccg acaccaacgg ccagggcgtg 240
   ctgcactact ccatggtcct ggagggcggc aacgacgcgc tcaagctggc catcgacaac 300
   gccctcagca tcaccagcga cggcctgacc atccgcctcg aaggcggcgt cgagccgaac 360
   aagccggtgc gctacagcta cacgcgccag gcgcgcggca gttggtcgct gaactggctg 420
   gtaccgatcg gccacgagaa gccctcgaac atcaaggtgt tcatccacga actgaacgcc 480
   ggtaaccagc tcagccacat gtcgccgatc tacaccatcg agatgggcga cgagttgctg 540
   gcgaagctgg cgcgcgatgc caccttcttc gtcagggcgc acgagagcaa cgagatgcag 600
   ccgacgctcg ccatcagcca tgccggggtc agcgtggtca tggcccaggc ccagccgcgc 660
   cgggaaaagc gctggagcga atgggccagc ggcaaggtgt tgtgcctgct cgacccgctg 720
   gacggggtct acaactacct cgcccagcag cgctgcaacc tcgacgatac ctgggaaggc 780
   aagatctacc gggtgctcgc cggcaacccg gcgaagcatg acctggacat caagcccacg 840
   gtcatcagtc atcgcctgca cttccccgag ggcggcagcc tggccgcgct gaccgcgcac 900
   caggcttgcc acctgccgct ggagactttc acccgtcatc gccagccgcg cggctgggaa 960
   caactggagc agtgcggcta tccggtgcag cggctggtcg ccctctacct ggcggcgcgg 1020
   ctgtcgtgga accaggtcga ccaggtgatc ggcaacgccc tggccagccc cggcagcggc 1080
   ggcgacctgg gcgaagcgat ccgcgagcag ccggagcagg cccgtctggc cctgaccctg 1140
   gccgccgccg agagcgagcg cttcgtccgg cagggcaccg gcaacgacga ggccggcgcg 1200
   gccagcgccg acgtggtgag cctgacctgc ccggtcgccg ccggtgaatg cgcgggcccg 1260
   gcggacagcg gcgacgccct gctggagcgc aactatccca ctggcgcgga gttcctcggc 1320
   gacggcggcg acatcagctt cagcacccgc ggcacgcaga actggacggt ggagcggctg 1380
   ctccaggcgc accgccaact ggaggagcgc ggctatgtgt tcgtcggcta ccacggcacc 1440
   ttcctcgaag cggcgcaaag catcgtcttc ggcggggtgc gcgcgcgcag ccaggacctc 1500
   gacgcgatct ggcgcggttt ctatatcgcc ggcgatccgg cgctggccta cggctacgcc 1560
   caggaccagg aacccgacgc gcgcggccgg atccgcaacg gtgccctgct gcgggtctat 1620
   gtgccgcgct cgagcctgcc gggcttctac cgcaccggcc tgaccctggc cgcgccggag 1680
   gcggcgggcg aggtcgaacg gctgatcggc catccgctgc cgctgcgcct ggacgccatc 1740
   accggccccg aggaggaagg cgggcgcgtg accattctcg gctggccgct ggccgagcgc 1800
   accgtggtga ttccctcggc gatccccacc gacccgcgca acgtcggcgg cgacctcgac 1860
   ccgtccagca tccccgacaa ggaacaggcg atcagcgccc tgccggacta cgccagccag 1920
   cccggcaaac cgccgcgcga ggacctgaag gatcagaacg cgaccggagg tggacaccac 1980
   caccaccacc actaaaagct t 2001
<210> 50
   <211> 662
   <212> PRT
   <213> artificial
<220>
   <223> recombinant fusion protein rEPA5569N
<400> 50
   Met Lys Lys Ile Trp Leu Ala Leu Ala Gly Leu Val Leu Ala Phe Ser
   1               5                   10                  15
   Ala Ser Ala Ala Glu Gly Glu Trp Pro Gly Asn Asn Thr Ser Ala Gly
               20                  25                  30
   Val Ala Thr Ser Thr Ser Glu Ala Phe Asp Leu Trp Asn Glu Cys Ala
           35                  40                  45
   Lys Ala Cys Val Leu Asp Leu Lys Asp Gly Val Arg Ser Ser Arg Met
       50                  55                  60
   Ser Val Asp Pro Ala Ile Ala Asp Thr Asn Gly Gln Gly Val Leu His
   65                  70                  75                  80
   Tyr Ser Met Val Leu Glu Gly Gly Asn Asp Ala Leu Lys Leu Ala Ile
                   85                  90                  95
   Asp Asn Ala Leu Ser Ile Thr Ser Asp Gly Leu Thr Ile Arg Leu Glu
               100                 105                 110
   Gly Gly Val Glu Pro Asn Lys Pro Val Arg Tyr Ser Tyr Thr Arg Gln
           115                 120                 125
   Ala Arg Gly Ser Trp Ser Leu Asn Trp Leu Val Pro Ile Gly His Glu
       130                 135                 140
   Lys Pro Ser Asn Ile Lys Val Phe Ile His Glu Leu Asn Ala Gly Asn
   145                 150                 155                 160
   Gln Leu Ser His Met Ser Pro Ile Tyr Thr Ile Glu Met Gly Asp Glu
                   165                 170                 175
   Leu Leu Ala Lys Leu Ala Arg Asp Ala Thr Phe Phe Val Arg Ala His
               180                 185                 190
   Glu Ser Asn Glu Met Gln Pro Thr Leu Ala Ile Ser His Ala Gly Val
           195                 200                 205
   Ser Val Val Met Ala Gln Ala Gln Pro Arg Arg Glu Lys Arg Trp Ser
       210                 215                 220
   Glu Trp Ala Ser Gly Lys Val Leu Cys Leu Leu Asp Pro Leu Asp Gly
   225                 230                 235                 240
   Val Tyr Asn Tyr Leu Ala Gln Gln Arg Cys Asn Leu Asp Asp Thr Trp
                   245                 250                 255
   Glu Gly Lys Ile Tyr Arg Val Leu Ala Gly Asn Pro Ala Lys His Asp
               260                 265                 270
   Leu Asp Ile Lys Pro Thr Val Ile Ser His Arg Leu His Phe Pro Glu
           275                 280                 285
   Gly Gly Ser Leu Ala Ala Leu Thr Ala His Gln Ala Cys His Leu Pro
       290                 295                 300
   Leu Glu Thr Phe Thr Arg His Arg Gln Pro Arg Gly Trp Glu Gln Leu
   305                 310                 315                 320
   Glu Gln Cys Gly Tyr Pro Val Gln Arg Leu Val Ala Leu Tyr Leu Ala
                   325                 330                 335
   Ala Arg Leu Ser Trp Asn Gln Val Asp Gln Val Ile Gly Asn Ala Leu
               340                 345                 350
   Ala Ser Pro Gly Ser Gly Gly Asp Leu Gly Glu Ala Ile Arg Glu Gln
           355                 360                 365
   Pro Glu Gln Ala Arg Leu Ala Leu Thr Leu Ala Ala Ala Glu Ser Glu
       370                 375                 380
   Arg Phe Val Arg Gln Gly Thr Gly Asn Asp Glu Ala Gly Ala Ala Ser
   385                 390                 395                 400
   Ala Asp Val Val Ser Leu Thr Cys Pro Val Ala Ala Gly Glu Cys Ala
                   405                 410                 415
   Gly Pro Ala Asp Ser Gly Asp Ala Leu Leu Glu Arg Asn Tyr Pro Thr
               420                 425                 430
   Gly Ala Glu Phe Leu Gly Asp Gly Gly Asp Ile Ser Phe Ser Thr Arg
           435                 440                 445
   Gly Thr Gln Asn Trp Thr Val Glu Arg Leu Leu Gln Ala His Arg Gln
       450                 455                 460
   Leu Glu Glu Arg Gly Tyr Val Phe Val Gly Tyr His Gly Thr Phe Leu
   465                 470                 475                 480
   Glu Ala Ala Gln Ser Ile Val Phe Gly Gly Val Arg Ala Arg Ser Gln
                   485                 490                 495
   Asp Leu Asp Ala Ile Trp Arg Gly Phe Tyr Ile Ala Gly Asp Pro Ala
               500                 505                 510
   Leu Ala Tyr Gly Tyr Ala Gln Asp Gln Glu Pro Asp Ala Arg Gly Arg
           515                 520                 525
   Ile Arg Asn Gly Ala Leu Leu Arg Val Tyr Val Pro Arg Ser Ser Leu
       530                 535                 540
   Pro Gly Phe Tyr Arg Thr Gly Leu Thr Leu Ala Ala Pro Glu Ala Ala
   545                 550                 555                 560
   Gly Glu Val Glu Arg Leu Ile Gly His Pro Leu Pro Leu Arg Leu Asp
                   565                 570                 575
   Ala Ile Thr Gly Pro Glu Glu Glu Gly Gly Arg Val Thr Ile Leu Gly
               580                 585                 590
   Trp Pro Leu Ala Glu Arg Thr Val Val Ile Pro Ser Ala Ile Pro Thr
           595                 600                 605
   Asp Pro Arg Asn Val Gly Gly Asp Leu Asp Pro Ser Ser Ile Pro Asp
       610                 615                 620
   Lys Glu Gln Ala Ile Ser Ala Leu Pro Asp Tyr Ala Ser Gln Pro Gly
   625                 630                 635                 640
   Lys Pro Pro Arg Glu Asp Leu Lys Asp Gln Asn Ala Thr Gly Gly Gly
                   645                     650                 655
   His His His His His His
               660
<210> 51
   <211> 1992
   <212> DNA
   <213> artificial
<220>
   <223> sequence encoding rEPA5566N
<400> 51
   gaattcatga aaaagatttg gctggcgctg gctggtttag ttttagcgtt tagcgcatcg      60
   gcggccgagg gcgaatggcc cggcaacaac acttctgccg gcgtgactag tgaagccttc     120
   gacctctgga acgaatgcgc caaagcctgc gtgctcgacc tcaaggacgg cgtgcgttcc     180
   agccgcatga gcgtcgaccc ggccatcgcc gacaccaacg gccagggcgt gctgcactac     240
   tccatggtcc tggagggcgg caacgacgcg ctcaagctgg ccatcgacaa cgccctcagc     300
   atcaccagcg acggcctgac catccgcctc gaaggcggcg tcgagccgaa caagccggtg     360
   cgctacagct acacgcgcca ggcgcgcggc agttggtcgc tgaactggct ggtaccgatc     420
   ggccacgaga agccctcgaa catcaaggtg ttcatccacg aactgaacgc cggtaaccag     480
   ctcagccaca tgtcgccgat ctacaccatc gagatgggcg acgagttgct ggcgaagctg     540
   gcgcgcgatg ccaccttctt cgtcagggcg cacgagagca acgagatgca gccgacgctc     600
   gccatcagcc atgccggggt cagcgtggtc atggcccagg cccagccgcg ccgggaaaag     660
   cgctggagcg aatgggccag cggcaaggtg ttgtgcctgc tcgacccgct ggacggggtc     720
   tacaactacc tcgcccagca gcgctgcaac ctcgacgata cctgggaagg caagatctac     780
   cgggtgctcg ccggcaaccc ggcgaagcat gacctggaca tcaagcccac ggtcatcagt     840
   catcgcctgc acttccccga gggcggcagc ctggccgcgc tgaccgcgca ccaggcttgc     900
   cacctgccgc tggagacttt cacccgtcat cgccagccgc gcggctggga acaactggag     960
   cagtgcggct atccggtgca gcggctggtc gccctctacc tggcggcgcg gctgtcgtgg    1020
   aaccaggtcg accaggtgat cggcaacgcc ctggccagcc ccggcagcgg cggcgacctg    1080
   ggcgaagcga tccgcgagca gccggagcag gcccgtctgg ccctgaccct ggccgccgcc    1140
   gagagcgagc gcttcgtccg gcagggcacc ggcaacgacg aggccggcgc ggccagcgcc    1200
   gacgtggtga gcctgacctg cccggtcgcc gccggtgaat gcgcgggccc ggcggacagc    1260
   ggcgacgccc tgctggagcg caactatccc actggcgcgg agttcctcgg cgacggcggc    1320
   gacatcagct tcagcacccg cggcacgcag aactggacgg tggagcggct gctccaggcg    1380
   caccgccaac tggaggagcg cggctatgtg ttcgtcggct accacggcac cttcctcgaa 1440
   gcggcgcaaa gcatcgtctt cggcggggtg cgcgcgcgca gccaggacct cgacgcgatc 1500
   tggcgcggtt tctatatcgc cggcgatccg gcgctggcct acggctacgc ccaggaccag 1560
   gaacccgacg cgcgcggccg gatccgcaac ggtgccctgc tgcgggtcta tgtgccgcgc 1620
   tcgagcctgc cgggcttcta ccgcaccggc ctgaccctgg ccgcgccgga ggcggcgggc 1680
   gaggtcgaac ggctgatcgg ccatccgctg ccgctgcgcc tggacgccat caccggcccc 1740
   gaggaggaag gcgggcgcgt gaccattctc ggctggccgc tggccgagcg caccgtggtg 1800
   attccctcgg cgatccccac cgacccgcgc aacgtcggcg gcgacctcga cccgtccagc 1860
   atccccgaca aggaacaggc gatcagcgcc ctgccggact acgccagcca gcccggcaaa 1920
   ccgccgcgcg aggacctgaa ggatcagaac gcgaccggag gtggacacca ccaccaccac 1980
   cactaaaagc tt 1992
<210> 52
   <211> 659
   <212> PRT
   <213> artificial
<220>
   <223> recombinant fusion protein rEPA5566N
<400> 52
   Met Lys Lys Ile Trp Leu Ala Leu Ala Gly Leu Val Leu Ala Phe Ser
   1               5                   10                  15
   Ala Ser Ala Ala Glu Gly Glu Trp Pro Gly Asn Asn Thr Ser Ala Gly
               20                  25                  30
   Val Thr Ser Glu Ala Phe Asp Leu Trp Asn Glu Cys Ala Lys Ala Cys
           35                  40                  45
   Val Leu Asp Leu Lys Asp Gly Val Arg Ser Ser Arg Met Ser Val Asp
       50                  55                  60
   Pro Ala Ile Ala Asp Thr Asn Gly Gln Gly Val Leu His Tyr Ser Met
   65                  70                  75                  80
   Val Leu Glu Gly Gly Asn Asp Ala Leu Lys Leu Ala Ile Asp Asn Ala
                   85                  90                  95
   Leu Ser Ile Thr Ser Asp Gly Leu Thr Ile Arg Leu Glu Gly Gly Val
               100                 105                 110
   Glu Pro Asn Lys Pro Val Arg Tyr Ser Tyr Thr Arg Gln Ala Arg Gly
           115                 120                 125
   Ser Trp Ser Leu Asn Trp Leu Val Pro Ile Gly His Glu Lys Pro Ser
       130                 135                 140
   Asn Ile Lys Val Phe Ile His Glu Leu Asn Ala Gly Asn Gln Leu Ser
   145                 150                 155                 160
   His Met Ser Pro Ile Tyr Thr Ile Glu Met Gly Asp Glu Leu Leu Ala
                   165                 170                 175
   Lys Leu Ala Arg Asp Ala Thr Phe Phe Val Arg Ala His Glu Ser Asn
               180                 185                 190
   Glu Met Gln Pro Thr Leu Ala Ile Ser His Ala Gly Val Ser Val Val
           195                 200                 205
   Met Ala Gln Ala Gln Pro Arg Arg Glu Lys Arg Trp Ser Glu Trp Ala
       210                 215                 220
   Ser Gly Lys Val Leu Cys Leu Leu Asp Pro Leu Asp Gly Val Tyr Asn
   225                 230                 235                 240
   Tyr Leu Ala Gln Gln Arg Cys Asn Leu Asp Asp Thr Trp Glu Gly Lys
                   245                 250                 255
   Ile Tyr Arg Val Leu Ala Gly Asn Pro Ala Lys His Asp Leu Asp Ile
               260                 265                 270
   Lys Pro Thr Val Ile Ser His Arg Leu His Phe Pro Glu Gly Gly Ser
           275                 280                 285
   Leu Ala Ala Leu Thr Ala His Gln Ala Cys His Leu Pro Leu Glu Thr
       290                 295                 300
   Phe Thr Arg His Arg Gln Pro Arg Gly Trp Glu Gln Leu Glu Gln Cys
   305                 310                 315                 320
   Gly Tyr Pro Val Gln Arg Leu Val Ala Leu Tyr Leu Ala Ala Arg Leu
                   325                 330                 335
   Ser Trp Asn Gln Val Asp Gln Val Ile Gly Asn Ala Leu Ala Ser Pro
               340                 345                 350
   Gly Ser Gly Gly Asp Leu Gly Glu Ala Ile Arg Glu Gln Pro Glu Gln
           355                 360                 365
   Ala Arg Leu Ala Leu Thr Leu Ala Ala Ala Glu Ser Glu Arg Phe Val
   370 375 380
   Arg Gln Gly Thr Gly Asn Asp Glu Ala Gly Ala Ala Ser Ala Asp Val
   385 390 395 400
   Val Ser Leu Thr Cys Pro Val Ala Ala Gly Glu Cys Ala Gly Pro Ala
   405 410 415
   Asp Ser Gly Asp Ala Leu Leu Glu Arg Asn Tyr Pro Thr Gly Ala Glu
   420 425 430
   Phe Leu Gly Asp Gly Gly Asp Ile Ser Phe Ser Thr Arg Gly Thr Gln
   435 440 445
   Asn Trp Thr Val Glu Arg Leu Leu Gln Ala His Arg Gln Leu Glu Glu
   450 455 460
   Arg Gly Tyr Val Phe Val Gly Tyr His Gly Thr Phe Leu Glu Ala Ala
   465 470 475 480
   Gln Ser Ile Val Phe Gly Gly Val Arg Ala Arg Ser Gln Asp Leu Asp
   485 490 495
   Ala Ile Trp Arg Gly Phe Tyr Ile Ala Gly Asp Pro Ala Leu Ala Tyr
   500 505 510
   Gly Tyr Ala Gln Asp Gln Glu Pro Asp Ala Arg Gly Arg Ile Arg Asn
   515 520 525
   Gly Ala Leu Leu Arg Val Tyr Val Pro Arg Ser Ser Leu Pro Gly Phe
   530 535 540
   Tyr Arg Thr Gly Leu Thr Leu Ala Ala Pro Glu Ala Ala Gly Glu Val
   545 550 555 560
   Glu Arg Leu Ile Gly His Pro Leu Pro Leu Arg Leu Asp Ala Ile Thr
   565 570 575
   Gly Pro Glu Glu Glu Gly Gly Arg Val Thr Ile Leu Gly Trp Pro Leu
   580 585 590
   Ala Glu Arg Thr Val Val Ile Pro Ser Ala Ile Pro Thr Asp Pro Arg
   595 600 605
   Asn Val Gly Gly Asp Leu Asp Pro Ser Ser Ile Pro Asp Lys Glu Gln
   610 615 620
   Ala Ile Ser Ala Leu Pro Asp Tyr Ala Ser Gln Pro Gly Lys Pro Pro
   625                 630                 635                 640
   Arg Glu Asp Leu Lys Asp Gln Asn Ala Thr Gly Gly Gly His His His
                   645                 650                 655
   His His His
<210> 53
   <211> 1983
   <212> DNA
   <213> artificial
<220>
   <223> sequence encoding rEPA5866N
<400> 53
   gaattcatga aaaagatttg gctggcgctg gctggtttag ttttagcgtt tagcgcatcg      60
   gcggccgagc ccggcaacaa cacttctgcc ggcgtgacta gtgaagcctt cgacctctgg     120
   aacgaatgcg ccaaagcctg cgtgctcgac ctcaaggacg gcgtgcgttc cagccgcatg     180
   agcgtcgacc cggccatcgc cgacaccaac ggccagggcg tgctgcacta ctccatggtc     240
   ctggagggcg gcaacgacgc gctcaagctg gccatcgaca acgccctcag catcaccagc     300
   gacggcctga ccatccgcct cgaaggcggc gtcgagccga acaagccggt gcgctacagc     360
   tacacgcgcc aggcgcgcgg cagttggtcg ctgaactggc tggtaccgat cggccacgag     420
   aagccctcga acatcaaggt gttcatccac gaactgaacg ccggtaacca gctcagccac     480
   atgtcgccga tctacaccat cgagatgggc gacgagttgc tggcgaagct ggcgcgcgat     540
   gccaccttct tcgtcagggc gcacgagagc aacgagatgc agccgacgct cgccatcagc     600
   catgccgggg tcagcgtggt catggcccag gcccagccgc gccgggaaaa gcgctggagc     660
   gaatgggcca gcggcaaggt gttgtgcctg ctcgacccgc tggacggggt ctacaactac     720
   ctcgcccagc agcgctgcaa cctcgacgat acctgggaag gcaagatcta ccgggtgctc     780
   gccggcaacc cggcgaagca tgacctggac atcaagccca cggtcatcag tcatcgcctg     840
   cacttccccg agggcggcag cctggccgcg ctgaccgcgc accaggcttg ccacctgccg     900
   ctggagactt tcacccgtca tcgccagccg cgcggctggg aacaactgga gcagtgcggc     960
   tatccggtgc agcggctggt cgccctctac ctggcggcgc ggctgtcgtg gaaccaggtc    1020
   gaccaggtga tcggcaacgc cctggccagc cccggcagcg gcggcgacct gggcgaagcg    1080
   atccgcgagc agccggagca ggcccgtctg gccctgaccc tggccgccgc cgagagcgag    1140
   cgcttcgtcc ggcagggcac cggcaacgac gaggccggcg cggccagcgc cgacgtggtg    1200
   agcctgacct gcccggtcgc cgccggtgaa tgcgcgggcc cggcggacag cggcgacgcc    1260
   ctgctggagc gcaactatcc cactggcgcg gagttcctcg gcgacggcgg cgacatcagc      1320
   ttcagcaccc gcggcacgca gaactggacg gtggagcggc tgctccaggc gcaccgccaa      1380
   ctggaggagc gcggctatgt gttcgtcggc taccacggca ccttcctcga agcggcgcaa      1440
   agcatcgtct tcggcggggt gcgcgcgcgc agccaggacc tcgacgcgat ctggcgcggt      1500
   ttctatatcg ccggcgatcc ggcgctggcc tacggctacg cccaggacca ggaacccgac      1560
   gcgcgcggcc ggatccgcaa cggtgccctg ctgcgggtct atgtgccgcg ctcgagcctg      1620
   ccgggcttct accgcaccgg cctgaccctg gccgcgccgg aggcggcggg cgaggtcgaa      1680
   cggctgatcg gccatccgct gccgctgcgc ctggacgcca tcaccggccc cgaggaggaa      1740
   ggcgggcgcg tgaccattct cggctggccg ctggccgagc gcaccgtggt gattccctcg      1800
   gcgatcccca ccgacccgcg caacgtcggc ggcgacctcg acccgtccag catccccgac      1860
   aaggaacagg cgatcagcgc cctgccggac tacgccagcc agcccggcaa accgccgcgc      1920
   gaggacctga aggatcagaa cgcgaccgga ggtggacacc accaccacca ccactaaaag      1980
   ctt                                                                    1983
<210> 54
   <211> 656
   <212> PRT
   <213> artificial
<220>
   <223> recombinant fusion protein rEPA5866N
<400> 54
   Met Lys Lys Ile Trp Leu Ala Leu Ala Gly Leu Val Leu Ala Phe Ser
   1               5                   10                  15
   Ala Ser Ala Ala Glu Pro Gly Asn Asn Thr Ser Ala Gly Val Thr Ser
               20                  25                  30
   Glu Ala Phe Asp Leu Trp Asn Glu Cys Ala Lys Ala Cys Val Leu Asp
           35                  40                  45
   Leu Lys Asp Gly Val Arg Ser Ser Arg Met Ser Val Asp Pro Ala Ile
       50                  55                  60
   Ala Asp Thr Asn Gly Gln Gly Val Leu His Tyr Ser Met Val Leu Glu
   65                  70                  75                  80
   Gly Gly Asn Asp Ala Leu Lys Leu Ala Ile Asp Asn Ala Leu Ser Ile
                   85                  90                  95
   Thr Ser Asp Gly Leu Thr Ile Arg Leu Glu Gly Gly Val Glu Pro Asn
               100                 105                 110
   Lys Pro Val Arg Tyr Ser Tyr Thr Arg Gln Ala Arg Gly Ser Trp Ser
           115                 120                 125
   Leu Asn Trp Leu Val Pro Ile Gly His Glu Lys Pro Ser Asn Ile Lys
       130                 135                 140
   Val Phe Ile His Glu Leu Asn Ala Gly Asn Gln Leu Ser His Met Ser
   145                 150                 155                 160
   Pro Ile Tyr Thr Ile Glu Met Gly Asp Glu Leu Leu Ala Lys Leu Ala
                   165                 170                 175
   Arg Asp Ala Thr Phe Phe Val Arg Ala His Glu Ser Asn Glu Met Gln
               180                 185                 190
   Pro Thr Leu Ala Ile Ser His Ala Gly Val Ser Val Val Met Ala Gln
           195                 200                 205
   Ala Gln Pro Arg Arg Glu Lys Arg Trp Ser Glu Trp Ala Ser Gly Lys
       210                 215                 220
   Val Leu Cys Leu Leu Asp Pro Leu Asp Gly Val Tyr Asn Tyr Leu Ala
   225                 230                 235                 240
   Gln Gln Arg Cys Asn Leu Asp Asp Thr Trp Glu Gly Lys Ile Tyr Arg
                   245                 250                 255
   Val Leu Ala Gly Asn Pro Ala Lys His Asp Leu Asp Ile Lys Pro Thr
               260                 265                 270
   Val Ile Ser His Arg Leu His Phe Pro Glu Gly Gly Ser Leu Ala Ala
           275                 280                 285
   Leu Thr Ala His Gln Ala Cys His Leu Pro Leu Glu Thr Phe Thr Arg
       290                 295                 300
   His Arg Gln Pro Arg Gly Trp Glu Gln Leu Glu Gln Cys Gly Tyr Pro
   305                 310                 315                 320
   Val Gln Arg Leu Val Ala Leu Tyr Leu Ala Ala Arg Leu Ser Trp Asn
                   325                 330                 335
   Gln Val Asp Gln Val Ile Gly Asn Ala Leu Ala Ser Pro Gly Ser Gly
               340                 345                 350
   Gly Asp Leu Gly Glu Ala Ile Arg Glu Gln Pro Glu Gln Ala Arg Leu
   355 360 365
   Ala Leu Thr Leu Ala Ala Ala Glu Ser Glu Arg Phe Val Arg Gln Gly
   370 375 380
   Thr Gly Asn Asp Glu Ala Gly Ala Ala Ser Ala Asp Val Val Ser Leu
   385 390 395 400
   Thr Cys Pro Val Ala Ala Gly Glu Cys Ala Gly Pro Ala Asp Ser Gly
   405 410 415
   Asp Ala Leu Leu Glu Arg Asn Tyr Pro Thr Gly Ala Glu Phe Leu Gly
   420 425 430
   Asp Gly Gly Asp Ile Ser Phe Ser Thr Arg Gly Thr Gln Asn Trp Thr
   435 440 445
   Val Glu Arg Leu Leu Gln Ala His Arg Gln Leu Glu Glu Arg Gly Tyr
   450 455 460
   Val Phe Val Gly Tyr His Gly Thr Phe Leu Glu Ala Ala Gln Ser Ile
   465 470 475 480
   Val Phe Gly Gly Val Arg Ala Arg Ser Gln Asp Leu Asp Ala Ile Trp
   485 490 495
   Arg Gly Phe Tyr Ile Ala Gly Asp Pro Ala Leu Ala Tyr Gly Tyr Ala
   500 505 510
   Gln Asp Gln Glu Pro Asp Ala Arg Gly Arg Ile Arg Asn Gly Ala Leu
   515 520 525
   Leu Arg Val Tyr Val Pro Arg Ser Ser Leu Pro Gly Phe Tyr Arg Thr
   530 535 540
   Gly Leu Thr Leu Ala Ala Pro Glu Ala Ala Gly Glu Val Glu Arg Leu
   545 550 555 560
   Ile Gly His Pro Leu Pro Leu Arg Leu Asp Ala Ile Thr Gly Pro Glu
   565 570 575
   Glu Glu Gly Gly Arg Val Thr Ile Leu Gly Trp Pro Leu Ala Glu Arg
   580 585 590
   Thr Val Val Ile Pro Ser Ala Ile Pro Thr Asp Pro Arg Asn Val Gly
   595 600 605
   Gly Asp Leu Asp Pro Ser Ser Ile Pro Asp Lys Glu Gln Ala Ile Ser
       610                 615                 620
   Ala Leu Pro Asp Tyr Ala Ser Gln Pro Gly Lys Pro Pro Arg Glu Asp
   625                 630                 635                 640
   Leu Lys Asp Gln Asn Ala Thr Gly Gly Gly His His His His His His
                   645                 650                 655
<210> 55
   <211> 612
   <212> DNA
   <213> artificial
<220>
   <223> sequence encoding rCTB45732
<400> 55
   gaattcatga agaaaatttg gctggcctta gccggcctgg ttctggcatt cagcgccagc      60
   gcaaccccgc agaacatcac cgacctgtgc gccgagtacc acaacaccca aatttatacc     120
   ctgaacgaca aaatttttag ctacaccgag agcctggcag gcaagcgcga gatggccatc     180
   atcaccttca agaacggcgc cattttccag gtggaggtgc cgggcagcca gcacatcgac     240
   agtcagaaga aggccatcga gcgcatgaag gacaccctgc gcatcgccta cctgaccgag     300
   gccaaggtgg agaagctgtg cgtgtggaac aacaagaccc cgcacgccat cgccgcaatc     360
   agcatggcca acgaccagaa cgccaccagc gccgtgaccg agtactatct gaaccatggc     420
   gagtggccgg gtaataacac cagcgccggc gtggccacaa gcagtgagat caagggcggc     480
   ggatctagcg ccgtgaccga gtactatctg aaccatggcg agtggccggg taataacacc     540
   agcgccggcg tggccacaag cagtgagatc aagggcggcg gatcccacca tcaccaccac     600
   cattaaaagc tt                                                         612
<210> 56
   <211> 199
   <212> PRT
   <213> artificial
<220>
   <223> recombinant fusion protein rCTB45732
<400> 56
   Met Lys Lys Ile Trp Leu Ala Leu Ala Gly Leu Val Leu Ala Phe Ser
   1               5                   10                  15
   Ala Ser Ala Thr Pro Gln Asn Ile Thr Asp Leu Cys Ala Glu Tyr His
               20                  25                  30
   Asn Thr Gln Ile Tyr Thr Leu Asn Asp Lys Ile Phe Ser Tyr Thr Glu
           35                  40                  45
   Ser Leu Ala Gly Lys Arg Glu Met Ala Ile Ile Thr Phe Lys Asn Gly
       50                  55                  60
   Ala Ile Phe Gln Val Glu Val Pro Gly Ser Gln His Ile Asp Ser Gln
   65                  70                  75                  80
   Lys Lys Ala Ile Glu Arg Met Lys Asp Thr Leu Arg Ile Ala Tyr Leu
                   85                  90                  95
   Thr Glu Ala Lys Val Glu Lys Leu Cys Val Trp Asn Asn Lys Thr Pro
               100                 105                 110
   His Ala Ile Ala Ala Ile Ser Met Ala Asn Asp Gln Asn Ala Thr Ser
           115                 120                 125
   Ala Val Thr Glu Tyr Tyr Leu Asn His Gly Glu Trp Pro Gly Asn Asn
       130                 135                 140
   Thr Ser Ala Gly Val Ala Thr Ser Ser Glu Ile Lys Gly Gly Gly Ser
   145                 150                 155                 160
   Ser Ala Val Thr Glu Tyr Tyr Leu Asn His Gly Glu Trp Pro Gly Asn
                   165                 170                 175
   Asn Thr Ser Ala Gly Val Ala Thr Ser Ser Glu Ile Lys Gly Gly Gly
               180                 185                 190
   Ser His His His His His His
           195
<210> 57
   <211> 2223
   <212> DNA
   <213> artificial
<220>
   <223> sequence encoding rEPA4573NMC
<400> 57
   gaattcatga aaaagatttg gctggcgctg gctggtttag ttttagcgtt tagcgcatcg        60
   gcggccgagt cagccgttac cgagtattac ctgaatcacg gcgaatggcc cggcaacaac       120
   acttctgccg gcgtggcaac ctcctctgaa atcaaaacta gtgaagcctt cgacctctgg       180
   aacgaatgcg ccaaagcctg cgtgctcgac ctcaaggacg gcgtgcgttc cagccgcatg       240
   agcgtcgacc cggccatcgc cgacaccaac ggccagggcg tgctgcacta ctccatggtc       300
   ctggagggcg gcaacgacgc gctcaagctg gccatcgaca acgccctcag catcaccagc       360
   gacggcctga ccatccgcct cgaaggcggc gtcgagccga acaagccggt gcgctacagc 420
   tacacgcgcc aggcgcgcgg cagttggtcg ctgaactggc tggtaccgat cggccacgag 480
   aagccctcga acatcaaggt gttcatccac gaactgaacg ccggtaacca gctcagccac 540
   atgtcgccga tctacaccat cgagatgggc gacgagttgc tggcgaagct ggcgcgcgat 600
   gccaccttct tcgtcagggc gcacgagagc aacgagatgc agccgacgct cgccatcagc 660
   catgccgggg tcagcgtggt catggcccag gcccagccgc gccgggaaaa gcgctggagc 720
   gaatgggcca gcggcaaggt gttgtgcctg ctcgacccgc tggacggggt ctacaactac 780
   ctcgcccagc agcgctgcaa cctcgacgat acctgggaag gcaagatcta ccgggtgctc 840
   gccggcaacc cggcgaagca tgacctggac atcaagtcag ccgttaccga gtattacctg 900
   aatcacggcg aatggcccgg caacaacact tctgccggcg tggcaacctc ctctgaaatc 960
   aaacatatgc ccacggtcat cagtcatcgc ctgcacttcc ccgagggcgg cagcctggcc 1020
   gcgctgaccg cgcaccaggc ttgccacctg ccgctggaga ctttcacccg tcatcgccag 1080
   ccgcgcggct gggaacaact ggagcagtgc ggctatccgg tgcagcggct ggtcgccctc 1140
   tacctggcgg cgcggctgtc gtggaaccag gtcgaccagg tgatcggcaa cgccctggcc 1200
   agccccggca gcggcggcga cctgggcgaa gcgatccgcg agcagccgga gcaggcccgt 1260
   ctggccctga ccctggccgc cgccgagagc gagcgcttcg tccggcaggg caccggcaac 1320
   gacgaggccg gcgcggccag cgccgacgtg gtgagcctga cctgcccggt cgccgccggt 1380
   gaatgcgcgg gcccggcgga cagcggcgac gccctgctgg agcgcaacta tcccactggc 1440
   gcggagttcc tcggcgacgg cggcgacatc agcttcagca cccgcggcac gcagaactgg 1500
   acggtggagc ggctgctcca ggcgcaccgc caactggagg agcgcggcta tgtgttcgtc 1560
   ggctaccacg gcaccttcct cgaagcggcg caaagcatcg tcttcggcgg ggtgcgcgcg 1620
   cgcagccagg acctcgacgc gatctggcgc ggtttctata tcgccggcga tccggcgctg 1680
   gcctacggct acgcccagga ccaggaaccc gacgcgcgcg gccggatccg caacggtgcc 1740
   ctgctgcggg tctatgtgcc gcgctcgagc ctgccgggct tctaccgcac cggcctgacc 1800
   ctggccgcgc cggaggcggc gggcgaggtc gaacggctga tcggccatcc gctgccgctg 1860
   cgcctggacg ccatcaccgg ccccgaggag gaaggcgggc gcgtgaccat tctcggctgg 1920
   ccgctggccg agcgcaccgt ggtgattccc tcggcgatcc ccaccgaccc gcgcaacgtc 1980
   ggcggcgacc tcgacccgtc cagcatcccc gacaaggaac aggcgatcag cgccctgccg 2040
   gactacgcca gccagcccgg caaaccgccg cgcgaggacc tgaaggatca gaacgcgacc 2100
   tcagccgtta ccgagtatta cctgaatcac ggcgaatggc ccggcaacaa cacttctgcc 2160
   ggcgtggcaa cctcctctga aatcaaagga ggtggacacc accaccacca ccactaaaag 2220
   ctt 2223
<210> 58
   <211> 736
   <212> PRT
   <213> artificial
<220>
   <223> recombinant fusion protein rEPA4573NMC
<400> 58
   Met Lys Lys Ile Trp Leu Ala Leu Ala Gly Leu Val Leu Ala Phe Ser
   1               5                   10                  15
   Ala Ser Ala Ala Glu Ser Ala Val Thr Glu Tyr Tyr Leu Asn His Gly
               20                  25                  30
   Glu Trp Pro Gly Asn Asn Thr Ser Ala Gly Val Ala Thr Ser Ser Glu
           35                  40                  45
   Ile Lys Thr Ser Glu Ala Phe Asp Leu Trp Asn Glu Cys Ala Lys Ala
       50                  55                  60
   Cys Val Leu Asp Leu Lys Asp Gly Val Arg Ser Ser Arg Met Ser Val
   65                  70                  75                  80
   Asp Pro Ala Ile Ala Asp Thr Asn Gly Gln Gly Val Leu His Tyr Ser
                   85                  90                  95
   Met Val Leu Glu Gly Gly Asn Asp Ala Leu Lys Leu Ala Ile Asp Asn
               100                 105                 110
   Ala Leu Ser Ile Thr Ser Asp Gly Leu Thr Ile Arg Leu Glu Gly Gly
           115                 120                 125
   Val Glu Pro Asn Lys Pro Val Arg Tyr Ser Tyr Thr Arg Gln Ala Arg
       130                 135                 140
   Gly Ser Trp Ser Leu Asn Trp Leu Val Pro Ile Gly His Glu Lys Pro
   145                 150                 155                 160
   Ser Asn Ile Lys Val Phe Ile His Glu Leu Asn Ala Gly Asn Gln Leu
                   165                 170                 175
   Ser His Met Ser Pro Ile Tyr Thr Ile Glu Met Gly Asp Glu Leu Leu
               180                 185                 190
   Ala Lys Leu Ala Arg Asp Ala Thr Phe Phe Val Arg Ala His Glu Ser
           195                 200                 205
   Asn Glu Met Gln Pro Thr Leu Ala Ile Ser His Ala Gly Val Ser Val
       210                 215                 220
   Val Met Ala Gln Ala Gln Pro Arg Arg Glu Lys Arg Trp Ser Glu Trp
   225                 230                 235                 240
   Ala Ser Gly Lys Val Leu Cys Leu Leu Asp Pro Leu Asp Gly Val Tyr
                   245                 250                 255
   Asn Tyr Leu Ala Gln Gln Arg Cys Asn Leu Asp Asp Thr Trp Glu Gly
               260                 265                 270
   Lys Ile Tyr Arg Val Leu Ala Gly Asn Pro Ala Lys His Asp Leu Asp
           275                 280                 285
   Ile Lys Ser Ala Val Thr Glu Tyr Tyr Leu Asn His Gly Glu Trp Pro
       290                 295                 300
   Gly Asn Asn Thr Ser Ala Gly Val Ala Thr Ser Ser Glu Ile Lys His
   305                 310                 315                 320
   Met Pro Thr Val Ile Ser His Arg Leu His Phe Pro Glu Gly Gly Ser
                   325                 330                 335
   Leu Ala Ala Leu Thr Ala His Gln Ala Cys His Leu Pro Leu Glu Thr
               340                 345                 350
   Phe Thr Arg His Arg Gln Pro Arg Gly Trp Glu Gln Leu Glu Gln Cys
           355                 360                 365
   Gly Tyr Pro Val Gln Arg Leu Val Ala Leu Tyr Leu Ala Ala Arg Leu
       370                 375                 380
   Ser Trp Asn Gln Val Asp Gln Val Ile Gly Asn Ala Leu Ala Ser Pro
   385                 390                 395                 400
   Gly Ser Gly Gly Asp Leu Gly Glu Ala Ile Arg Glu Gln Pro Glu Gln
                   405                 410                 415
   Ala Arg Leu Ala Leu Thr Leu Ala Ala Ala Glu Ser Glu Arg Phe Val
               420                 425                 430
   Arg Gln Gly Thr Gly Asn Asp Glu Ala Gly Ala Ala Ser Ala Asp Val
           435                 440                 445
   Val Ser Leu Thr Cys Pro Val Ala Ala Gly Glu Cys Ala Gly Pro Ala
   450 455 460
   Asp Ser Gly Asp Ala Leu Leu Glu Arg Asn Tyr Pro Thr Gly Ala Glu
   465 470 475 480
   Phe Leu Gly Asp Gly Gly Asp Ile Ser Phe Ser Thr Arg Gly Thr Gln
   485 490 495
   Asn Trp Thr Val Glu Arg Leu Leu Gln Ala His Arg Gln Leu Glu Glu
   500 505 510
   Arg Gly Tyr Val Phe Val Gly Tyr His Gly Thr Phe Leu Glu Ala Ala
   515 520 525
   Gln Ser Ile Val Phe Gly Gly Val Arg Ala Arg Ser Gln Asp Leu Asp
   530 535 540
   Ala Ile Trp Arg Gly Phe Tyr Ile Ala Gly Asp Pro Ala Leu Ala Tyr
   545 550 555 560
   Gly Tyr Ala Gln Asp Gln Glu Pro Asp Ala Arg Gly Arg Ile Arg Asn
   565 570 575
   Gly Ala Leu Leu Arg Val Tyr Val Pro Arg Ser Ser Leu Pro Gly Phe
   580 585 590
   Tyr Arg Thr Gly Leu Thr Leu Ala Ala Pro Glu Ala Ala Gly Glu Val
   595 600 605
   Glu Arg Leu Ile Gly His Pro Leu Pro Leu Arg Leu Asp Ala Ile Thr
   610 615 620
   Gly Pro Glu Glu Glu Gly Gly Arg Val Thr Ile Leu Gly Trp Pro Leu
   625 630 635 640
   Ala Glu Arg Thr Val Val Ile Pro Ser Ala Ile Pro Thr Asp Pro Arg
   645 650 655
   Asn Val Gly Gly Asp Leu Asp Pro Ser Ser Ile Pro Asp Lys Glu Gln
   660 665 670
   Ala Ile Ser Ala Leu Pro Asp Tyr Ala Ser Gln Pro Gly Lys Pro Pro
   675 680 685
   Arg Glu Asp Leu Lys Asp Gln Asn Ala Thr Ser Ala Val Thr Glu Tyr
   690 695 700
   Tyr Leu Asn His Gly Glu Trp Pro Gly Asn Asn Thr Ser Ala Gly Val
   705 710 715 720
   Ala Thr Ser Ser Glu Ile Lys Gly Gly Gly His His His His His His
   725 730 735
<210> 59
   <211> 1509
   <212> DNA
   <213> artificial
<220>
   <223> sequence encoding rTTC4573
<400> 59
   gaattcatga aaaagatttg gctggcgctg gctggtttag ttttagcgtt tagcgcatcg      60
   gcggcgcagt atgaatccac catcctgaac ctggacatca acaatgacat catctccgat     120
   atctctggct tcaactcctc tgtgatcacc tatccagatg ctcagctggt gccaggtatc     180
   aacggcaaag ccatccacct ggtgaacaat gaatcctctg aagtgatcgt tcacaaggcc     240
   atggacatcg aatacaacga catgttcaat aacttcaccg tgtccttctg gctgcgtgtt     300
   ccgaaagtgt ccgcttctca cctggaacag tacgatacca acgaatattc catcatctct     360
   tccatgaaga aatactctct gtccatcggt tctggctggt ccgtgtctct gaaaggtaac     420
   aatctgatct ggaccctgaa ggactccgcc ggtgaagtgc gtcagatcac cttccgtgac     480
   ctgtctgata aattcaacgc ctacctggct aacaagtggg tgttcatcac catcactaat     540
   gaccgtctgt cctctgctaa cctgtacatc aatggcgttc tgatgggttc cgccgaaatc     600
   actggtctgg gcgctatccg tgaagacaac aatatcaccc tgaagctgga ccgttgcaac     660
   aataacaatc agtacgtgtc catcgacaag ttccgtatct tctgcaaagc cctgaacccg     720
   aaggaaatcg aaaagctgta cacttcttat ctgtccatca ccttcctgcg tgacttctgg     780
   ggtaacccac tgcgttacga tactgaatat tacctgatcc cagtggctta ttcctctaaa     840
   gacgttcagc tgaagaacat caccgattac atgtatctga ctaacgctcc atcctacact     900
   aacggcaaac tgaatatcta ctatcgtcgt ctgtactccg gtctgaagtt catcatcaaa     960
   cgttatactc cgaacaatga aatcgactcc ttcgtgcgtt ctggtgattt catcaaactg    1020
   tacgtttcct ataacaataa cgaacacatc gttggctacc caaaggacgg taatgccttc    1080
   aacaatctgg atcgtatcct gcgtgttggc tacaacgctc caggtatccc gctgtataag    1140
   aaaatggaag ccgtgaaact gcgtgacctg aagacctact ccgtgcagct gaaactgtat    1200
   gacgataaag acgcctctct gggtctggtt ggcactcaca acggtcagat cggcaatgac    1260
   ccaaaccgtg atatcctgat cgcctccaac tggtacttca atcacctgaa agacaagact    1320
   ctgacctgcg attggtactt cgtgccaact gatgaaggtt ggaccaacga cgatcagaac    1380
   gcgacctcag ccgttaccga gtattacctg aatcacggcg aatggcccgg caacaacact    1440
   tctgccggcg tggcaacctc ctctgaaatc aaaggaggtg gacaccacca ccaccaccac    1500
   taaaagctt                                                            1509
<210> 60
   <211> 498
   <212> PRT
   <213> artificial
<220>
   <223> recombinant fusion protein rTTC4573
<400> 60 His His
<210> 61
   <211> 26
   <212> DNA
   <213> artificial
<220>
   <223> primer sequence
<400> 61
   cgggatccag gctttgacta tgtgga 26
<210> 62
   <211> 27
   <212> DNA
   <213> artificial
<220>
   <223> primer sequence
<400> 62
   gcgtcgacat ctggcgatat gagtatg 27
<210> 63
   <211> 26
   <212> DNA
   <213> artificial
<220>
   <223> primer sequence
<400> 63
   ccaagcttta gtgcaggcat attggg 26
<210> 64
   <211> 26
   <212> DNA
   <213> artificial
<220>
   <223> primer sequence
<400> 64
   ccctcgagta tcacctcgca gaacct 26
<210> 65
   <211> 19
   <212> DNA
   <213> artificial
<220>
   <223> primer sequence
<400> 65
   aacaccggat tacggataa 19
<210> 66
   <211> 18
   <212> DNA
   <213> artificial
<220>
   <223> primer sequence
<400> 66
   tgcatggtgg ctgtagaa 18
<210> 67
   <211> 18
   <212> DNA
   <213> artificial
<220>
   <223> primer sequence
<400> 67
   agaaacggtt gcgaaaat 18
<210> 68
   <211> 18
   <212> DNA
   <213> artificial
<220>
   <223> primer sequence
<400> 68
   atagccgtag cccttgat 18
<210> 69
   <211> 28
   <212> DNA
   <213> artificial
<220>
   <223> primer sequence
<400> 69
   gcgtcgacgt gtaggctgga gctgcttc 28
<210> 70
   <211> 28
   <212> DNA
   <213> artificial
<220>
   <223> primer sequence
<400> 70
   ccaagcttat gggaattagc catggtcc 28
<210> 71
   <211> 2549
   <212> DNA
   <213> artificial
<220>
   <223> linear targeting DNA fragment
<400> 71
   ggatccaggc tttgactatg tggatgtgtt accgcgcatg agcctggagg aggtcgccag      60
   agtgctggct ggcgcaaaat ttgtcgtatc ggttgatacc ggcctgagcc atctttccgc     120
   ggcgctcgac agaccgaata ttacgctata tggcccaacg gaccctgggt taattggagg     180
   ttatgggaag aaccaaatgg catgctgctc accagaacaa aacctggcga atttagatgc     240
   cacaagcgta tttggaaaga ttcattaaag agactctgtc tcatcccaaa cctattgtgg     300
   agaaaagatg ctaaccacat cattaacgtt aaataaagag aaatggaagc cgatctggaa     360
   taaagcgctg gtttttcttt ttgttgccac gtattttctg gatggtatta cgcgttataa     420
   acatttgata atcatactta tggttatcac cgcgatttat caggtctcac gctcaccgaa     480
   aagtttcccc cctcttttca aaaatagcgt attttatagc gtagcagtat tatcattaat     540
   ccttgtttat tccatactca tatcgccaga tgtcgacgtg taggctggag ctgcttcgaa     600
   gttcctatac tttctagaga ataggaactt cggaatagga acttcaagat cccctcacgc     660
   tgccgcaagc actcagggcg caagggctgc taaaggaagc ggaacacgta gaaagccagt     720
   ccgcagaaac ggtgctgacc ccggatgaat gtcagctact gggctatctg gacaagggaa     780
   aacgcaagcg caaagagaaa gcaggtagct tgcagtgggc ttacatggcg atagctagac     840
   tgggcggttt tatggacagc aagcgaaccg gaattgccag ctggggcgcc ctctggtaag     900
   gttgggaagc cctgcaaagt aaactggatg gctttcttgc cgccaaggat ctgatggcgc     960
   aggggatcaa gatctgatca agagacagga tgaggatcgt ttcgcatgat tgaacaagat    1020
   ggattgcacg caggttctcc ggccgcttgg gtggagaggc tattcggcta tgactgggca    1080
   caacagacaa tcggctgctc tgatgccgcc gtgttccggc tgtcagcgca ggggcgcccg    1140
   gttctttttg tcaagaccga cctgtccggt gccctgaatg aactgcagga cgaggcagcg    1200
   cggctatcgt ggctggccac gacgggcgtt ccttgcgcag ctgtgctcga cgttgtcact    1260
   gaagcgggaa gggactggct gctattgggc gaagtgccgg ggcaggatct cctgtcatct 1320
   caccttgctc ctgccgagaa agtatccatc atggctgatg caatgcggcg gctgcatacg 1380
   cttgatccgg ctacctgccc attcgaccac caagcgaaac atcgcatcga gcgagcacgt 1440
   actcggatgg aagccggtct tgtcgatcag gatgatctgg acgaagagca tcaggggctc 1500
   gcgccagccg aactgttcgc caggctcaag gcgcgcatgc ccgacggcga ggatctcgtc 1560
   gtgacccatg gcgatgcctg cttgccgaat atcatggtgg aaaatggccg cttttctgga 1620
   ttcatcgact gtggccggct gggtgtggcg gaccgctatc aggacatagc gttggctacc 1680
   cgtgatattg ctgaagagct tggcggcgaa tgggctgacc gcttcctcgt gctttacggt 1740
   atcgccgctc ccgattcgca gcgcatcgcc ttctatcgcc ttcttgacga gttcttctga 1800
   gcgggactct ggggttcgaa atgaccgacc aagcgacgcc caacctgcca tcacgagatt 1860
   tcgattccac cgccgccttc tatgaaaggt tgggcttcgg aatcgttttc cgggacgccg 1920
   gctggatgat cctccagcgc ggggatctca tgctggagtt cttcgcccac cccagcttca 1980
   aaagcgctct gaagttccta tactttctag agaataggaa cttcggaata ggaactaagg 2040
   aggatattca tatggaccat ggctaattcc cataagcttt agtgcaggca tattgggtct 2100
   ggcgagcctg gcctatttat atggtgctat catcagggaa acagccagct ctaccttcag 2160
   gaaagtagag ataagcccct acaatgctca tctcttgcta tttttatctt tcgtcggttt 2220
   ttatatcgtt cgtggcaatt ttgaacaggt cgatattgct caaattggta tcattaccgg 2280
   ttttctgctg gcgctaagaa atagataaaa aacgcgctga tacttattac ggtatcagcg 2340
   cgttttccat catcaggact caatcactta tcaaaccagt ttttcatttg ttcctcgaaa 2400
   cgctgcgcta cattttccca actgtatttt gaaaacacca gggattttgc tttttcggca 2460
   atctggtggc gttccttatc agcaagcgca cggttaatat cattaattat actgtcgctc 2520
   gacataggtt ctgcgaggtg atactcgag 2549
<210> 72
   <211> 61
   <212> DNA
   <213> artificial
<220>
   <223> primer sequence
<400> 72
   gcagttttgg aaaagttatc atcattataa aggtaaaaca tagcgattgt gtaggctgga 60
   g 61
<210> 73
   <211> 66
   <212> DNA
   <213> artificial
<220>
   <223> primer sequence
<400> 73
   agtgagtttt aactcacttc ttaaacttgt ttattcttaa taattaacgg ctgacatggg 60
   aattag 66
<210> 74
   <211> 19
   <212> DNA
   <213> artificial
<220>
   <223> primer sequence
<400> 74
   tacaaatagt atccccaac 19
<210> 75
   <211> 19
   <212> DNA
   <213> artificial
<220>
   <223> primer sequence
<400> 75
   aattaacctc aacagtcaa 19
<210> 76
   <211> 1133
   <212> DNA
   <213> artificial
<220>
   <223> linear targeting DNA fragment
<400> 76
   gcagttttgg aaaagttatc atcattataa aggtaaaaca tagcgattgt gtaggctgga 60
   gctgcttcga agttcctata ctttctagag aataggaact tcggaatagg aacttcattt 120
   aaatggcgcg ccttacgccc cgccctgcca ctcatcgcag tactgttgta ttcattaagc 180
   atctgccgac atggaagcca tcacaaacgg catgatgaac ctgaatcgcc agcggcatca 240
   gcaccttgtc gccttgcgta taatatttgc ccatggtgaa aacgggggcg aagaagttgt 300
   ccatattggc cacgtttaaa tcaaaactgg tgaaactcac ccagggattg gctgagacga 360
   aaaacatatt ctcaataaac cctttaggga aataggccag gttttcaccg taacacgcca 420
   catcttgcga atatatgtgt agaaactgcc ggaaatcgtc gtggtattca ctccagagcg 480
   atgaaaacgt ttcagtttgc tcatggaaaa cggtgtaaca agggtgaaca ctatcccata 540
   tcaccagctc accgtctttc attgccatac gtaattccgg atgagcattc atcaggcggg 600
   caagaatgtg aataaaggcc ggataaaact tgtgcttatt tttctttacg gtctttaaaa 660
   aggccgtaat atccagctga acggtctggt tataggtaca ttgagcaact gactgaaatg 720
   cctcaaaatg ttctttacga tgccattggg atatatcaac ggtggtatat ccagtgattt 780
   ttttctccat tttagcttcc ttagctcctg aaaatctcga caactcaaaa aatacgcccg 840
   gtagtgatct tatttcatta tggtgaaagt tggaacctct tacgtgccga tcaacgtctc 900
   attttcgcca aaagttggcc cagggcttcc cggtatcaac agggacacca ggatttattt 960
   attctgcgaa gtgatcttcc gtcacaggta ggcgcgccga agttcctata ctttctagag 1020
   aataggaact tcggaatagg aactaaggag gatattcata tggaccatgg ctaattccca 1080
   tgtcagccgt taattaagaa taaacaagtt taagaagtga gttaaaactc act 1133
<210> 77
   <211> 52
   <212> DNA
   <213> artificial
<220>
   <223> primer sequence
<400> 77
   agaaggcgcg ccaagaatga cgaatttaaa agcagttata ccggtagcag gt 52
<210> 78
   <211> 48
   <212> DNA
   <213> artificial
<220>
   <223> primer sequence
<400> 78
   atatgcggcc gcttaatcca gccattcggt atggaacaca ccttcttt 48
<210> 79
   <211> 16582
   <212> DNA
   <213> artificial
<220>
   <223> gene cluster for 0157 polysaccharide synthesis
<400> 79
   ggcgcgccaa gaatgacgaa tttaaaagca gttataccgg tagcaggtct tgggatgcat 60
   atgttgcctg ccactaaggc gattcccaaa gagatgctac cgatcgtcga caagccaatg 120
   attcagtaca ttgttgacga gattgtggct gcagggatca aagaaatcct cctggtaact 180
   cacgcgtcca agaacgcagt cgaaaaccac ttcgacacct cttatgaatt agaatctctc 240
   cttgaacagc gcgtgaagcg tcaactactg gcggaagtac aatctatctg tccgccgggc 300
   gtgaccatta tgaacgtgcg tcagggtgaa cctttaggtt taggccactc cattttatgt 360
   gcgcgacctg ccattggtga caatccattt gtcgtggtgc tgccagacgt tgtgatcgac 420
   gacgccagcg ccgacccgct acgctacaac cttgctgcca tgattgcgcg cttcaatgaa 480
   acgggacgca gccaggtgct ggcaaaacgt atgccgggcg acctctctga atactccgtc 540
   atccagacca aagaaccgct ggatcgtgaa ggcaaagtca gccgcattgt tgaatttatc 600
   gaaaagccgg atcagccgca gacgctggac tcagacatca tggccgttgg tcgatatgtg 660
   ctttctgccg atatttggcc ggaactggaa cgtactcagc ctggtgcatg gggacgtatt 720
   cagctgactg atgccattgc tgagctggca aaaaaacaat ccgttgatgc aatgctgatg 780
   actggcgaca gttacgactg cggtaaaaaa atgggctata tgcaggcgtt tgtgaagtat 840
   ggactacgca acttgaaaga aggggcgaag ttccgcaaag gtatcgagaa gctgttaagc 900
   gaataatgaa aatctgaccg aatgtaacgg ttgataagga aataatatcg gcagtgaaaa 960
   actatagatc aataaatatc actgctgttg agcttttata attaactatg ctaccaatga 1020
   ataaatagtg tgcagatatg tattttttgt ttggtagtct tgtttctccc tctgaataat 1080
   aagataataa acgcttggag atgatttttt taacatggat tatcatttcg tacactggta 1140
   gcgattaagc caggggcggt agcgtgcata aattttaatg cttatcaaaa ctattagcat 1200
   taaaaatata taagaaattc tcaaatgaac aaagaaaccg tttcaataat tatgcccgtt 1260
   tacaatgggg ccaaaactat aatctcatca gtagaatcaa ttatacatca atcttatcaa 1320
   gattttgttt tgtatatcat tgacgattgt agcaccgatg atacattttc attaatcaac 1380
   agtcgataca aaaacaatca gaaaataaga atattgcgta acaagacaaa tttaggtgtt 1440
   gcagaaagtc gaaattatgg aatagaaatg gccacgggga aatatatttc tttttgtgat 1500
   gcggatgatt tgtggcacga gaaaaaatta gagcgtcaaa tcgaagtgtt aaataatgaa 1560
   tgtgtagatg tggtatgttc taattattat gttatagata acaaaagaaa tattgttggc 1620
   gaagttaatg ctcctcatgt gataaattat agaaaaatgc tcatgaaaaa ctacataggg 1680
   aatttgacag gaatctataa tgccaacaaa ttgggtaagt tttatcaaaa aaagattggt 1740
   cacgaggatt atttgatgtg gctggaaata attaataaaa caaatggtgc tatttgtatt 1800
   caagataatc tggcgtatta catgcgttca aataattcac tatcgggtaa taaaattaaa 1860
   gctgcaaaat ggacatggag tatatataga gaacatttac atttgtcctt tccaaaaaca 1920
   ttatattatt ttttattata tgcttcaaat ggagtcatga aaaaaataac acattcacta 1980
   ttaaggagaa aggagactaa aaagtgaagt cagcggctaa gttgattttt ttattcctat 2040
   ttacacttta tagtctccag ttgtatgggg ttatcataga tgatcgtata acaaattttg 2100
   atacaaaggt attaactagt attataatta tatttcagat tttttttgtt ttattatttt 2160
   atctaacgat tataaatgaa agaaaacagc agaaaaaatt tatcgtgaac tgggagctaa 2220
   agttaatact cgttttcctt tttgtgacta tagaaattgc tgctgtagtt ttatttctta 2280
   aagaaggtat tcctatattt gatgatgatc cagggggggc taaacttaga atagctgaag 2340
   gtaatggact ttacattaga tatattaagt attttggtaa tatagttgtg tttgcattaa 2400
   ttattcttta tgatgagcat aaattcaaac agaggaccat catatttgta tattttacaa 2460
   cgattgcttt atttggttat cgttctgaat tggtgttgct cattcttcaa tatatattga 2520
   ttaccaatat cctgtcaaag gataaccgta atcctaaaat aaaaagaata atagggtatt 2580
   ttttattggt aggggttgta tgctcgttgt tttatctaag tttaggacaa gacggagaac 2640
   aaaatgactc atataataat atgttaagga taattaatag gttaacaata gagcaagttg 2700
   aaagtgttcc atatgttgtt tctgaatcta ttaagaacga tttctttccg acaccagagt 2760
   tagaaaagga attaaaagca ataataaata gaatacaggg aataaagcat caagacttat 2820
   tttatggaga acggttacat aaacaagtat ttggagacat gggagcaaat tttttatcag 2880
   ttactacgta tggagcagaa ctgttagttt tttttggttt tctctgtgta ttcattatcc 2940
   ctttagggat atatatacct ttttatcttt taaagagaat gaaaaaaacc catagctcga 3000
   taaattgcgc attctattca tatatcatta tgattttatt gcaatactta gtggctggga 3060
   atgcatcggc cttctttttt ggtccttttc tctccgtatt gataatgtgt actcctctga 3120
   tcttattgca tgatacgtta aagagattat cacgaaatga aaatatcagt tataactgtg 3180
   acttataata atgctgaagg gttagaaaaa actttaagta gtttatcaat tttaaaaata 3240
   aaaccttttg agattattat agttgatggc ggctctacag atggaacgaa tcgtgtcatt 3300
   agtagattta ctagtatgaa tattacacat gtttatgaaa aagatgaagg gatatatgat 3360
   gcgatgaata agggccgaat gttggccaaa ggcgacttaa tacattattt aaacgccggc 3420
   gatagcgtaa ttggagatat atataaaaat atcaaagagc catgtttgat taaagttggc 3480
   cttttcgaaa atgataaact tctgggattt tcttctataa cccattcaaa tacagggtat 3540
   tgtcatcaag ggtgattttt cccaaagaat cattcagaat atgatctaag gtataaaata 3600
   tgtgctgatt ataagcttat tcaagaggtg tttcctgaag ggttaagatc tctatctttg 3660
   attacttcgg gttatgtaaa atatgatatg gggggagtat cttcaaaaaa aagaatttta 3720
   agagataaag agcttgccaa aattatgttt gaaaaaaata aaaaaaacct tattaagttt 3780
   attccaattt caataatcaa aattttattc cctgaacgtt taagaagagt attgcggaaa 3840
   atgcaatata tttgtctaac tttattcttc atgaagaata gttcaccata tgataatgaa 3900
   taaaatcaaa aaaatactta aattttgcac tttaaaaaaa tatgatacat caagtgcttt 3960
   aggtagagaa caggaaaggt acaggattat atccttgtct gttatttcaa gtttgattag 4020
   taaaatactc tcactacttt ctcttatatt aactgtaagt ttaactttac cttatttagg 4080
   acaagagaga tttggtgtat ggatgactat taccagtctt ggtgctgctc tgacattttt 4140
   ggacttaggt ataggaaatg cattaacaaa caggatcgca cattcatttg cgtgtggcaa 4200
   aaatttaaag atgagtcggc aaattagtgg tgggctcact ttgctggctg gattatcgtt 4260
   tgtcataact gcaatatgct atattacttc tggcatgatt gattggcaac tagtaataaa 4320
   aggtataaac gagaatgtgt atgcagagtt acaacactca attaaagtct ttgtaatcat 4380
   atttggactt ggaatttatt caaatggtgt gcaaaaagtt tatatgggaa tacaaaaagc 4440
   ctatataagt aatattgtta atgccatatt tatattgtta tctattatta ctctagtaat 4500
   atcgtcgaaa ctacatgcgg gactaccagt tttaattgtc agcactcttg gtattcaata 4560
   catatcggga atctatttaa caattaatct tattataaag cgattaataa agtttacaaa 4620
   agttaacata catgctaaaa gagaagctcc atatttgata ttaaacggtt ttttcttttt 4680
   tattttacag ttaggcactc tggcaacatg gagtggtgat aactttataa tatctataac 4740
   attgggtgtt acttatgttg ctgtttttag cattacacag agattatttc aaatatctac 4800
   ggtccctctt acgatttata acatcccgtt atgggctgct tatgcagatg ctcatgcacg 4860
   caatgatact caatttataa aaaagacgct cagaacatca ttgaaaatag tgggtatttc 4920
   atcattctta ttggccttca tattagtagt gttcggtagt gaagtcgtta atatttggac 4980
   agaaggaaag attcaggtac ctcgaacatt cataatagct tatgctttat ggtctgttat 5040
   tgatgctttt tcgaatacat ttgcaagctt tttaaatggt ttgaacatag ttaaacaaca 5100
   aatgcttgct gttgtaacat tgatattgat cgcaattcca gcaaaataca tcatagttag 5160
   ccattttggg ttaactgtta tgttgtactg cttcattttt atatatattg taaattactt 5220
   tatatggtat aaatgtagtt ttaaaaaaca tatcgataga cagttaaata taagaggatg 5280
   aaaatgaaat atataccagt ttaccaaccg tcattgacag gaaaagaaaa agaatatgta 5340
   aatgaatgtc tggactcaac gtggatttca tcaaaaggaa actatattca gaagtttgaa 5400
   aataaatttg cggaacaaaa ccatgtgcaa tatgcaacta ctgtaagtaa tggaacggtt 5460
   gctcttcatt tagctttgtt agcgttaggt atatcggaag gagatgaagt tattgttcca 5520
   acactgacat atatagcatc agttaatgct ataaaataca caggagccac ccccattttc 5580
   gttgattcag ataatgaaac ttggcaaatg tctgttagtg acatagaaca aaaaatcact 5640
   aataaaacta aagctattat gtgtgtccat ttatacggac atccatgtga tatggaacaa 5700
   attgtagaac tggccaaaag tagaaatttg tttgtaattg aagattgcgc tgaagccttt 5760
   ggttctaaat ataaaggtaa atatgtggga acatttggag atatttctac ttttagcttt 5820
   tttggaaata aaactattac tacaggtgaa ggtggaatgg ttgtcacgaa tgacaaaaca 5880
   ctttatgacc gttgtttaca ttttaaaggc caaggattag ctgtacatag gcaatattgg 5940
   catgacgtta taggctacaa ttataggatg acaaatatct gcgctgctat aggattagcc 6000
   cagttagaac aagctgatga ttttatatca cgaaaacgtg aaattgctga tatttataaa 6060
   aaaaatatca acagtcttgt acaagtccac aaggaaagta aagatgtttt tcacacttat 6120
   tggatggtct caattctaac taggaccgca gaggaaagag aggaattaag gaatcacctt 6180
   gcagataaac tcatcgaaac aaggccagtt ttttaccctg tccacacgat gccaatgtac 6240
   tcggaaaaat atcaaaagca ccctatagct gaggatcttg gttggcgtgg aattaattta 6300
   cctagtttcc ccagcctatc gaatgagcaa gttatttata tttgtgaatc tattaacgaa 6360
   ttttatagtg ataaatagcc taaaatattg taaaggtcat tcatgaaaat tgcgttgaat 6420
   tcagatggat tttacgagtg gggcggtgga attgatttta ttaaatatat tctgtcaata 6480
   ttagaaacga aaccagaaat atgtatcgat attcttttac cgagaaatga tatacattct 6540
   cttataagag aaaaagcatt tccttttaaa agtatattaa aagcaatttt aaagagggaa 6600
   aggcctcgat ggatttcatt aaatagattt aatgagcaat actatagaga tgcctttaca 6660
   caaaataata tagagacgaa tcttaccttt attaaaagta agagctctgc cttttattca 6720
   tattttgata gtagcgattg tgatgttatt cttccttgca tgcgtgttcc ttcgggaaat 6780
   ttgaataaaa aagcatggat tggttatatt tatgactttc aacactgtta ctatccttca 6840
   ttttttagta agcgagaaat agatcaaagg aatgtgtttt ttaaattgat gctcaattgc 6900
   gctaacaata ttattgttaa tgcacattca gttattaccg atgcaaataa atatgttggg 6960
   aattattctg caaaactaca ttctcttcca tttagtccat gccctcaatt aaaatggttc 7020
   gctgattact ctggtaatat tgccaaatat aatattgaca aggattattt tataatttgc 7080
   aatcaatttt ggaaacataa agatcatgca actgctttta gggcatttaa aatttatact 7140
   gaatataatc ctgatgttta tttagtatgc acgggagcta ctcaagatta tcgattccct 7200
   ggatatttta atgaattgat ggttttggca aaaaagctcg gaattgaatc gaaaattaag 7260
   atattagggc atatacctaa acttgaacaa attgaattaa tcaaaaattg cattgctgta 7320
   atacaaccaa ccttatttga aggcgggcct ggaggggggg taacatttga cgctattgca 7380
   ttagggaaaa aagttatact atctgacata gatgtcaata aagaagttaa ttgcggtgat 7440
   gtatatttct ttcaggcaaa aaaccattat tcattaaatg acgcgatggt aaaagctgat 7500
   gaatctaaaa ttttttatga acctacaact ctgatagaat tgggtctcaa aagacgcaat 7560
   gcgtgtgcag attttctttt agatgttgtg aaacaagaaa ttgaatcccg atcttaatat 7620
   attcaagagg tatataatga ctaaagtcgc tcttattaca ggtgtaactg gacaagatgg 7680
   atcttatcta gctgagtttt tgcttgataa agggtatgaa gttcatggta tcaaacgccg 7740
   agcctcatct tttaatacag aacgcataga ccatatttat caagatccac atggttctaa 7800
   cccaaatttt cacttgcact atggagatct gactgattca tctaacctca ctagaattct 7860
   aaaggaggta cagccagatg aagtatataa tttagctgct atgagtcacg tagcagtttc 7920
   ttttgagtct ccagaatata cagccgatgt cgatgcaatt ggtacattac gtttactgga 7980
   agcaattcgc tttttaggat tggaaaacaa aacgcgtttc tatcaagctt caacctcaga 8040
   attatatgga cttgttcagg aaatccctca aaaagaatcc accccttttt atcctcgttc 8100
   cccttatgca gttgcaaaac tttacgcata ttggatcacg gtaaattatc gagagtcata 8160
   tggtatttat gcatgtaatg gtatattgtt caatcatgaa tctccacgcc gtggagaaac 8220
   gtttgtaaca aggaaaatta ctcgaggact tgcaaatatt gcacaaggct tggaatcatg 8280
   tttgtattta gggaatatgg attcgttacg agattgggga catgcaaaag attatgttag 8340
   aatgcaatgg ttgatgttac aacaggagca acccgaagat tttgtgattg caacaggagt 8400
   ccaatactca gtccgtcagt ttgtcgaaat ggcagcagca caacttggta ttaagatgag 8460
   ctttgttggt aaaggaatcg aagaaaaagg cattgtagat tcggttgaag gacaggatgc 8520
   tccaggtgtg aaaccaggtg atgtcattgt tgctgttgat cctcgttatt tccgaccagc 8580
   tgaagttgat actttgcttg gagatccgag caaagctaat ctcaaacttg gttggagacc 8640
   agaaattact cttgctgaaa tgatttctga aatggttgcc aaagatcttg aagccgctaa 8700
   aaaacattct cttttaaaat cgcatggttt ttctgtaagc ttagctctgg aatgatgatg 8760
   aataagcaac gtatttttat tgctggtcac caaggaatgg ttggatcagc tattacccga 8820
   cgcctcaaac aacgtgatga tgttgagttg gttttacgta ctcgggatga attgaacttg 8880
   ttggatagta gcgctgtttt ggattttttt tcttcacaga aaatcgacca ggtttatttg 8940
   gcagcagcaa aagtcggagg tattttagct aacagttctt atcctgccga ttttatatat 9000
   gagaatataa tgatagaggc gaatgtcatt catgctgccc acaaaaataa tgtaaataaa 9060
   ctgcttttcc tcggttcgtc gtgtatttat cctaagttag cacaccaacc gattatggaa 9120
   gacgaattat tacaagggaa acttgagcca acaaatgaac cttatgctat cgcaaaaatt 9180
   gcaggtatta aattatgtga atcttataac cgtcagtttg ggcgtgatta ccgttcagta 9240
   atgccaacca atctttatgg tccaaatgac aattttcatc caagtaattc tcatgtgatt 9300
   ccggcgcttt tgcgccgctt tcatgatgct gtggaaaaca attctccgaa tgttgttgtt 9360
   tggggaagtg gtactccaaa gcgtgaattc ttacatgtag atgatatggc ttctgcaagc 9420
   atttatgtca tggagatgcc atacgatata tggcaaaaaa atactaaagt aatgttgtct 9480
   catatcaata ttggaacagg tattgactgc acgatttgtg agcttgcgga aacaatagca 9540
   aaagttgtag gttataaagg gcatattacg ttcgatacaa caaagcccga tggagcccct 9600
   cgaaaactac ttgatgtaac gcttcttcat caactaggtt ggaatcataa aattaccctt 9660
   cacaagggtc ttgaaaatac atacaactgg tttcttgaaa accaacttca atatcggggg 9720
   taataatgtt tttacattcc caagactttg ccacaattgt aaggtctact cctcttattt 9780
   ctatagattt gattgtggaa aacgagtttg gcgaaatttt gctaggaaaa cgaatcaacc 9840
   gcccggcaca gggctattgg ttcgttcctg gtggtagggt gttgaaagat gaaaaattgc 9900
   agacagcctt tgaacgattg acagaaattg aactaggaat tcgtttgcct ctctctgtgg 9960
   gtaagtttta tggtatctgg cagcacttct acgaagacaa tagtatgggg ggagactttt 10020
   caacgcatta tatagttata gcattccttc ttaaattaca accaaacatt ttgaaattac 10080
   cgaagtcaca acataatgct tattgctggc tatcgcgagc aaagctgata aatgatgacg 10140
   atgtgcatta taattgtcgc gcatatttta acaataaaac aaatgatgcg attggcttag 10200
   ataataagga tataatatgt ctgatgcgcc aataattgct gtagttatgg ccggtggtac 10260
   aggcagtcgt ctttggccac tttctcgtga actatatcca aagcagtttt tacaactctc 10320
   tggtgataac accttgttac aaacgacttt gctacgactt tcaggcctat catgtcaaaa 10380
   accattagtg ataacaaatg aacagcatcg ctttgttgtg gctgaacagt taagggaaat 10440
   aaataaatta aatggtaata ttattctaga accatgcggg cgaaatactg caccagcaat 10500
   agcgatatct gcgtttcatg cgttaaaacg taatcctcag gaagatccat tgcttctagt 10560
   tcttgcggca gaccacgtta tagctaaaga aagtgttttc tgtgatgcta ttaaaaatgc 10620
   aactcccatc gctaatcaag gtaaaattgt aacgtttgga attataccag aatatgctga 10680
   aactggttat gggtatattg agagaggtga actatctgta ccgcttcaag ggcatgaaaa 10740
   tactggtttt tattatgtaa ataagtttgt cgaaaagcct aatcgtgaaa ccgcagaatt 10800
   gtatatgact tctggtaatc actattggaa tagtggaata ttcatgttta aggcatctgt 10860
   ttatcttgag gaattgagaa aatttagacc tgacatttac aatgtttgtg aacaggttgc 10920
   ctcatcctca tacattgatc tagattttat tcgattatca aaagaacaat ttcaagattg 10980
   tcctgctgaa tctattgatt ttgctgtaat ggaaaaaaca gaaaaatgtg ttgtatgccc 11040
   tgttgatatt ggttggagtg acgttggatc ttggcaatcg ttatgggaca ttagtctaaa 11100
   atcgaaaaca ggagatgtat gtaaaggtga tatattaacc tatgatacta agaataatta 11160
   tatctactct gagtcagcgt tggtagccgc cattggaatt gaagatatgg ttatcgtgca 11220
   aactaaagat gccgttcttg tgtctaaaaa gagtgatgta cagcatgtaa aaaaaatagt 11280
   cgaaatgctt aaattgcagc aacgtacaga gtatattagt catcgtgaag ttttccgacc 11340
   atggggaaaa tttgattcga ttgaccaagg tgagcgatac aaagtcaaga aaattattgt 11400
   gaaacctggt gaggggcttt ctttaaggat gcatcaccat cgttctgaac attggatcgt 11460
   gctttctggt acagcaaaag taacccttgg cgataaaact aaactagtca ccgcaaatga 11520
   atcgatatac attccccttg gcgcagcgta tagtcttgag aatccgggca taatccctct 11580
   taatcttatt gaagtcagtt caggggatta tttgggagag gatgatatta taagacagaa 11640
   agaacgttac aaacatgaag attaacatat gaaatcttta acctgcttta aagcctatga 11700
   tattcgcggg aaattaggcg aagaactgaa tgaagatatt gcctggcgca ttgggcgtgc 11760
   ctatggcgaa tttctcaaac cgaaaaccat tgttttaggc ggtgatgtcc gcctcaccag 11820
   cgaagcgtta aaactggcgc ttgcgaaagg tttacaggat gcgggcgtcg atgtgctgga 11880
   tatcggtatg tccggcaccg aagagatcta tttcgccacg ttccatctcg gagtggatgg 11940
   cggcatcgaa gttaccgcca gccataaccc gatggattac aacggcatga agctggtgcg 12000
   cgaaggggct cgcccgatca gcggtgatac cggactgcgc gatgtccagc gtctggcaga 12060
   agccaatgac ttccctcctg tcgatgaaac caaacgtggt cgctatcagc aaatcaatct 12120
   gcgtgacgct tacgttgatc acctgttcgg ttatatcaac gtcaaaaacc tcacgccgct 12180
   caagctggtg atcaactccg ggaacggcgc agcgggtccg gtggtggacg ccattgaagc 12240
   ccgatttaaa gccctcggcg caccggtgga attaatcaaa gtacacaaca cgccggacgg 12300
   caatttcccc aacggtattc ctaacccgct gctgccggaa tgccgcgacg acacccgtaa 12360
   tgcggtcatc aaacacggcg cggatatggg cattgccttt gatggcgatt ttgaccgctg 12420
   tttcctgttt gacgaaaaag ggcagtttat cgagggctac tacattgtcg gcctgctggc 12480
   agaagcgttc ctcgaaaaaa atcccggcgc gaagatcatc cacgatccac gtctctcctg 12540
   gaacaccgtt gatgtggtga ctgccgcagg cggcaccccg gtaatgtcga aaaccggaca 12600
   cgcctttatt aaagaacgta tgcgcaagga agacgccatc tacggtggcg aaatgagcgc 12660
   tcaccattac ttccgtgatt tcgcttactg cgacagcggc atgatcccgt ggctgctggt 12720
   cgccgaactg gtgtgcctga aaggaaaaac gctgggcgaa atggtgcgcg accggatggc 12780
   ggcgtttccg gcaagcggtg agatcaacag caaactggcg caacccgttg aggcaattaa 12840
   tcgcgtggaa cagcatttta gccgcgaggc gctggcggtg gatcgcaccg atggcatcag 12900
   catgaccttt gccgactggc gctttaacct gcgctcctcc aacaccgaac cggtggtgcg 12960
   gttgaatgtg gaatcacgcg gtgatgtaaa gctaatggaa aagaaaacta aagctcttct 13020
   taaattgcta agtgagtgat tatttacatt aatcattaag cgtatttaag attatattaa 13080
   agtaatgtta ttgcggtata tgatgaatat gtgggctttt ttatgtataa cgactatacc 13140
   gcaactttat ctaggaaaag attaatagaa ataaagtttt gtactgacca atttgcattt 13200
   cacgtcacga ttgagacgtt cctttgctta agacattttt tcatcgctta tgtaataaca 13260
   aatgtgcctt atataaaaag gagaacaaaa tggaacttaa aataattgag acaatagatt 13320
   tttattatcc ctgtttacga tattatagcc aaagttgtat cctgcatcag tcctgcaata 13380
   tttcacgagt gctttgttaa ctgaatacat gtctgccatt ttccagatga taacgacgtc 13440
   atcgcaattg atggtaaaac acttcggcac acttatgaca agagtcgtcg cagaggagtg 13500
   gttcatgtca ttagtgcgtt tcagcaatgc acagtctggt cctcggatag atcaagacgg 13560
   atgagaaacc taatgcgttc acagttattc atgaactttc taaaatgatg ggtattaaag 13620
   gaaaaataat cataactgat gcgatggctt gccagaaaga tattgcagag aagatataaa 13680
   aacagagatg tgattattta ttcgctgtaa aaggaaataa gagtcggctt aatagagtct 13740
   ttgaggagat atttacgctg aaagaattaa ataatccaaa acatgacagt tacgcaatta 13800
   gtgaaaagag gcacggcaga gacgatgtcc gtcttcatat tgtttgagat gctcctgatg 13860
   agcttattga tttcacgttt gaatggaaag ggctgcagaa tttatgaatg gcagtccact 13920
   ttctctcaat aatagcagag caaaagaaag aatccgaaat gacgatcaaa tattatatta 13980
   gatctgctgc tttaaccgca gagaagttcg ccacagtaaa tcgaaatcac tggcgcatgg 14040
   agaataagtt gcacagtagc ctgatgtggt aatgaatgaa atcgactata atataagaag 14100
   gcgagttgca ttcgaatgat tttctagaat gcggcacatc gctattaata tctgacaatg 14160
   ataatgtatt caaggcagga ttatcatgta agatgcgaaa agcagtcatg gacagaaact 14220
   tcctagcgtc aggcattgca gcgtgcgggc tttcataatc ttgcattggt tttgataaga 14280
   tatttctttg gagatgggaa aatgaatttg tatggtattt ttggtgctgg aagttatggt 14340
   agagaaacaa tacccattct aaatcaacaa ataaagcaag aatgtggttc tgactatgct 14400
   ctggtttttg tggatgatgt tttggcagga aagaaagtta atggttttga agtgctttca 14460
   accaactgct ttctaaaagc cccttattta aaaaagtatt ttaatgttgc tattgctaat 14520
   gataagatac gacagagagt gtctgagtca atattattac acggggttga accaataact 14580
   ataaaacatc caaatagcgt tgtttatgat catactatga taggtagtgg cgctattatt 14640
   tctccctttg ttacaatatc tactaatact catataggga ggttttttca tgcaaacata 14700
   tactcatacg ttgcacatga ttgtcaaata ggagactatg ttacatttgc tcctggggct 14760
   aaatgtaatg gatatgttgt tattgaagac aatgcatata taggctcggg tgcagtaatt 14820
   aagcagggtg ttcctaatcg cccacttatt attggcgcgg gagccattat aggtatgggg 14880
   gctgttgtca ctaaaagtgt tcctgccggt ataactgtgt gcggaaatcc agcaagagaa 14940
   atgaaaagat cgccaacatc tatttaatgg gaatgcgaaa acacgttcca aatgggacta 15000
   atgtttaaaa tatatataat ttcgctaatt tactaaatta tggcttcttt ttaagctatc 15060
   ctttacttag ttattactga tacagcatga aatttataat actctgatac atttttatac 15120
   gttattcaag ccgcatatct agcggtaacc cctgacagga gtaaacaatg tcaaagcaac 15180
   agatcggcgt agtcggtatg gcagtgatgg ggcgcaacct tgcgctcaac atcgaaagcc 15240
   gtggttatac cgtctctatt ttcaaccgtt cccgtgaaaa gacggaagaa gtgattgccg 15300
   aaaatccagg caagaaactg gttccttact atacggtgaa agaatttgtt gaatctctgg 15360
   aaacgcctcg tcgcatcttg ttaatggtga aagcaggtgc aggcacggat gctgctattg 15420
   attcccttaa gccatacctc gataaaggtg acatcatcat tgatggtggt aataccttct 15480
   tccaggacac cattcgtcgt aaccgtgagc tttctgcaga aggctttaac ttcatcggta 15540
   ccggtgtttc cggtggtgag gagggcgcac taaaaggtcc ttccattatg cctggtgggc 15600
   agaaagaagc ctatgaacta gttgcgccga tcctgaccaa aatcgccgca gtggctgaag 15660
   acggtgagcc atgcgttacc tatattggtg ccgatggcgc aggtcactat gtgaagatgg 15720
   ttcacaacgg tattgaatac ggcgatatgc agctgattgc tgaagcctat tctctgctta 15780
   aaggtggtct gaacctcacc aacgaagaac tggcgcagat ctttaccgag tggaataacg 15840
   gtgaactgag cagctacctg atcgacatta ccaaagacat cttcactaaa aaagatgaag 15900
   acggtaacta cctggttgat gtgatcctgg atgaagcggc aaacaaaggt acgggcaaat 15960
   ggaccagcca gagcgcactg gatctcggcg aaccgctgtc gctgattacc gagtctgtgt 16020
   ttgcacgata catctcttct ctgaaagatc agcgcgttgc tgcgtctaaa gttctctctg 16080
   gcccacaagc gcagccagct ggcgacaagg ctgagttcat cgaaaaagtt cgccgtgcac 16140
   tgtatctggg caaaatcgtt tcttacgctc aggggttctc tcaactgcgt gcggcgtctg 16200
   aagagtacaa ctgggatctg aactacggcg aaatcgcgaa gattttccgt gctggctgca 16260
   tcatccgtgc gcagttcctg cagaaaatca ccgatgctta tgccgaaaat ccgcagatcg 16320
   ctaacctgct gctggctcct tacttcaagc aaattgccga tgactaccag caggcgctgc 16380
   gcgatgtcgt cgcttatgcg gtacagaacg gtatcccggt tccgaccttc gccgctgcgg 16440
   ttgcctatta tgacagctac cgcgccgctg ttctgcctgc gaacctgatc caggcacagc 16500
   gtgactattt cggtgcgcat acttataagc gcattgataa agaaggtgtg ttccataccg 16560
   aatggctgga ttaagcggcc gc 16582
<210> 80
   <211> 32
   <212> DNA
   <213> artificial
<220>
   <223> primer sequence
<400> 80
   atatggcgcg cctctgagtt acaacagtcc gc 32
<210> 81
   <211> 32
   <212> DNA
   <213> artificial
<220>
   <223> primer sequence
<400> 81
   ataagcggcc gcttcaggtg ctacatttga ag 32
<210> 82
   <211> 2488
   <212> DNA
   <213> artificial
<220>
   <223> linear pACYC184 vector fragment
<400> 82
   ggcgcgcctc tgagttacaa cagtccgcac cgctgtccgg tagctccttc cggtgggcgc 60
   ggggcatgac tatcgtcgcc gcacttatga ctgtcttctt tatcatgcaa ctcgtaggac 120
   aggtgccggc agcgcccaac agtcccccgg ccacggggcc tgccaccata cccacgccga 180
   aacaagcgcc ctgcaccatt atgttccgga tctgcatcgc aggatgctgc tggctaccct 240
   gtggaacacc tacatctgta ttaacgaagc gctaaccgtt tttatcaggc tctgggaggc 300
   agaataaatg atcatatcgt caattattac ctccacgggg agagcctgag caaactggcc 360
   tcaggcattt gagaagcaca cggtcacact gcttccggta gtcaataaac cggtaaacca 420
   gcaatagaca taagcggcta tttaacgacc ctgccctgaa ccgacgaccg ggtcgaattt 480
   gctttcgaat ttctgccatt catccgctta ttatcactta ttcaggcgta gcaccaggcg 540
   tttaagggca ccaataactg ccttaaaaaa attacgcccc gccctgccac tcatcgcagt 600
   actgttgtaa ttcattaagc attctgccga catggaagcc atcacagacg gcatgatgaa 660
   cctgaatcgc cagcggcatc agcaccttgt cgccttgcgt ataatatttg cccatggtga 720
   aaacgggggc gaagaagttg tccatattgg ccacgtttaa atcaaaactg gtgaaactca 780
   cccagggatt ggctgagacg aaaaacatat tctcaataaa ccctttaggg aaataggcca 840
   ggttttcacc gtaacacgcc acatcttgcg aatatatgtg tagaaactgc cggaaatcgt 900
   cgtggtattc actccagagc gatgaaaacg tttcagtttg ctcatggaaa acggtgtaac 960
   aagggtgaac actatcccat atcaccagct caccgtcttt cattgccata cggaattccg 1020
   gatgagcatt catcaggcgg gcaagaatgt gaataaaggc cggataaaac ttgtgcttat 1080
   ttttctttac ggtctttaaa aaggccgtaa tatccagctg aacggtctgg ttataggtac 1140
   attgagcaac tgactgaaat gcctcaaaat gttctttacg atgccattgg gatatatcaa 1200
   cggtggtata tccagtgatt tttttctcca ttttagcttc cttagctcct gaaaatctcg 1260
   ataactcaaa aaatacgccc ggtagtgatc ttatttcatt atggtgaaag ttggaacctc 1320
   ttacgtgccg atcaacgtct cattttcgcc aaaagttggc ccagggcttc ccggtatcaa 1380
   cagggacacc aggatttatt tattctgcga agtgatcttc cgtcacaggt atttattcgg 1440
   cgcaaagtgc gtcgggtgat gctgccaact tactgattta gtgtatgatg gtgtttttga 1500
   ggtgctccag tggcttctgt ttctatcagc tgtccctcct gttcagctac tgacggggtg 1560
   gtgcgtaacg gcaaaagcac cgccggacat cagcgctagc ggagtgtata ctggcttact     1620
   atgttggcac tgatgagggt gtcagtgaag tgcttcatgt ggcaggagaa aaaaggctgc     1680
   accggtgcgt cagcagaata tgtgatacag gatatattcc gcttcctcgc tcactgactc     1740
   gctacgctcg gtcgttcgac tgcggcgagc ggaaatggct tacgaacggg gcggagattt     1800
   cctggaagat gccaggaaga tacttaacag ggaagtgaga gggccgcggc aaagccgttt     1860
   ttccataggc tccgcccccc tgacaagcat cacgaaatct gacgctcaaa tcagtggtgg     1920
   cgaaacccga caggactata aagataccag gcgtttcccc ctggcggctc cctcgtgcgc     1980
   tctcctgttc ctgcctttcg gtttaccggt gtcattccgc tgttatggcc gcgtttgtct     2040
   cattccacgc ctgacactca gttccgggta ggcagttcgc tccaagctgg actgtatgca     2100
   cgaacccccc gttcagtccg accgctgcgc cttatccggt aactatcgtc ttgagtccaa     2160
   cccggaaaga catgcaaaag caccactggc agcagccact ggtaattgat ttagaggagt     2220
   tagtcttgaa gtcatgcgcc ggttaaggct aaactgaaag gacaagtttt ggtgactgcg     2280
   ctcctccaag ccagttacct cggttcaaag agttggtagc tcagagaacc ttcgaaaaac     2340
   cgccctgcaa ggcggttttt tcgttttcag agcaagagat tacgcgcaga ccaaaacgat     2400
   ctcaagaaga tcatcttatt aatcagataa aatatttcta gatttcagtg caatttatct     2460
   cttcaaatgt agcacctgaa gcggccgc                                        2488